# EUROPEAN PATENT SPECIFICATION

(11) **EP 3 409 659 B1**
(45) Date of publication and mention of the grant of the patent: **30.12.2020**
(21) Application number: 17744327.2
(22) Date of filing: 26.01.2017
(51) Int. Cl.: C07C 233/47, A61K 31/198, A61K 31/221, A61K 31/223, A61K 31/655, A61P 3/10, A61P 25/00, A61P 25/16, A61P 25/18, A61P 25/28, A61P 43/00, C07C 229/28, C07C 247/14, C07D 213/40

(54) **ADAMANTANE DERIVATIVE AND USE THEREOF**
ADAMANTANDERIVAT UND VERWENDUNG DAVON
DÉRIVÉ D'ADAMANTANE ET SON UTILISATION

(30) Priority: 26.01.2016 JP 2016012392
(43) Date of publication of application: 05.12.2018
(73) Proprietor: TOHOKU UNIVERSITY, Sendai-shi Miyagi 980-8577 (JP); Brain Innovation Co., Inc., Miyagi 980-8579 (JP)
(72) Inventor: MORIGUCHI, Shigeki, Sendai-shi Miyagi 980-8577 (JP); FUKUNAGA, Koji, Sendai-shi Miyagi 980-8577 (JP); IWABUCHI, Yoshiharu, Sendai-shi Miyagi 980-8577 (JP)
(74) Representative: Mewburn Ellis LLP
(86) International application number: PCT/JP2017/002760
(87) International publication number: WO 2017/131097

(56) References cited:
- WO-A1-2009/020140
- WO-A1-2011/142246
- WO-A1-2015/087262
- WO-A2-2010/043953
- CN-A- 104 001 150
- JP-A- 2009 508 956
- JP-A- 2010 522 203
- JP-A- 2011 529 057

### SEQUENCE LISTING

<110> Tohoku University
<120> Adamantane derivative and use thereof
<130> FA5026-16272
<150> JP 2016-012392
   <151> 2016-01-26
<160> 2
<170> PatentIn version 3.5
<210> 1
   <211> 6631
   <212> DNA
   <213> Artificial Sequence
<220>
   <223> vector
<400> 1 
gacggatcgg gagatctccc gatcccctat ggtgcactct cagtacaatc tgctctgatg 60

ccgcatagtt aagccagtat ctgctccctg cttgtgtgtt ggaggtcgct gagtagtgcg 120

cgagcaaaat ttaagctaca acaaggcaag gcttgaccga caattgcatg aagaatctgc 180

ttagggttag gcgttttgcg ctgcttcgcg atgtacgggc cagatatacg cgttgacatt 240

gattattgac tagttattaa tagtaatcaa ttacggggtc attagttcat agcccatata 300

tggagttccg cgttacataa cttacggtaa atggcccgcc tggctgaccg cccaacgacc 360

cccgcccatt gacgtcaata atgacgtatg ttcccatagt aacgccaata gggactttcc 420

attgacgtca atgggtggac tatttacggt aaactgccca cttggcagta catcaagtgt 480

atcatatgcc aagtacgccc cctattgacg tcaatgacgg taaatggccc gcctggcatt 540

atgcccagta catgacctta tgggactttc ctacttggca gtacatctac gtattagtca 600

tcgctattac catggtgatg cggttttggc agtacatcaa tgggcgtgga tagcggtttg 660

actcacgggg atttccaagt ctccacccca ttgacgtcaa tgggagtttg ttttggcacc 720

aaaatcaacg ggactttcca aaatgtcgta acaactccgc cccattgacg caaatgggcg 780

gtaggcgtgt acggtgggag gtctatataa gcagagctct ctggctaact agagaaccca 840

ctgcttactg gcttatcgaa attaatacga ctcactatag ggagacccaa gctggctagt 900

taagcttggt accgagctcg gatccgccac catgctgtcc cgaaaggcca ttatccctga 960

ggaatatgtg ctgacccggc tggcagagga ccctacagag cccaggtacc gtactcggga 1020

gaggagggcc cgcttcgtgt ccaagaaagg caactgcaac gtcgcccaca agaacatccg 1080

agagcagggc cgcttcctgc aagatgtgtt caccacgctg gtggacctca agtggcccca 1140

cacgctgctc attttcacca tgtccttcct gtgcagctgg ctgctcttcg ccatggtctg 1200
 
gtggctcatc gcctttgccc acggtgactt ggcccccgga gagggcacca atgtgccctg 1260

cgtcacaagc atccactcct tttcgtctgc cttccttttc tccatcgagg tccaggtgac 1320

cattggtttc ggcgggcgca tggtgacaga ggaatgtccc ctggccatcc ttattctgat 1380

cgtgcagaat atcgtagggc taatgatcaa cgccatcatg ctgggctgca tcttcatgaa 1440

aacggcacag gcccatcggc gggcagaaac cctcatcttc agcaagcatg ccgtgatcac 1500

cctgcgacat ggccgcctgt gcttcatgct tcgcgtaggg gacctccgaa aaagcatgat 1560

cattagcgcc accattcata tgcaggtggt gcgcaagacc accagcccgg agggcgaggt 1620

tgtgcctctc caccaggtgg acatccccat ggagaacggt gtgggtggta acagcatctt 1680

tctggtggcc ccactcatca tctaccacgt catcgactcc aacagcccgc tctacgacct 1740

ggctcctagt gacctgcacc accaccagga cctggagatc attgtcatct tggaaggtgt 1800

ggtagaaacc acaggcatta ccacccaggc ccgcacctcc tatctggctg acgagattct 1860

gtgggggcag cgttttgtcc ccatcgtggc cgaggaggat ggccgctatt ctgtggacta 1920

ctccaaattc gggaacaccg ttaaagtgcc cacaccactc tgcacagccc gccagcttga 1980

tgaggaccgc agcctgctgg atgccctgac cctcgcctcg tcgcgagggc ccctgcgcaa 2040

gcgcagtgtg gctgtggcaa aggccaagcc caagtttagc atctctccgg attccttgtc 2100

ctgatagcgg ccgctcgagt ctagagggcc cttcgaacaa aaactcatct cagaagagga 2160

tctgaatatg cataccggtc atcatcacca tcaccattga gtttaaaccc gctgatcagc 2220

ctcgactgtg ccttctagtt gccagccatc tgttgtttgc ccctcccccg tgccttcctt 2280

gaccctggaa ggtgccactc ccactgtcct ttcctaataa aatgaggaaa ttgcatcgca 2340

ttgtctgagt aggtgtcatt ctattctggg gggtggggtg gggcaggaca gcaaggggga 2400

ggattgggaa gacaatagca ggcatgctgg ggatgcggtg ggctctatgg cttctgaggc 2460

ggaaagaacc agctggggct ctagggggta tccccacgcg ccctgtagcg gcgcattaag 2520

cgcggcgggt gtggtggtta cgcgcagcgt gaccgctaca cttgccagcg ccctagcgcc 2580

cgctcctttc gctttcttcc cttcctttct cgccacgttc gccggctttc cccgtcaagc 2640

tctaaatcgg gggctccctt tagggttccg atttagtgct ttacggcacc tcgaccccaa 2700

aaaacttgat tagggtgatg gttcacgtag tgggccatcg ccctgataga cggtttttcg 2760

ccctttgacg ttggagtcca cgttctttaa tagtggactc ttgttccaaa ctggaacaac 2820

actcaaccct atctcggtct attcttttga tttataaggg attttgccga tttcggccta 2880

ttggttaaaa aatgagctga tttaacaaaa atttaacgcg aattaattct gtggaatgtg 2940

tgtcagttag ggtgtggaaa gtccccaggc tccccagcag gcagaagtat gcaaagcatg 3000

catctcaatt agtcagcaac caggtgtgga aagtccccag gctccccagc aggcagaagt 3060
 
atgcaaagca tgcatctcaa ttagtcagca accatagtcc cgcccctaac tccgcccatc 3120

ccgcccctaa ctccgcccag ttccgcccat tctccgcccc atggctgact aatttttttt 3180

atttatgcag aggccgaggc cgcctctgcc tctgagctat tccagaagta gtgaggaggc 3240

ttttttggag gcctaggctt ttgcaaaaag ctcccgggag cttgtatatc cattttcgga 3300

tctgatcaag agacaggatg aggatcgttt cgcatgattg aacaagatgg attgcacgca 3360

ggttctccgg ccgcttgggt ggagaggcta ttcggctatg actgggcaca acagacaatc 3420

ggctgctctg atgccgccgt gttccggctg tcagcgcagg ggcgcccggt tctttttgtc 3480

aagaccgacc tgtccggtgc cctgaatgaa ctgcaggacg aggcagcgcg gctatcgtgg 3540

ctggccacga cgggcgttcc ttgcgcagct gtgctcgacg ttgtcactga agcgggaagg 3600

gactggctgc tattgggcga agtgccgggg caggatctcc tgtcatctca ccttgctcct 3660

gccgagaaag tatccatcat ggctgatgca atgcggcggc tgcatacgct tgatccggct 3720

acctgcccat tcgaccacca agcgaaacat cgcatcgagc gagcacgtac tcggatggaa 3780

gccggtcttg tcgatcagga tgatctggac gaagagcatc aggggctcgc gccagccgaa 3840

ctgttcgcca ggctcaaggc gcgcatgccc gacggcgagg atctcgtcgt gacccatggc 3900

gatgcctgct tgccgaatat catggtggaa aatggccgct tttctggatt catcgactgt 3960

ggccggctgg gtgtggcgga ccgctatcag gacatagcgt tggctacccg tgatattgct 4020

gaagagcttg gcggcgaatg ggctgaccgc ttcctcgtgc tttacggtat cgccgctccc 4080

gattcgcagc gcatcgcctt ctatcgcctt cttgacgagt tcttctgagc gggactctgg 4140

ggttcgcgaa atgaccgacc aagcgacgcc caacctgcca tcacgagatt tcgattccac 4200

cgccgccttc tatgaaaggt tgggcttcgg aatcgttttc cgggacgccg gctggatgat 4260

cctccagcgc ggggatctca tgctggagtt cttcgcccac cccaacttgt ttattgcagc 4320

ttataatggt tacaaataaa gcaatagcat cacaaatttc acaaataaag catttttttc 4380

actgcattct agttgtggtt tgtccaaact catcaatgta tcttatcatg tctgtatacc 4440

gtcgacctct agctagagct tggcgtaatc atggtcatag ctgtttcctg tgtgaaattg 4500

ttatccgctc acaattccac acaacatacg agccggaagc ataaagtgta aagcctgggg 4560

tgcctaatga gtgagctaac tcacattaat tgcgttgcgc tcactgcccg ctttccagtc 4620

gggaaacctg tcgtgccagc tgcattaatg aatcggccaa cgcgcgggga gaggcggttt 4680

gcgtattggg cgctcttccg cttcctcgct cactgactcg ctgcgctcgg tcgttcggct 4740

gcggcgagcg gtatcagctc actcaaaggc ggtaatacgg ttatccacag aatcagggga 4800

taacgcagga aagaacatgt gagcaaaagg ccagcaaaag gccaggaacc gtaaaaaggc 4860

cgcgttgctg gcgtttttcc ataggctccg cccccctgac gagcatcaca aaaatcgacg 4920

ctcaagtcag aggtggcgaa acccgacagg actataaaga taccaggcgt ttccccctgg 4980
 
aagctccctc gtgcgctctc ctgttccgac cctgccgctt accggatacc tgtccgcctt 5040

tctcccttcg ggaagcgtgg cgctttctca tagctcacgc tgtaggtatc tcagttcggt 5100

gtaggtcgtt cgctccaagc tgggctgtgt gcacgaaccc cccgttcagc ccgaccgctg 5160

cgccttatcc ggtaactatc gtcttgagtc caacccggta agacacgact tatcgccact 5220

ggcagcagcc actggtaaca ggattagcag agcgaggtat gtaggcggtg ctacagagtt 5280

cttgaagtgg tggcctaact acggctacac tagaagaaca gtatttggta tctgcgctct 5340

gctgaagcca gttaccttcg gaaaaagagt tggtagctct tgatccggca aacaaaccac 5400

cgctggtagc ggtggttttt ttgtttgcaa gcagcagatt acgcgcagaa aaaaaggatc 5460

tcaagaagat cctttgatct tttctacggg gtctgacgct cagtggaacg aaaactcacg 5520

ttaagggatt ttggtcatga gattatcaaa aaggatcttc acctagatcc ttttaaatta 5580

aaaatgaagt tttaaatcaa tctaaagtat atatgagtaa acttggtctg acagttacca 5640

atgcttaatc agtgaggcac ctatctcagc gatctgtcta tttcgttcat ccatagttgc 5700

ctgactcccc gtcgtgtaga taactacgat acgggagggc ttaccatctg gccccagtgc 5760

tgcaatgata ccgcgagacc cacgctcacc ggctccagat ttatcagcaa taaaccagcc 5820

agccggaagg gccgagcgca gaagtggtcc tgcaacttta tccgcctcca tccagtctat 5880

taattgttgc cgggaagcta gagtaagtag ttcgccagtt aatagtttgc gcaacgttgt 5940

tgccattgct acaggcatcg tggtgtcacg ctcgtcgttt ggtatggctt cattcagctc 6000

cggttcccaa cgatcaaggc gagttacatg atcccccatg ttgtgcaaaa aagcggttag 6060

ctccttcggt cctccgatcg ttgtcagaag taagttggcc gcagtgttat cactcatggt 6120

tatggcagca ctgcataatt ctcttactgt catgccatcc gtaagatgct tttctgtgac 6180

tggtgagtac tcaaccaagt cattctgaga atagtgtatg cggcgaccga gttgctcttg 6240

cccggcgtca atacgggata ataccgcgcc acatagcaga actttaaaag tgctcatcat 6300

tggaaaacgt tcttcggggc gaaaactctc aaggatctta ccgctgttga gatccagttc 6360

gatgtaaccc actcgtgcac ccaactgatc ttcagcatct tttactttca ccagcgtttc 6420

tgggtgagca aaaacaggaa ggcaaaatgc cgcaaaaaag ggaataaggg cgacacggaa 6480

atgttgaata ctcatactct tcctttttca atattattga agcatttatc agggttattg 6540

tctcatgagc ggatacatat ttgaatgtat ttagaaaaat aaacaaatag gggttccgcg 6600

cacatttccc cgaaaagtgc cacctgacgt c 6631
<210> 2
   <211> 6733
   <212> DNA
   <213> Artificial Sequence
<220>
   <223> vector
<400> 2

gacggatcgg gagatctccc gatcccctat ggtgcactct cagtacaatc tgctctgatg 60

ccgcatagtt aagccagtat ctgctccctg cttgtgtgtt ggaggtcgct gagtagtgcg 120

cgagcaaaat ttaagctaca acaaggcaag gcttgaccga caattgcatg aagaatctgc 180

ttagggttag gcgttttgcg ctgcttcgcg atgtacgggc cagatatacg cgttgacatt 240

gattattgac tagttattaa tagtaatcaa ttacggggtc attagttcat agcccatata 300

tggagttccg cgttacataa cttacggtaa atggcccgcc tggctgaccg cccaacgacc 360

cccgcccatt gacgtcaata atgacgtatg ttcccatagt aacgccaata gggactttcc 420

attgacgtca atgggtggac tatttacggt aaactgccca cttggcagta catcaagtgt 480

atcatatgcc aagtacgccc cctattgacg tcaatgacgg taaatggccc gcctggcatt 540

atgcccagta catgacctta tgggactttc ctacttggca gtacatctac gtattagtca 600

tcgctattac catggtgatg cggttttggc agtacatcaa tgggcgtgga tagcggtttg 660

actcacgggg atttccaagt ctccacccca ttgacgtcaa tgggagtttg ttttggcacc 720

aaaatcaacg ggactttcca aaatgtcgta acaactccgc cccattgacg caaatgggcg 780

gtaggcgtgt acggtgggag gtctatataa gcagagctct ctggctaact agagaaccca 840

ctgcttactg gcttatcgaa attaatacga ctcactatag ggagacccaa gctggctagt 900

taagcttggt accgagctcg gatccgccac catgctggcc aggaagagca tcatcccgga 960

ggagtatgtg ctggcccgca tcgcggcgga gaacctgcgc aaaccgcgca tccgcgaccg 1020

cctccccaaa gcccgcttca tcgccaagag cggagcctgc aacctggctc acaagaacat 1080

ccgagagcaa ggtcgcttcc tgcaggacat cttcaccacc ttggtagacc tgaagtggcg 1140

tcacacgctg gtcatcttca ccatgtcctt cctctgcagc tggctgctct tcgctatcat 1200

gtggtggctg gtggccttcg cccacgggga catctatgct tacatggaga aaggcatcac 1260

ggagaagagt ggcctggagt ctgccgtctg tgtgaccaat gtcaggtcat tcacttctgc 1320

gtttctcttc tccatcgagg ttcaagtgac cattgggttt ggagggagaa tgatgactga 1380

ggagtgccct ctggccatca cggttttgat tctgcagaac attgtgggtc tgatcatcaa 1440

cgcggtcatg ttgggctgca tcttcatgaa gacggcccag gcccacagaa gggcagagac 1500

gctgattttc agccgccatg ctgtaattgc ggtccgtaat ggcaagctgt gcttcatgtt 1560

ccgggtgggt gacctgagga aaagcatgat cattagcgcc tcggtgcgca tccaggtggt 1620

caagaaaacc acgacgccag aaggagaggt ggtgcctatt caccagcagg acatccctgt 1680

ggataatccc atcgagagca ataacatctt cctagtggcc cctttgatca tctgccatgt 1740

gattgataag cgtagccccc tgtacgatat ctcagccact gaccttgtca accaagacct 1800

ggaggtcata gtgattctcg agggcgtggt ggaaaccacg ggcatcacca cgcaagcgcg 1860

gacctcctac attgcagagg agatccagtg gggacaccgc ttcgtgtcga ttgtgactga 1920

ggaggaggga gtgtactctg tggactattc taaatttggt aatactgtga gagtggcggc 1980

gccaagatgc agtgcccggg agctggacga gaaaccttcc atcttgattc agaccctcca 2040

aaagagtgaa ctgtcgcacc agaattctct gaggaagcgc aactctatga gaagaaacaa 2100

ctccatgagg aggagcaact ccatccggag gaataactct tccctcatgg tgcccaaggt 2160

gcaattcatg actccagaag gaaaccagtg cccatcagaa tcatgatagc ggccgctcga 2220

gtctagaggg cccttcgaac aaaaactcat ctcagaagag gatctgaata tgcataccgg 2280

tcatcatcac catcaccatt gagtttaaac ccgctgatca gcctcgactg tgccttctag 2340

ttgccagcca tctgttgttt gcccctcccc cgtgccttcc ttgaccctgg aaggtgccac 2400

tcccactgtc ctttcctaat aaaatgagga aattgcatcg cattgtctga gtaggtgtca 2460

ttctattctg gggggtgggg tggggcagga cagcaagggg gaggattggg aagacaatag 2520

caggcatgct ggggatgcgg tgggctctat ggcttctgag gcggaaagaa ccagctgggg 2580

ctctaggggg tatccccacg cgccctgtag cggcgcatta agcgcggcgg gtgtggtggt 2640

tacgcgcagc gtgaccgcta cacttgccag cgccctagcg cccgctcctt tcgctttctt 2700

cccttccttt ctcgccacgt tcgccggctt tccccgtcaa gctctaaatc gggggctccc 2760

tttagggttc cgatttagtg ctttacggca cctcgacccc aaaaaacttg attagggtga 2820

tggttcacgt agtgggccat cgccctgata gacggttttt cgccctttga cgttggagtc 2880

cacgttcttt aatagtggac tcttgttcca aactggaaca acactcaacc ctatctcggt 2940

ctattctttt gatttataag ggattttgcc gatttcggcc tattggttaa aaaatgagct 3000

gatttaacaa aaatttaacg cgaattaatt ctgtggaatg tgtgtcagtt agggtgtgga 3060

aagtccccag gctccccagc aggcagaagt atgcaaagca tgcatctcaa ttagtcagca 3120

accaggtgtg gaaagtcccc aggctcccca gcaggcagaa gtatgcaaag catgcatctc 3180

aattagtcag caaccatagt cccgccccta actccgccca tcccgcccct aactccgccc 3240

agttccgccc attctccgcc ccatggctga ctaatttttt ttatttatgc agaggccgag 3300

gccgcctctg cctctgagct attccagaag tagtgaggag gcttttttgg aggcctaggc 3360

ttttgcaaaa agctcccggg agcttgtata tccattttcg gatctgatca agagacagga 3420

tgaggatcgt ttcgcatgat tgaacaagat ggattgcacg caggttctcc ggccgcttgg 3480

gtggagaggc tattcggcta tgactgggca caacagacaa tcggctgctc tgatgccgcc 3540

gtgttccggc tgtcagcgca ggggcgcccg gttctttttg tcaagaccga cctgtccggt 3600

gccctgaatg aactgcagga cgaggcagcg cggctatcgt ggctggccac gacgggcgtt 3660

ccttgcgcag ctgtgctcga cgttgtcact gaagcgggaa gggactggct gctattgggc 3720

gaagtgccgg ggcaggatct cctgtcatct caccttgctc ctgccgagaa agtatccatc 3780

atggctgatg caatgcggcg gctgcatacg cttgatccgg ctacctgccc attcgaccac 3840

caagcgaaac atcgcatcga gcgagcacgt actcggatgg aagccggtct tgtcgatcag 3900

gatgatctgg acgaagagca tcaggggctc gcgccagccg aactgttcgc caggctcaag 3960

gcgcgcatgc ccgacggcga ggatctcgtc gtgacccatg gcgatgcctg cttgccgaat 4020

atcatggtgg aaaatggccg cttttctgga ttcatcgact gtggccggct gggtgtggcg 4080

gaccgctatc aggacatagc gttggctacc cgtgatattg ctgaagagct tggcggcgaa 4140

tgggctgacc gcttcctcgt gctttacggt atcgccgctc ccgattcgca gcgcatcgcc 4200

ttctatcgcc ttcttgacga gttcttctga gcgggactct ggggttcgcg aaatgaccga 4260

ccaagcgacg cccaacctgc catcacgaga tttcgattcc accgccgcct tctatgaaag 4320

gttgggcttc ggaatcgttt tccgggacgc cggctggatg atcctccagc gcggggatct 4380

catgctggag ttcttcgccc accccaactt gtttattgca gcttataatg gttacaaata 4440

aagcaatagc atcacaaatt tcacaaataa agcatttttt tcactgcatt ctagttgtgg 4500

tttgtccaaa ctcatcaatg tatcttatca tgtctgtata ccgtcgacct ctagctagag 4560

cttggcgtaa tcatggtcat agctgtttcc tgtgtgaaat tgttatccgc tcacaattcc 4620

acacaacata cgagccggaa gcataaagtg taaagcctgg ggtgcctaat gagtgagcta 4680

actcacatta attgcgttgc gctcactgcc cgctttccag tcgggaaacc tgtcgtgcca 4740

gctgcattaa tgaatcggcc aacgcgcggg gagaggcggt ttgcgtattg ggcgctcttc 4800

cgcttcctcg ctcactgact cgctgcgctc ggtcgttcgg ctgcggcgag cggtatcagc 4860

tcactcaaag gcggtaatac ggttatccac agaatcaggg gataacgcag gaaagaacat 4920

gtgagcaaaa ggccagcaaa aggccaggaa ccgtaaaaag gccgcgttgc tggcgttttt 4980

ccataggctc cgcccccctg acgagcatca caaaaatcga cgctcaagtc agaggtggcg 5040

aaacccgaca ggactataaa gataccaggc gtttccccct ggaagctccc tcgtgcgctc 5100

tcctgttccg accctgccgc ttaccggata cctgtccgcc tttctccctt cgggaagcgt 5160

ggcgctttct catagctcac gctgtaggta tctcagttcg gtgtaggtcg ttcgctccaa 5220

gctgggctgt gtgcacgaac cccccgttca gcccgaccgc tgcgccttat ccggtaacta 5280

tcgtcttgag tccaacccgg taagacacga cttatcgcca ctggcagcag ccactggtaa 5340

caggattagc agagcgaggt atgtaggcgg tgctacagag ttcttgaagt ggtggcctaa 5400

ctacggctac actagaagaa cagtatttgg tatctgcgct ctgctgaagc cagttacctt 5460

cggaaaaaga gttggtagct cttgatccgg caaacaaacc accgctggta gcggtggttt 5520

ttttgtttgc aagcagcaga ttacgcgcag aaaaaaagga tctcaagaag atcctttgat 5580

cttttctacg gggtctgacg ctcagtggaa cgaaaactca cgttaaggga ttttggtcat 5640

gagattatca aaaaggatct tcacctagat ccttttaaat taaaaatgaa gttttaaatc 5700

aatctaaagt atatatgagt aaacttggtc tgacagttac caatgcttaa tcagtgaggc 5760

acctatctca gcgatctgtc tatttcgttc atccatagtt gcctgactcc ccgtcgtgta 5820

gataactacg atacgggagg gcttaccatc tggccccagt gctgcaatga taccgcgaga 5880

cccacgctca ccggctccag atttatcagc aataaaccag ccagccggaa gggccgagcg 5940

cagaagtggt cctgcaactt tatccgcctc catccagtct attaattgtt gccgggaagc 6000

tagagtaagt agttcgccag ttaatagttt gcgcaacgtt gttgccattg ctacaggcat 6060

cgtggtgtca cgctcgtcgt ttggtatggc ttcattcagc tccggttccc aacgatcaag 6120

gcgagttaca tgatccccca tgttgtgcaa aaaagcggtt agctccttcg gtcctccgat 6180

cgttgtcaga agtaagttgg ccgcagtgtt atcactcatg gttatggcag cactgcataa 6240

ttctcttact gtcatgccat ccgtaagatg cttttctgtg actggtgagt actcaaccaa 6300

gtcattctga gaatagtgta tgcggcgacc gagttgctct tgcccggcgt caatacggga 6360

taataccgcg ccacatagca gaactttaaa agtgctcatc attggaaaac gttcttcggg 6420

gcgaaaactc tcaaggatct taccgctgtt gagatccagt tcgatgtaac ccactcgtgc 6480

acccaactga tcttcagcat cttttacttt caccagcgtt tctgggtgag caaaaacagg 6540

aaggcaaaat gccgcaaaaa agggaataag ggcgacacgg aaatgttgaa tactcatact 6600

cttccttttt caatattatt gaagcattta tcagggttat tgtctcatga gcggatacat 6660

atttgaatgt atttagaaaa ataaacaaat aggggttccg cgcacatttc cccgaaaagt 6720

gccacctgac gtc 6733

## Description

### TECHNICAL FIELD

The present invention relates to an adamantane derivative and a pharmaceutically acceptable salt thereof. The present invention further relates to a pharmaceutical composition containing the compound and a method of treating or preventing a disease by using the compound.

### BACKGROUND ART

ATP-sensitive K⁺ channel (K_{ATP} channel) is an inwardly rectifying K⁺ channel connecting intracellular metabolism and cell-membrane excitation and known to have a heterologous octamer structure constituted of a sulfonyl urea receptor (SUR) belonging to an ABC protein family and two-span transmembrane subunits Kir6.1 and Kir6.2. The activity of the K_{ATP} channel is controlled by various types of K⁺ channel openers, inhibitors or intracellular nucleotides. All of these have active sites in SUR subunits. It has been reported that the reactions of them differ depending on the subtype of SUR (NPL 1).

Some of adamantane derivatives having a cage type structure are used as medical drugs. Amantadine is used as antiviral drug and a therapeutic agent for Parkinson's disease. Memantine hydrochloride has been approved as a therapeutic agent for moderate/severe Alzheimer's dementia even in Japan. Memantine is a noncompetitive NMDA-receptor inhibitor and reported to have an action mechanism which prevents neuronal cell death of excessive glutamic acid release caused by ischemia (NPL 2).

Adamantane derivatives having an activity as a medical drug are reported in several literatures (PTLs 1 to 3).

### CITATION LIST

### PATENT LITERATURE

PTL 1: National Publication of International Patent Application No. 2011-529057
PTL 2: Japanese Patent Laid-Open No. 2010-522203
PTL 3: National Publication of International Patent Application No. 2009-508956

### NON PATENT LITERATURE

NPL 1: Folia pharmacologica Japonica, 126, 311 to 316 (2005)
NPL 2: Folia pharmacologica Japonica, 124, 145 to 151 (2004)

### SUMMARY OF INVENTION

### TECHNICAL PROBLEM

A therapeutic and prophylactic method exerting a sufficient effect on a cognitive disease or disorder such as Alzheimer's disease has not yet been established, and thus, development of a novel therapeutic and prophylactic agent different in action mechanism from existing medicinal agents has been desired. In addition, development of a novel therapeutic and prophylactic agent for diabetes has been strongly desired.

In one aspect, an object of the present invention is to provide a pharmaceutical composition for use in treating or preventing a cognitive disease or disorder. Another object of the present invention is to provide a method of treating or preventing a cognitive disease or disorder by using a predetermined adamantane derivative.

In one aspect, an object of the present invention is to provide a pharmaceutical composition for use in treating or preventing diabetes or a diabetic complication. Another object of the present invention is to provide a method of treating or preventing diabetes or a diabetic complication by using a predetermined adamantane derivative.

ATP-sensitive K⁺ channel (K_{ATP} channel) contains subunits Kir6.1 and Kir6.2 and is known to serve as an active site of e.g., an anti-diabetic drug.

In one aspect, an object of the present invention is to provide a channel inhibitor against Kir6.1 or a channel inhibitor against Kir6.2 of the K_{ATP} channel. Another object of the present invention is to provide a pharmaceutical composition for use in treating or preventing a disease involving Kir6.1 channel or Kir6.2 channel of the K_{ATP} channel. Another object of the present invention is to provide a method of treating or preventing a disease involving Kir6.1 channel or Kir6.2 channel of the K_{ATP} channel, by using a predetermined adamantane derivative.

### SOLUTION TO PROBLEM

The present inventors conducted intensive studies with a view to attaining the aforementioned objects. As a result, they found that an adamantane derivative has a Kir6.2 channel inhibitory activity, a Kir6.1 channel inhibitory activity, a therapeutic effect for a cognitive disease or disorder and a hypoglycemic effect. Based on the finding, the present invention was accomplished. In the specification, the following inventions set forth in [1-1] to [1-20] are disclosed.
[1-1] A compound represented by Formula (I): 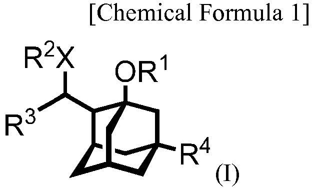
   wherein R¹ represents a hydrogen atom or (C₁₋₆ alkyl)carbonyl optionally substituted with one or more halogen atoms;
   R² represents a hydrogen atom or (C₁₋₆ alkyl)carbonyl optionally substituted with one or more halogen atoms;
   X represents O or NR⁵;
   R³ represents phenyl optionally substituted with one or more substituents selected from X¹, 5- or 6-membered heteroaryl optionally substituted with one or more substituents selected from X¹, or COOR⁶;
   R⁴ represents a hydrogen atom, a halogen atom, azido, -OR⁷ or -NHR⁸;
   R⁵ represents a hydrogen atom or C₁₋₆ alkyl;
   R⁶ represents a hydrogen atom or C₁₋₆ alkyl;
   R⁷ represents a hydrogen atom, C₁₋₆ alkyl, C₁₋₆ alkoxy-C₁₋₆ alkyl or (C₁₋₆ alkyl)carbonyl optionally substituted with one or more halogen atoms;
   R⁸ represents a hydrogen atom, C₁₋₆ alkyl or (C₁₋₆ alkyl)carbonyl optionally substituted with one or more halogen atoms; and
   X¹ represents C₁₋₆ alkyl, a halogen atom, C₁₋₆ alkoxy, nitro or cyano,
   an enantiomer thereof, a diastereomer thereof, or a pharmaceutically acceptable salt thereof.
[1-2] The compound, an enantiomer thereof, a diastereomer thereof, or a pharmaceutically acceptable salt thereof according to [1-1], wherein R⁴ represents a chlorine atom or azido.
[1-3] The compound, an enantiomer thereof, a diastereomer thereof, or a pharmaceutically acceptable salt thereof according to [1-1] or [1-2], wherein R¹ represents trifluoroacetyl.
[1-4] The compound, an enantiomer thereof, a diastereomer thereof, or a pharmaceutically acceptable salt thereof according to any one of [1-1] to [1-3], wherein R² represents (C₁₋₆ alkyl)carbonyl optionally substituted with one or more halogen atoms.
[1-5] The compound, an enantiomer thereof, a diastereomer thereof, or a pharmaceutically acceptable salt thereof according to [1-4], wherein R² represents trifluoroacetyl.
[1-6] The compound, an enantiomer thereof, a diastereomer thereof, or a pharmaceutically acceptable salt thereof according to any one of [1-1] to [1-5], wherein R³ represents phenyl optionally substituted with one or more substituents selected from X¹ or pyridyl optionally substituted with one or more substituents selected from X¹.
[1-7] The compound, an enantiomer thereof, a diastereomer thereof, or a pharmaceutically acceptable salt thereof according to [1-1], selected from
   (R)-((1R,2S,3R,5R,7S)-5-azido-1-hydroxyadamantan-2-yl)(phenyl)methyl acetate;
   ethyl (S)-2-acetamido-2-((1R,2S,3R,5R,7R)-5-chloro-1-hydroxyadamantan-2-yl)acetate;
   ethyl (R)-2-acetamido-2-((1R,2S,3R,5R,7R)-5-chloro-1-hydroxyadamantan-2-yl)acetate;
   (1R,2S,3R,5R,7R)-5-chloro-2-((S)-2-methoxy-2-oxo-1-(2,2,2-trifluoroacetamido)ethyl)adamantan-1-yl 2,2,2-trifluoroacetate;
   (1S,2R,3S,5S,7S)-5-chloro-2-((R)-phenyl(2,2,2-trifluoroacetamido)methyl)adamantan-1-yl 2,2,2-trifluoroacetate;
   (S)-2-amino-2-((1R,2S,3R,5R,7S)-1,5-dihydroxyadamantan-2-yl)acetic acid;
   N-((R)-((1S,2R,3S,5S,7S)-5-chloro-1-hydroxyadamantan-2-yl)(phenyl)methyl)-2,2,2-trifluoroacetamide;
   (1S,2R,3S,5R,7S)-2-((R)-phenyl(2,2,2-trifluoroacetamido)methyl)adamantan-1-yl 2,2,2-trifluoroacetate;
   (1S,2R,3S,5S,7R)-5-(2-methoxyethoxy)-2-((R)-phenyl(2,2,2-trifluoroacetamido)methyl)adamantan-1-yl 2,2,2-trifluoroacetate;
   N-((R)-((1S,2R,3S,5S,7S)-5-chloro-1-hydroxyadamantan-2-yl)(pyridin-3-yl)methyl)-2,2,2-trifluoroacetamide;
   2,2,2-trifluoro-N-((R)-((1S,2R,3S,5R,7S)-1-hydroxyadamantan-2-yl)(phenyl)methyl)acetamide; and
   (1S,2R,3S,5S,7R)-5-methoxy-2-((R)-phenyl(2,2,2-trifluoroacetamido)methyl)adamantan-1-yl 2,2,2-trifluoroacetate,
   or an enantiomer thereof, a diastereomer thereof, or a pharmaceutically acceptable salt thereof.
[1-8] A pharmaceutical composition containing the compound, an enantiomer thereof, a diastereomer thereof, or a pharmaceutically acceptable salt thereof according to any one of [1-1] to [1-7].
[1-9] The pharmaceutical composition according to [1-8], for use in treating or preventing a cognitive disease or disorder.
[1-10] The pharmaceutical composition according to [1-9], wherein the cognitive disease or disorder is selected from Alzheimer's dementia, cerebrovascular dementia, Lewy body dementia, frontotemporal dementia, Parkinson's disease, a mental disease and a neurodegenerative disease.
[1-11] The pharmaceutical composition according to [1-8] for use in treating or preventing diabetes or a diabetic complication.
[1-12] A Kir6.2 channel inhibitor containing the compound, an enantiomer thereof, a diastereomer thereof, or a pharmaceutically acceptable salt thereof according to any one of [1-1] to [1-7].
[1-13] The Kir6.1 channel inhibitor containing the compound, an enantiomer thereof, a diastereomer thereof, or a pharmaceutically acceptable salt thereof according to any one of [1-1] to [1-7].
[1-14] A method of treating or preventing a cognitive disease or disorder, comprising administering a therapeutically effective amount of the compound, an enantiomer thereof, a diastereomer thereof, or a pharmaceutically acceptable salt thereof according to any one of [1-1] to [1-7] to a subject.
[1-15] The method according to [1-14], wherein the cognitive disease or disorder is selected from Alzheimer's dementia, cerebrovascular dementia, Lewy body dementia, frontotemporal dementia, Parkinson's disease, a mental disease and a neurodegenerative disease.
[1-16] The method of treating or preventing diabetes or a diabetic complication, comprising administering a therapeutically effective amount of the compound, an enantiomer thereof, a diastereomer thereof, or a pharmaceutically acceptable salt thereof according to any one of [1-1] to [1-7] to a subject.
[1-17] A method of treating or preventing a disease involving Kir6.1 channel or Kir6.2 channel, comprising administering a therapeutically effective amount of the compound, an enantiomer thereof, a diastereomer thereof, or a pharmaceutically acceptable salt thereof according to any one of [1-1] to [1-7] to a subject as a Kir6.1 channel inhibitor or a Kir6.2 channel inhibitor.
[1-18] The method according to [1-17], wherein the disease involving Kir6.1 channel or Kir6.2 channel is a cognitive disease or disorder, or diabetes or a diabetic complication.
[1-19] The method according to [1-17] or [1-18], wherein the disease involving Kir6.1 channel or Kir6.2 channel is a cognitive disease or disorder selected from Alzheimer's dementia, cerebrovascular dementia, Lewy body dementia, frontotemporal dementia, Parkinson's disease, a mental disease and a neurodegenerative disease.
[1-20] The method according to [1-17] or [1-18], wherein the disease involving Kir6.1 channel or Kir6.2 channel is diabetes or a diabetic complication.

In the specification, the following inventions set forth in [2-1] to [2-12] are disclosed.
[2-1] A compound represented by Formula (I): 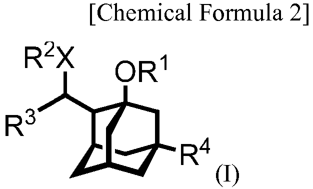
   wherein R¹ represents a hydrogen atom or (C₁₋₆ alkyl)carbonyl optionally substituted with one or more halogen atoms;
   R² represents a hydrogen atom or (C₁₋₆ alkyl)carbonyl optionally substituted with one or more halogen atoms;
   X represents O or NR⁵;
   R³ represents phenyl optionally substituted with one or more substituents selected from X¹, or COOR⁶;
   R⁴ represents a hydrogen atom, a halogen atom, azido,-OR⁷ or -NHR⁸;
   R⁵ represents a hydrogen atom or C₁₋₆ alkyl;
   R⁶ represents a hydrogen atom or C₁₋₆ alkyl;
   R⁷ represents a hydrogen atom, C₁₋₆ alkyl or (C₁₋₆ alkyl)carbonyl optionally substituted with one or more halogen atoms;
   R⁸ represents a hydrogen atom, C₁₋₆ alkyl or (C₁₋₆ alkyl)carbonyl optionally substituted with one or more halogen atoms; and
   X¹ represents C₁₋₆ alkyl, a halogen atom, C₁₋₆ alkoxy, nitro or cyano,
   an enantiomer thereof, a diastereomer thereof, or a pharmaceutically acceptable salt thereof.
[2-2] The compound, an enantiomer thereof, a diastereomer thereof, or a pharmaceutically acceptable salt thereof according to [2-1], wherein R⁴ represents a chlorine atom or azido.
[2-3] The compound, an enantiomer thereof, a diastereomer thereof, or a pharmaceutically acceptable salt thereof according to [2-1] or [2-2], wherein R¹ represents trifluoroacetyl.
[2-4] The compound, an enantiomer thereof, a diastereomer thereof, or a pharmaceutically acceptable salt thereof according to any one of [2-1] to [2-3], wherein R² represents trifluoroacetyl.
[2-5] The compound, an enantiomer thereof, a diastereomer thereof, or a pharmaceutically acceptable salt thereof according to any one of [2-1] to [2-4], wherein R³ represents phenyl optionally substituted with one or more substituents selected from X¹.
[2-6] The compound, an enantiomer thereof, a diastereomer thereof, or a pharmaceutically acceptable salt thereof according to [2-1], selected from
   (R)-((1R,2S,3R,5R,7S)-5-azido-1-hydroxyadamantan-2-yl)(phenyl)methyl acetate;
   ethyl (S)-2-acetamido-2-((1R,2S,3R,5R,7R)-5-chloro-1-hydroxyadamantan-2-yl)acetate;
   ethyl (R)-2-acetamido-2-((lR,2S,3R,5R,7R)-5-chloro-l-hydroxyadamantan-2-yl)acetate;
   (1R,2S,3R,5R,7R)-5-chloro-2-((S)-2-methoxy-2-oxo-1-(2,2,2-trifluoroacetamido)ethyl)adamantan-1-yl 2,2,2-trifluoroacetate;
   (1S,2R,3S,5S,7S)-5-chloro-2-((R)-phenyl(2,2,2-trifluoroacetamido)methyl)adamantan-1-yl 2,2,2-trifluoroacetate; and
   (S)-2-amino-2-((1R,2S,3R,5R,7S)-1,5-dihydroxyadamantan-2-yl)acetic acid,
   or an enantiomer thereof, a diastereomer thereof, or a pharmaceutically acceptable salt thereof.
[2-7] A pharmaceutical composition containing the compound, an enantiomer thereof, a diastereomer thereof, or a pharmaceutically acceptable salt thereof according to any one of [2-1] to [2-6].
[2-8] The pharmaceutical composition according to [2-7], for use in treating or preventing a cognitive disease or disorder.
[2-9] The pharmaceutical composition according to [2-8], wherein the cognitive disease or disorder is selected from Alzheimer's dementia, cerebrovascular dementia, Lewy body dementia, frontotemporal dementia, Parkinson's disease, a mental disease and a neurodegenerative disease.
[2-10] The pharmaceutical composition according to [2-7] for use in treating or preventing diabetes or a diabetic complication.
[2-11] A Kir6.2 channel inhibitor containing the compound, an enantiomer thereof, a diastereomer thereof, or a pharmaceutically acceptable salt thereof according to any one of [2-1] to [2-6].
[2-12] The Kir6.1 channel inhibitor containing the compound, an enantiomer thereof, a diastereomer thereof, or a pharmaceutically acceptable salt thereof according to any one of [2-1] to [2-6].

### ADVANTAGEOUS EFFECTS OF INVENTION

In one aspect, the present invention provides a pharmaceutical composition for use in treating or preventing a cognitive disease or disorder. In another aspect, the present invention provides an inhibitor of Kir6.1 channel or Kir6.2 channel of K_{ATP} channel.

### BRIEF DESCRIPTION OF DRAWINGS

[Fig. 1] Fig. 1 is a graph showing CaMKII activity enhanced by the compound of the present invention in cells (Neuro2A cells) overexpressing Kir6.2 channel. All indications regarding significant difference is relative to a control (C: Kir6.2 expressing cells not treated with a drug) are marked. In the drawings of this application, the significant difference indicated by ** or ++ means P < 0.01; or significant difference indicated by + or * means P < 0.05.
[Fig. 2a] Fig. 2a shows (the results of) expression of Kir6.2 channel in N2A cells, which is checked by applying immunoblotting using an anti-Kir6.2 channel antibody to Kir6.2 channel-overexpressing cells. The case having a significant difference with a non-drug treatment group (-) is indicated by **.
[Fig. 2b] Fig. 2b shows the assay results by a whole-cell patch-clamp method, showing that TP-014 suppresses outward potassium current in Kir6.2 channel-overexpressing cells. The results show that TP-014 inhibits Kir6.2 channel and attenuates potassium current.
[Fig. 3a] Fig. 3a shows the assay results by a calcium imaging method, showing that the intracellular calcium concentration in Kir6.2 channel-overexpressing cells increases by administration of TP-014. A change of concentration-dependent calcium amount with time (four minutes) was checked in the cases treated with the compound of the present invention and memantine. The results show that TP-014 inhibits Kir6.2 channel and increases intracellular calcium concentration.
[Fig. 3b] Fig. 3b shows the assay results by a calcium imaging method, showing that intracellular calcium concentration in Kir6.2 channel-overexpressing cells increases by administration of TP-014. The calcium amounts were measured 4 minutes after treatment with memantine and the compound of the present invention. The group using Kir6.2 expressing cells (Neuro2A cells) is confirmed to have a significant difference from a non-drug treatment group (-). The results show that TP-014 inhibits Kir6.2 channel and increases the intracellular calcium concentration.
[Fig. 4a] Fig. 4a shows the results of the experiment in which TP-014 was administered to Alzheimer's disease model mice (APP23 mice) (12 month-old) for two months and analysis was made by the Y-maze method, showing that cognitive function is effectively improved.
[Fig. 4b] Fig. 4b shows the results of an experiment in which TP-014 was administered to Alzheimer's disease model mice (APP23 mice) (12 month-old) for two months and analysis was made by the Y-maze method, showing that cognitive function is effectively improved. The case having a significant difference between wild-type (WT) and APP23 mice in the ratio giving a correct answer (alteration) in memory learning is indicated by **; and the case having a significant difference between APP mice and a control (non-treated group) is indicated by ++.
[Fig. 4c] Fig. 4c is a graph showing the results of an experiment in which TP-014 was administered to Alzheimer's disease model mice (APP23 mice) (12 month-old) for two months and analysis was made by a new object recognition test method, showing that cognitive function is effectively improved. The case of Novel (new object) having a significant difference with Familiar (same object) in each mouse group is indicated by **
[Fig. 4d] Fig. 4d shows results of an experiment in which TP-014 was administered to Alzheimer's disease model mice (APP23 mice)(12 month-old) for two months and analysis was made by a fear conditioning test method, showing that cognitive function is effectively improved. In Retention trials, a case having a significant difference with WT is indicated by **; and a case having a significant difference with APP23 mice is indicated by +.
[Fig. 4e] Fig. 4e shows the results of an experiment in which TP-014 was administered to Alzheimer's disease model mice (APP23 mice) (12 month-old) for two months and analysis was made on long-term potentiation phenomenon (LTP) serving as an index of memory formation, by an electrophysiological technique.
[Fig. 4f] Fig. 4f shows the results of an experiment in which TP-014 was administered to Alzheimer's disease model mice (APP23 mice) (12 month-old) for two months and analysis was made on long-term potentiation phenomenon (LTP) serving as an index of memory formation, by an electrophysiological technique.
[Fig. 4g] Fig. 4g shows the results of an experiment in which TP-014 was administered to Alzheimer's disease model mice (APP23 mice) (12 month-old) for two months and analysis was made on long-term potentiation phenomenon (LTP) serving as an index of memory formation, by an electrophysiological technique. The case having a significant difference with WT is indicated by **; and the case having a significant difference with APP23 mice is indicated by ++ or +.
[Fig. 5a] Fig. 5a shows bands (image) obtained by electrophoresis of immunoblots, showing the results of phosphorylation of proteins analyzed by immunoblotting using antibodies against CaMKII, CaMKIV and ERK.
[Fig. 5b] Fig. 5b shows the results of quantitative analysis of the signal intensity of the bands (in Fig. 5a) obtained by electrophoresis of immunoblots. The case having a significant difference with WT(-) (non-treated group with a drug) is indicated by **; and the case having a significant difference with a group of APP23 mice not treated with a drug (-) is indicated by +.
[Fig. 5c] Fig. 5c shows bands (image) obtained by electrophoresis of immunoblots, showing the results of phosphorylation of proteins analyzed by immunoblotting using antibodies against CaMKII, CaMKIV and ERK.
[Fig. 5d] Fig. 5d shows the results of quantitative analysis of the signal intensity of the bands (in Fig. 5c) obtained by electrophoresis of immunoblots. The case having a significant difference with WT(-) (non-treated group with a drug) is indicated by **; and the case having a significant difference with a group of APP23 mice not treated with a drug (-) is indicated by ++.
[Fig. 6a] Fig. 6a shows the results of an experiment in which TP-014 was administered to neurodegenerative disease model mice, i.e., olfactory bulbectomized mice (OBX mice) for two months and analysis was made by the Y-maze method, showing that cognitive function is effectively improved.
[Fig. 6b] Fig. 6b shows the results of an experiment in which TP-014 was administered to neurodegenerative disease model mice, i.e., olfactory bulbectomized mice (OBX mice) for two months and analysis was made by a Y-maze method, showing that cognitive function is effectively improved. In OBX mice, the case having a significant difference in the ratio giving a correct answer (alternation) of memory learning with WT is indicated by **; and the case having a significant difference with a control (non-treated group) of OBX mice is indicated by ++.
[Fig. 6c] Fig. 6c shows the results of an experiment in which TP-014 was administered to neurodegenerative disease model mice, i.e., olfactory bulbectomized mice (OBX mice) for two months and analysis was made by a new object recognition test method, showing that cognitive function is effectively improved.The case of Novel (new object) having a significant difference with Familiar (familiar object) in each mouse group is indicated by **
[Fig. 6d] Fig. 6d shows the results of an experiment in which TP-014 was administered to neurodegenerative disease model mice, i.e., olfactory bulbectomized mice (OBX mice) for two months and analysis was made by a fear conditioning test method, showing that cognitive function is effectively improved. In Retention trials, a case having a significant difference with WT is indicated by **; and a case having a significant difference with OBX mice is indicated by +.
[Fig. 6e] Fig. 6e shows the results of an experiment in which TP-014 was administered to neurodegenerative disease model mice, i.e., olfactory bulbectomized mice (OBX mice) for two months and analysis was made on long-term potentiation phenomenon (LTP) serving as an index of memory formation, by an electrophysiological technique.
[Fig. 6f] Fig. 6f shows the results of an experiment in which TP-014 was administered to neurodegenerative disease model mice, i.e., olfactory bulbectomized mice (OBX mice) for two months and analysis was made on long-term potentiation phenomenon (LTP) serving as an index of memory formation, by an electrophysiological technique.
[Fig. 6g] Fig. 6g shows the results of an experiment in which TP-014 was administered to neurodegenerative disease model mice, i.e., olfactory bulbectomized mice (OBX mice) for two months and analysis was made on long-term potentiation phenomenon (LTP) serving as an index of memory formation, by an electrophysiological technique. The case having a significant difference with Sham is indicated by **; and the case having a significant difference with OBX mice by ++ or +.
[Fig. 7a] Fig. 7a shows bands (image) obtained by electrophoresis of immunoblots, showing the results of phosphorylation of proteins analyzed by immunoblotting using antibodies against CaMKII, CaMKIV and ERK.
[Fig. 7b] Fig. 7b shows the results of quantitative analysis of the signal intensity of the bands (in Fig. 7a) obtained by electrophoresis of immunoblots.
[Fig. 7c] Fig. 7c shows bands (image) obtained by electrophoresis of immunoblots, showing the results of phosphorylation of proteins analyzed by immunoblotting using antibodies against CaMKII, CaMKIV and ERK.
[Fig. 7d] Fig. 7d shows the results of quantitative analysis of the signal intensity of the bands (in Fig. 7c) obtained by electrophoresis of immunoblots. The case having a significant difference with WT(-)(non-treated group with a drug) is indicated by **; and the case having a significant difference with a group of OBX mice not treated with a drug (-) is indicated by ++.
[Fig. 8a] Fig. 8a shows a graph showing the results of the experiment in which TP-014 was administered to Kir6.2 channel deficient mice for two months and analysis was made by the Y-maze method, showing that cognitive function is effectively improved.
[Fig. 8b] Fig. 8b shows the results of an experiment in which TP-014 was administered to Kir6.2 channel deficient mice for two months and analysis was made by the Y-maze method, showing that cognitive function is effectively improved. The case having a significant difference between Kir6.2 deficient mice and WT in the ratio giving a correct answer (alteration) in memory learning is indicated by * or **
[Fig. 8c] Fig. 8c shows a graph showing the results of an experiment in which TP-014 was administered to Kir6.2 channel deficient mice for two months and analysis was made by a new object recognition test method, showing that cognitive function is effectively improved. The case of Novel (new object) having a significant difference with Familiar (familiar object) in each mouse group is indicated by **
[Fig. 8d] Fig. 8d shows a graph showing the results of an experiment in which TP-014 was administered to Kir6.2 channel deficient mice for two months and analysis was made by a fear conditioning test method, showing that cognitive function is effectively improved. In Retention trials, a case having a significant difference with WT is shown by *.
[Fig. 8e] Fig. 8e shows the results of an experiment in which TP-014 was administered to Kir6.2 channel deficient mice for two months and analysis was made on long-term potentiation phenomenon (LTP) serving as an index of memory formation, by an electrophysiological technique.
[Fig. 8f] Fig. 8f shows the results of an experiment in which TP-014 was administered to Kir6.2 channel deficient mice for two months and analysis was made on long-term potentiation phenomenon (LTP) serving as an index of memory formation, by an electrophysiological technique.
[Fig. 8g] Fig. 8g shows the results of an experiment in which TP-014 was administered to Kir6.2 channel deficient mice for two months and analysis was made on long-term potentiation phenomenon (LTP) serving as an index of memory formation, by an electrophysiological technique. The case having a significant difference with WT is indicated by ** or *
[Fig. 9a] Fig. 9a shows bands (image) obtained by electrophoresis of immunoblots, showing the results of phosphorylation of proteins analyzed by immunoblotting using antibodies against CaMKII, CaMKIV and ERK.
[Fig. 9b] Fig. 9b shows the results of quantitative analysis of the signal intensity of the bands (in Fig. 9a) obtained by electrophoresis of immunoblots. The case having a significant difference with WT(-)(non-treated group with a drug) is indicated by ** or*.
[Fig. 10] Fig. 10 shows staining results of brain slice sections of APP23 mouse, showing the effect of the compound of the present invention on Aβ aggregation.
[Fig. 11a] Fig. 11a shows the results of a test, which examined whether or not the compound of the present invention exerts an improvement effect on a depression-like symptom of OBX mice. The case having a significant difference with Sham (control group) is indicated by **; and the case having a significant difference with OBX mice is indicated by +.
[Fig. 11b] Fig. 11 shows the results of a test, which examined whether or not the compound of the present invention exerts an improvement effect on a depression-like symptom of OBX mice. The case having a significant difference with Sham (control group) is indicated by ** and the significant difference with OBX mice is indicated by +.
[Fig. 12a] Fig. 12a shows the results of a test, which examined whether or not the compound of the present invention exerts an improvement effect on a depression through inhibitory action of the Kir6.1 channel. The case having a significant difference with WT (control group) is indicated by **.
[Fig. 12b] Fig. 12b shows the results of a test, which examined whether or not the compound of the present invention exerts an improvement effect on a depression through inhibitory action of the Kir6.1 channel. The case having a significant difference with WT (control group) is indicated by **.
[Fig. 13a] Fig. 13a shows the results of a test, which examined whether or not the compound of the present invention inhibits the Kir6.1 channel and activates CaMKIV, thereby exerting improvement effect on a depression. The case having a significant difference with WT (control group) is indicated by **.
[Fig. 13b] Fig. 13b shows the results of a test, which examined whether or not the compound of the present invention inhibits the Kir6.1 channel and activates CaMKIV, thereby exerting improvement effect on a depression-like symptom. The case having a significant difference with WT (control group) is indicated by **.
[Fig. 14] Fig. 14 shows the results of a test, which examined whether or not the compound of the present invention has a hypoglycemic effect. The term "weeks" refers to the time period of chronic administration, the case having a significant difference between ob/ob (saline) in each week is indicated by *.

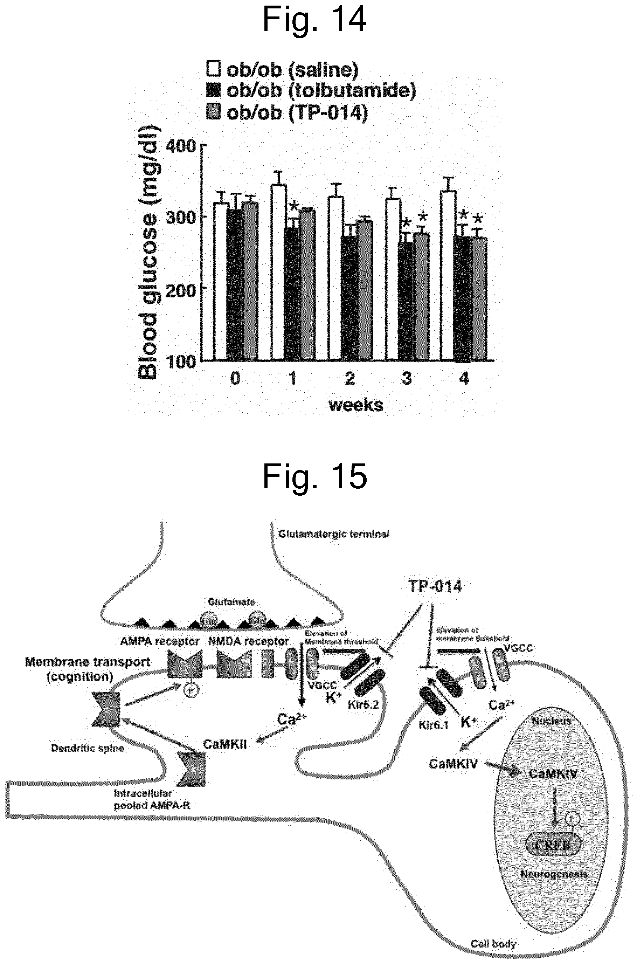

[Fig. 15] Fig. 15 shows an illustration showing the action mechanism of TP-014. When the Kir6.2 channel localized in the spine is inhibited, potassium present within cells cannot flow out, increasing the threshold of cell membrane. As a result, calcium outside cells is accelerated to flow into the cells, activates CaMKII, activates GluAl (Ser-831)(AMPA acceptor) downstream of CaMKII and presumably improves cognitive function. TP-014 similarly inhibits the Kir6.1 channel localizing in the nerve cell body. As a result, calcium flows into cells in the similar mechanism. Calcium influx activates CaMKIV, activates CREB (Ser-133) and presumably induces neurogenesis to improve depression. TP-014 is a novel cognitive function improving drug having both a cognitive function improvement effect (core symptom of Alzheimer's disease) based on a Kir6.2 channel inhibitory action and a depression improvement effect (peripheral symptom of Alzheimer's disease) based on a Kir6.1 channel inhibitory action.

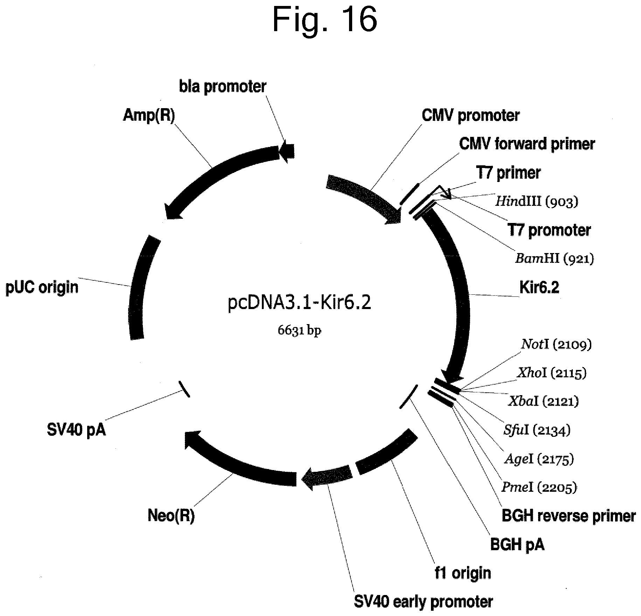

[Fig. 16] Fig. 16 shows the structure of a plasmid vector: pcDNA3.1-Kir6.2.

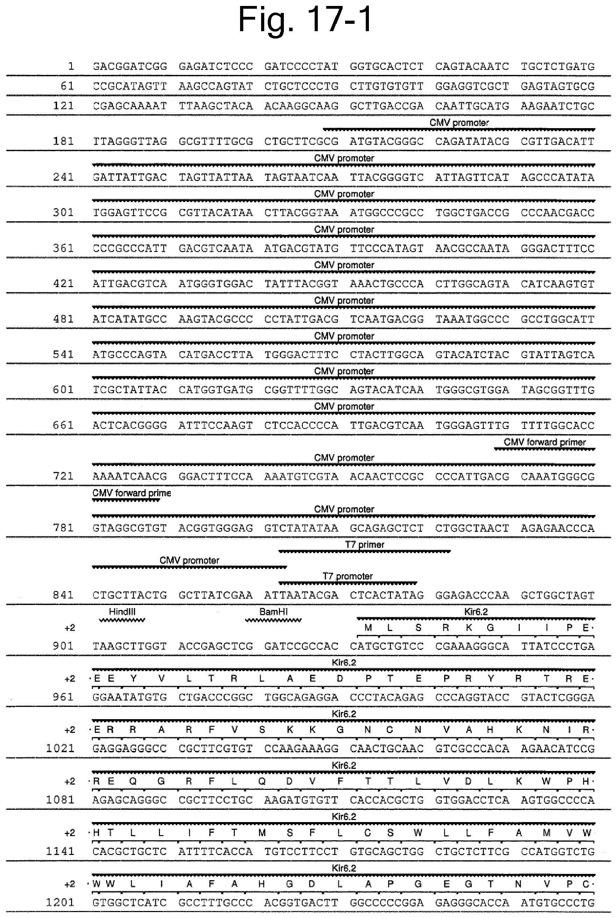

[Fig. 17-1] Fig. 17-1 shows the sequence of a plasmid vector: pcDNA3.1-Kir6.2.

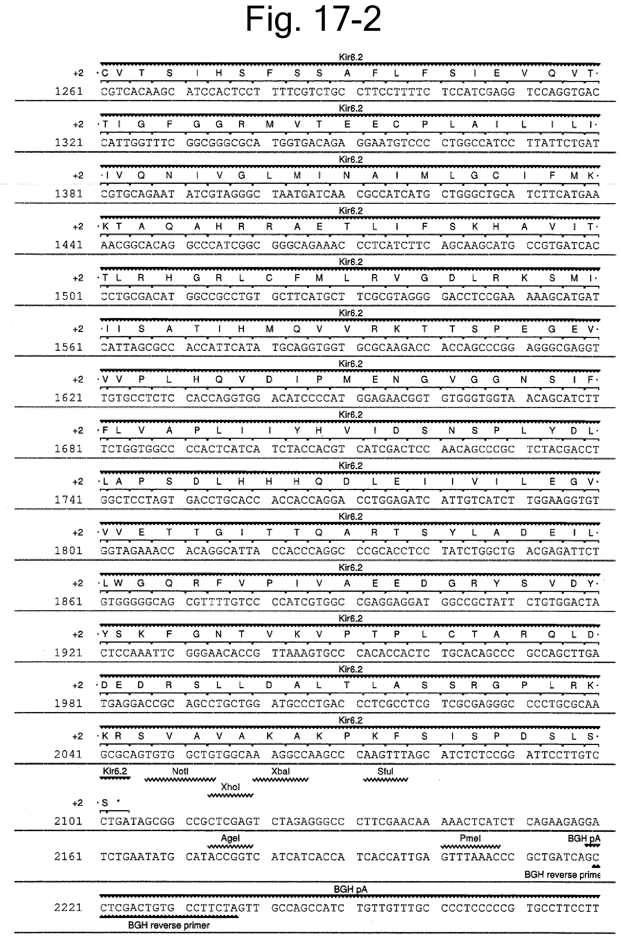

[Fig. 17-2] Fig. 17-2 shows the sequence of plasmid vector: pcDNA3.1-Kir6.2.

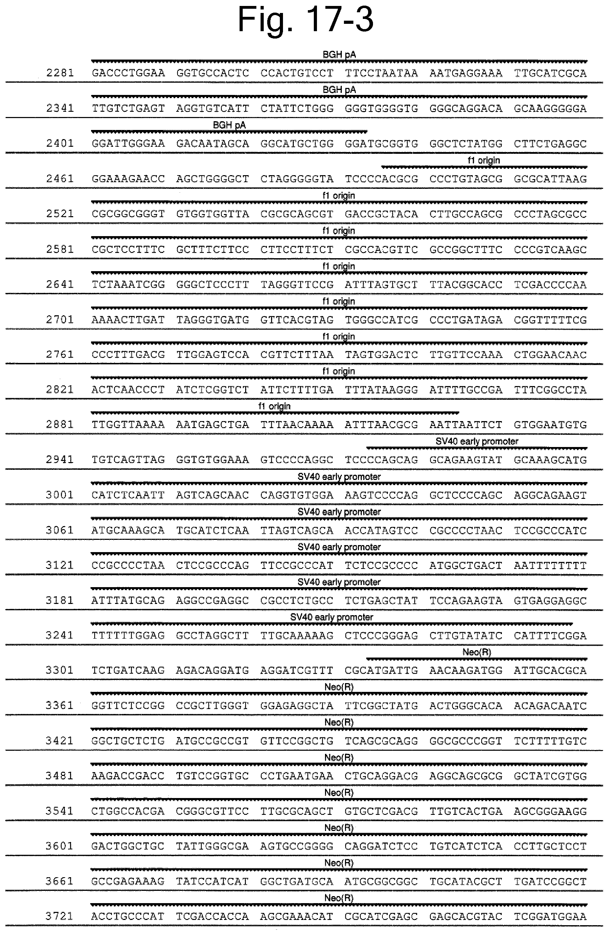

[Fig. 17-3] Fig. 17-3 shows the sequence of plasmid vector: pcDNA3.1-Kir6.2.

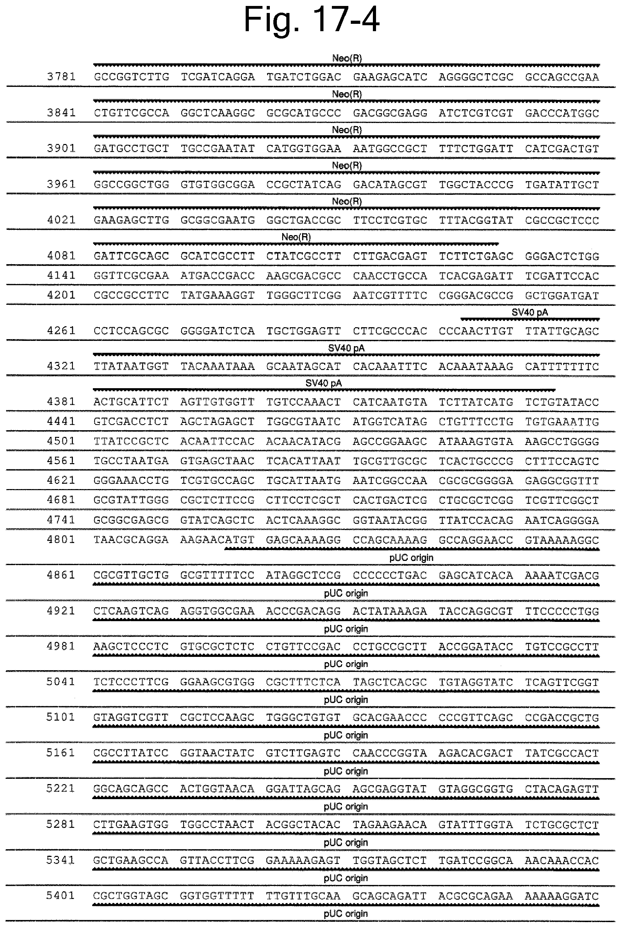

[Fig. 17-4] Fig. 17-4 shows the sequence of plasmid vector: pcDNA3.1-Kir6.2.

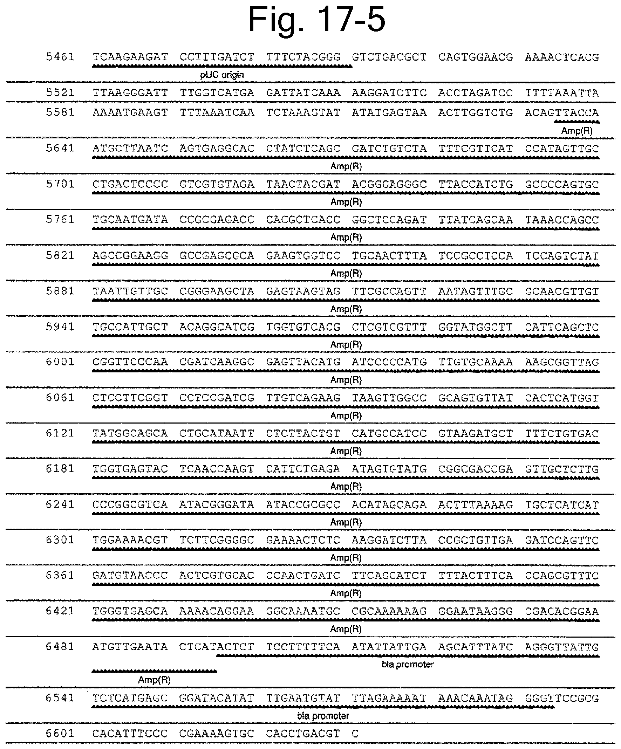

[Fig. 17-5] Fig. 17-5 shows the sequence of plasmid vector: pcDNA3.1-Kir6.2.
[Fig. 18a] Fig. 18a is a graph showing CaMKIV activity enhanced by the compound of the present invention in cells (Neuro2A cells) overexpressing Kir6.1 channel. All cases having a significant difference with a control (C: Kir6.1 expressing cells not treated with a drug) are marked.
[Fig. 18b] Fig. 18b shows the results of expression of Kir6.1 channel in N2A cells, which is checked by applying immunoblotting using an anti-Kir6.1 channel antibody to Kir6.1 channel-overexpressing cells. The case having a significant difference with a non-drug treatment group (-) is indicated by **.
[Fig. 18c] Fig. 18c shows the results of potassium current flowing out from cells outside the cells checked by using Kir6.1 channel-overexpressing cells and measured by an ordinary patch-clamp method.
[Fig. 19a] Fig. 19a shows results on vulnerability of mice groups (used in a test) to anxiety, analyzed by the elevated plus-maze method. With respect to the retention time of mice on the open arm, the case having a significant difference with WT(-) is indicated by ** or *; and the case having a significant difference with WT (CORT) is indicated by ++.
[Fig. 19b] Fig. 19b shows a photograph of the apparatus used in the elevated plus-maze method.
[Fig. 19c] Fig. 19c shows the results of the light/dark test method. The case having a significant difference with WT(-) is indicated by **; and the case having a significant difference with WT (CORT) is indicated by ++.
[Fig. 19d] Fig. 19d shows a photograph of the apparatus used in the light/dark test method.
[Fig. 19e] Fig. 19e shows the test results of the marble burying method. The case having a significant difference with WT(-) is indicated by **; and the case having a significant difference with WT (CORT) is indicated by +.
[Fig. 19f] Fig. 19f shows a photograph of the apparatus used in the marble burying method.
[Fig. 19g] Fig. 19g shows test results of the open field method. The case having a significant difference with WT(-) is indicated by **; and the case having a significant difference with WT (CORT) is indicated by ++.
[Fig. 19h] Fig. 19h shows a photograph of the apparatus used in the open field method.
[Fig. 19i] Fig. 19i test results of the fear conditioning test method. The case having a significant difference with WT(-) is indicated by ** or *; and the case having a significant difference with WT (CORT) is indicated by ++.
[Fig. 20] Fig. 20 shows the structure of a plasmid vector: pcDNA3.1-Kir6.1.

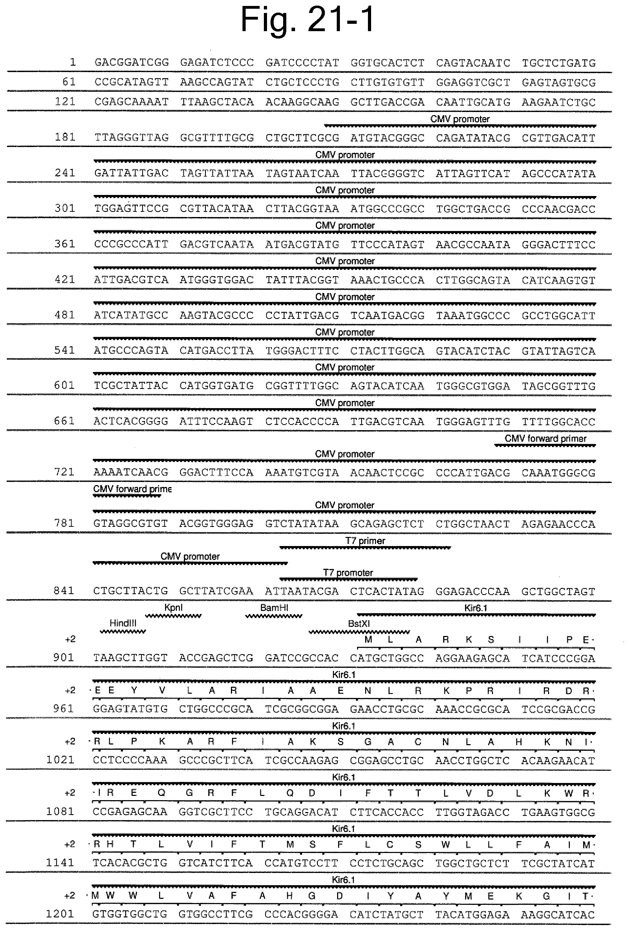

[Fig. 21-1] Fig. 21-1 shows the sequence of a plasmid vector: pcDNA3.1-Kir6.1.

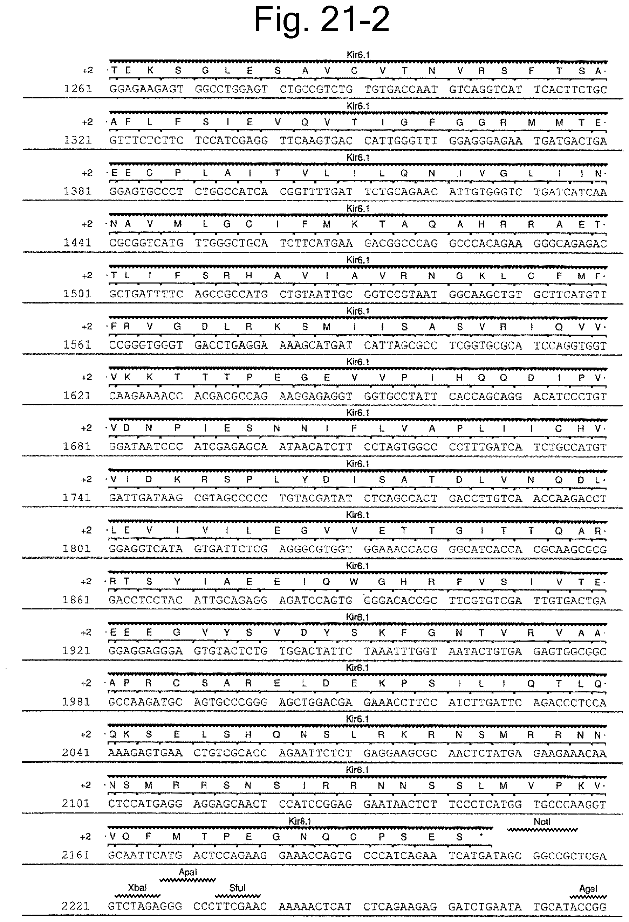

[Fig. 21-2] Fig. 21-2 shows the sequence of a plasmid vector: pcDNA3.1-Kir6.1.

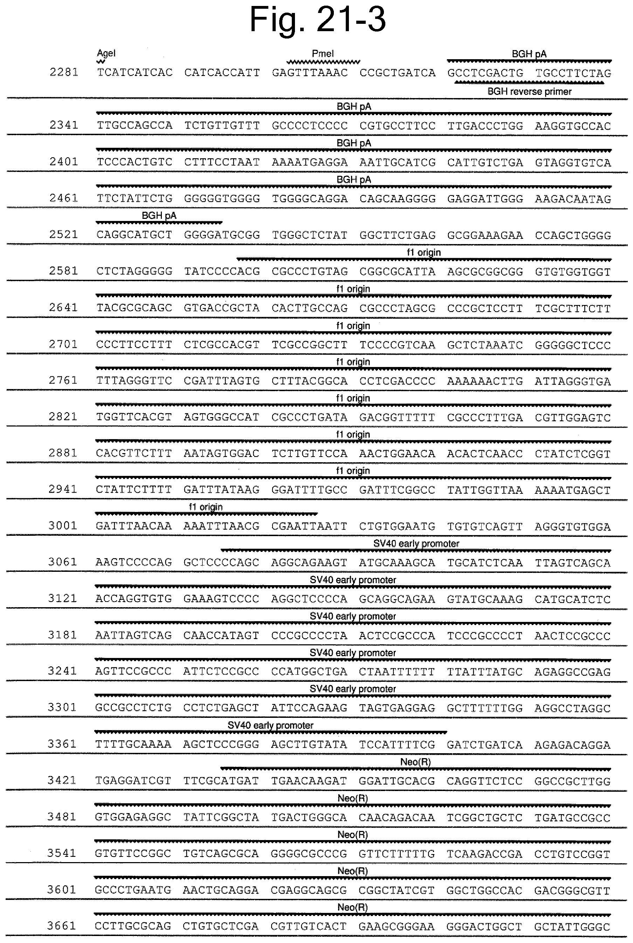

[Fig. 21-3] Fig. 21-3 shows the sequence of a plasmid vector: pcDNA3.1-Kir6.1.

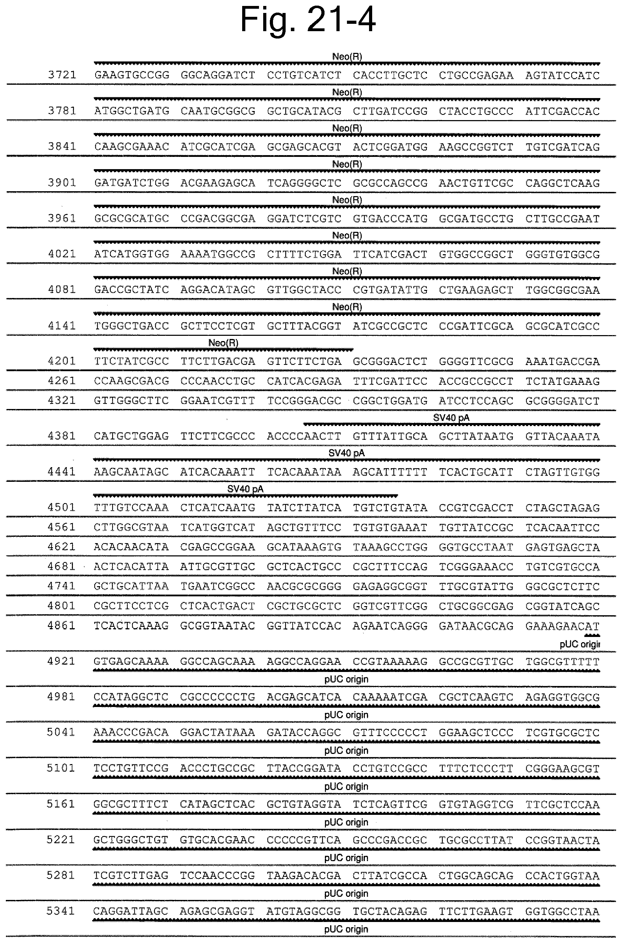

[Fig. 21-4] Fig. 21-4 shows the sequence of a plasmid vector: pcDNA3.1-Kir6.1.

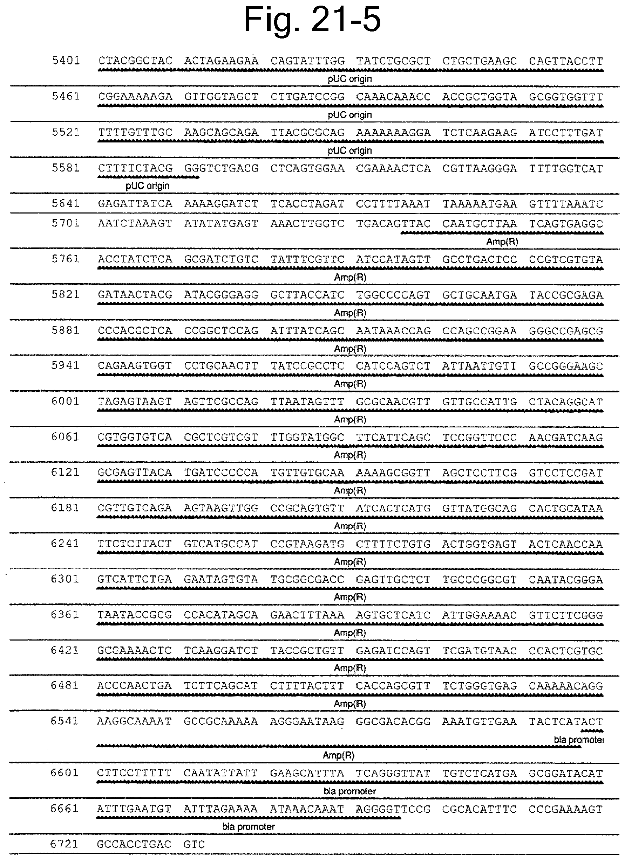

[Fig. 21-5] Fig. 21-5 shows the sequence of a plasmid vector: pcDNA3.1-Kir6.1.

### DESCRIPTION OF EMBODIMENTS

Now, the present invention will be more specifically described below.

According to one aspect of the present invention, there is provided a pharmaceutical composition for treating or preventing a cognitive disease or disorder, containing a compound represented by Formula (I), an enantiomer thereof, a diastereomer thereof, or a pharmaceutically acceptable salt thereof. More specifically, the compound of the present invention includes the compound represented by the following Formulas (I) and (II).

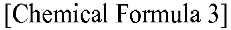

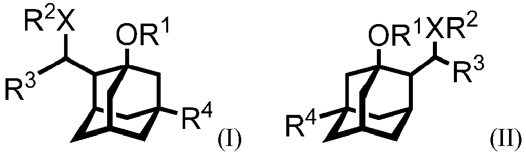

In the specification, "C₁₋₆ alkyl" refers to a linear, branched, cyclic or partial cyclic alkyl group having 1 to 6 carbon atoms. Examples thereof include methyl, ethyl, n-propyl, i-propyl, n-butyl, s-butyl, i-butyl, t-butyl, n-pentyl, 3-methylbutyl, 2-methylbutyl, 1-methylbutyl, 1-ethylpropyl, n-hexyl, 4-methylpentyl, 3-methylpentyl, 2-methylpentyl, 1-methylpentyl, 3-ethylbutyl and 2-ethylbutyl, cyclopropyl, cyclobutyl, cyclopentyl, cyclohexyl and cyclopropylmethyl. For example, C₁₋₄ alkyl and C₁₋₃ alkyl are also included.

In the specification, "C₁₋₆ alkoxy" refers to an alkyloxy group [-O-(C₁₋₆ alkyl)] having an alkyl group having 1 to 6 carbon atoms already defined. Examples thereof include methoxy, ethoxy, n-propoxy, i-propoxy, n-butoxy, s-butoxy, i-butoxy, t-butoxy, n-pentoxy, 3-methylbutoxy, 2-methylbutoxy, 1-methylbutoxy, 1-ethylpropoxy, n-hexyloxy, 4-methylpentoxy, 3-methylpentoxy, 2-methylpentoxy, 1-methylpentoxy, 3-ethylbutoxy, cyclopentyloxy, cyclohexyloxy and cyclopropylmethyloxy. For example, C₁₋₄ alkoxy and C₁₋₃ alkoxy are also included. In the specification, "C₁₋₄ alkoxy" include, for example, C₁₋₃ alkoxy.

In the specification, "azido" refers to -N₃.

In the specification, "(C₁₋₆ alkyl)carbonyl" refers to an alkylcarbonyl group having a C₁₋₆ alkyl group already defined. Examples thereof include methylcarbonyl(acetyl), ethylcarbonyl, tert-butylcarbonyl and (C₁₋₃ alkyl)carbonyl.

In the specification, "5- or 6-membered heteroaryl" is not particularly limited as long as it is a heteroaryl of a 5-membered ring or a 6-membered ring having at least one hetero atom selected from an oxygen atom, a nitrogen atom and a sulfur atom. Examples thereof include pyridyl, pyrimidyl, pyridazinyl, pyrazyl, furanyl (furyl), thiophenyl (thienyl), oxazolyl, oxadiazolyl, pyrrolyl, imidazolyl, pyrazolyl, triazolyl and tetrazolyl.

In the specification, "C₁₋₆ alkoxy-C₁₋₆ alkyl" refers to C₁₋₆ alkyl having a substituent of C₁₋₆ alkoxy already defined and the alkyl moiety of C₁₋₆ alkyl is the same as already defined. Examples thereof include methoxymethyl, ethoxymethyl, 2-methoxyethyl, 1-methoxyethyl, 3-methoxypropyl, 2-methoxypropyl and 1-methoxypropyl.

Examples of a halogen atom include a fluorine atom, a chlorine atom, a bromine atom and an iodine atom.

In the specification, examples of "(C₁₋₆ alkyl)carbonyl optionally substituted with one or more halogen atoms" include trifluoroacetyl, difluoroacetyl, 2,2,2-trifluoroethylcarbonyl and perfluoroethylcarbonyl.

If the compound represented by Formula (I) forms a solvate such as a hydrate, the present invention can be carried out by use of the solvate. Furthermore, the compound of the present invention can be appropriately carried out by use of the compound in the state of a mixture or a solution or crystal polymorphism.

In the specification, "substituted with one or more substituents" includes, for example, substitution with 1 to 3 substituents.

The present invention relating to the compound represented by Formula (I) includes a tautomer, a geometric isomer, various stereoisomers such as an optical isomer, and a diastereomer and a mixture of these. Examples of the compound represented by Formula (I) contains compound represented by the following Formulas (Ia) to (Ih).

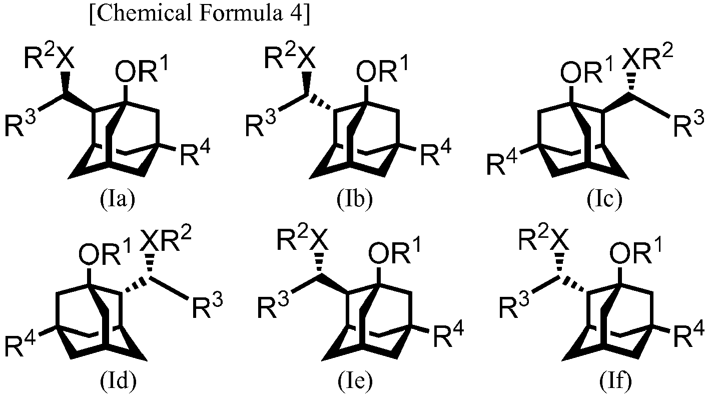

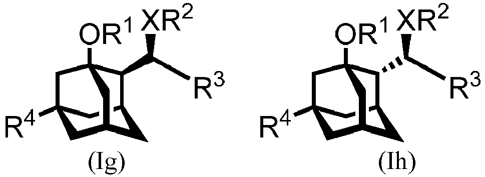

As the compound of the present invention, for example, a compound described in Examples of the specification can be used. More specifically, the following compounds can be used:
(R)-((1R,2S,3R,5R,7S)-5-azido-1-hydroxyadamantan-2-yl)(phenyl)methyl acetate (TP-009);
ethyl (S)-2-acetamido-2-((1R,2S,3R,5R,7R)-5-chloro-1-hydroxyadamantan-2-yl)acetate (TP-010);
ethyl (R)-2-acetamido-2-((1R,2S,3R,5R,7R)-5-chloro-1-hydroxyadamantan-2-yl)acetate (TP-011);
(1R,2S,3R,5R,7R)-5-chloro-2-((S)-2-methoxy-2-oxo-1-(2,2,2-trifluoroacetamido)ethyl)adamantan-1-yl 2,2,2-trifluoroacetate (TP-012);
(1S,2R,3S,5S,7S)-5-chloro-2-((R)-phenyl(2,2,2-trifluoroacetamido)methyl)adamantan-1-yl 2,2,2-trifluoroacetate (TP-014);
(S)-2-amino-2-((1R,2S,3R,5R,7S)-1,5-dihydroxyadamantan-2-yl)acetic acid (TP-015);
N-((R)-((1S,2R,3S,5S,7S)-5-chloro-1-hydroxyadamantan-2-yl)(phenyl)methyl)-2,2,2-trifluoroacetamide (TP-048);
(1S,2R,3S,5R,7S)-2-((R)-phenyl(2,2,2-trifluoroacetamido)methyl)adamantan-1-yl 2,2,2-trifluoroacetate (TP-049);
(1S,2R,3S,5S,7R)-5-(2-methoxyethoxy)-2-((R)-phenyl(2,2,2-trifluoroacetamido)methyl)adamantan-1-yl 2,2,2-trifluoroacetate (TP-050);
N-((R)-((1S,2R,3S,5S,7S)-5-chloro-1-hydroxyadamantan-2-yl)(pyridin-3-yl)methyl)-2,2,2-trifluoroacetamide (TP-051);
2,2,2-trifluoro-N-((R)-((1S,2R,3S,5R,7S)-1-hydroxyadamantan-2-yl)(phenyl)methyl) acetamide (TP-052); and
(1S,2R,3S,5S,7R)-5-methoxy-2-((R)-phenyl(2,2,2-trifluoroacetamido)methyl)adamantan-1-yl 2,2,2-trifluoroacetate (TP-053).

The "pharmaceutically acceptable salt" of the compound represented by Formula (I) is not particularly limited as long as it is a salt that can be used as a pharmaceutical product. Examples of the salt formed by the compound of the present invention and a base include a salt with an inorganic base such as sodium, potassium, magnesium, calcium and aluminum; and a salt with an organic base such as methylamine, ethylamine and ethanolamine. The salt may be an acid addition salt. Examples of the acid addition salt include a salt with a mineral acid such as hydrochloric acid, hydrobromic acid, hydriodic acid, sulfuric acid, nitric acid and phosphoric acid; and an acid addition salt with an organic acid such as formic acid, acetic acid, propionic acid, oxalic acid, malonic acid, succinic acid, fumaric acid, maleic acid, lactic acid, malic acid, tartaric acid, citric acid, methanesulfonic acid and ethanesulfonic acid.

The atoms (for example, a hydrogen atom, a carbon atom, an oxygen atom, a nitrogen atom and a sulfur atom) contained in the compound represented by Formula (I) may be isotope atoms other than those naturally most frequently exist. The isotope atoms may be radioactive isotope atoms. More specifically, according to one aspect of the present invention, there is provided a compound represented by Formula (I) already defined in the specification and labeled with an isotope atom or a salt thereof. Labelling with an isotope atom herein may be labelling with, for example, a radioactive isotope (e.g., ³H, ¹⁴C, ³²P). In order to easily prepare the compound, labeling with ³H is preferable.

In an embodiment of the present invention, the compound represented by Formula (I), an enantiomer thereof, a diastereomer thereof, or a pharmaceutically acceptable salt thereof is administered as a prodrug and converted into an active compound *in vivo.*

In the present invention, examples of the treatment for a cognitive disease or disorder include treatment for Alzheimer's dementia, cerebrovascular dementia, Lewy body dementia, frontotemporal dementia, Parkinson's disease, a mental disease and a neurodegenerative disease. In the present invention, a pharmaceutical composition may be applied to improvement of brain dysfunctions for example, brain dysfunctions caused by cerebral vascular disease, brain injury, brain tumor, viral encephalitis, hypoxic encephalopathy and alcoholic intoxication. The present invention can be applied to, particularly, cognitive dysfunctions such as memory disturbance, attentional deficit, executive function disorder and social behavior disorder. Examples of the cognitive dysfunction include a neurodegenerative disease (Alzheimer's disease, Parkinson's disease, Pick's disease and Huntington's disease, etc.), a mental disease (schizophrenia, bipolar disorder, depression, phobia, sleep disorder, drug addiction, etc.) and a pervasive developmental disorder (autism, Asperger's syndrome, mental retardation, hyperactivity disorder, tic disorder, etc.).

In the present invention, examples of the diabetic complication include hyperglycemia, diabetic coma, ketonic coma, nonketotic hyperosmolar coma, lactic acidosis, hypoglycemic coma, acute infection, microangiopathy, diabetic retinopathy, diabetic nephropathy, diabetic neuropathy, macroangiopathy, cerebral vascular disease, ischemic heart disease, diabetic gangrene, hyperlipidemia, chronic infection, cholelithiasis and cataract.

In an embodiment of the present invention, the compound represented by Formula (I), an enantiomer thereof, a diastereomer thereof, or a pharmaceutically acceptable salt thereof is used as a Kir6.2 channel inhibitor or a Kir6.1 channel inhibitor. More specifically, the compound represented by Formula (I), an enantiomer thereof, a diastereomer thereof, or a pharmaceutically acceptable salt thereof can be used for treating or preventing a disease to which the Kir6.2 channel is involved, such as a cognitive disease or disorder, hyperglycemia, diabetes and a diabetic complication; and for treating or preventing a disease to which the Kir6.1 channel is involved, such as a cognitive disease or disorder, hyperglycemia, diabetes, a diabetic complication and a mental disease.

The pharmaceutical composition of the present invention may have various dosage forms. Examples of dosage forms for oral administration include a tablet, a capsule, a powder medicine, a granule, a pill, a liquid medicine, an emulsion, a suspension, a solution, a sprit, a syrup, an extract and an elixir. Examples of dosage forms for parenteral administration include an injection such as a subcutaneous injection, an intravenous injection, an intramuscular injection, an intraperitoneal injection; transdermal administration or patch, and an ointment or a lotion. Examples of dosage forms for intraoral administration include a sublingual formulation and oral patch. Examples of nasal administration include an aerosol. However, the dosage forms are not limited to these. These preparations can be produced by methods known in the art and usually used in a drug formulation process.

The pharmaceutical composition may contain various components commonly used; for example, at least one type of pharmacologically accepted excipient, disintegrant, diluent, lubricant, flavoring agent, colorant, sweetener, corrigent, suspending agent, wetting agent, emulsifier, dispersant, adjuvant, preservative, buffer, binder, stabilizer and coating agent can be contained. The pharmaceutical composition of the present invention may be a prolonged action form or sustained release form.

The dose of the therapeutic agent, prophylactic agent or the pharmaceutical composition of the present invention can be appropriately selected depending on, e.g., the route of administration, the body size, age, physical condition of the patient, severe or mild symptom of a disease and the period of a disease after onset of the patient. The pharmaceutical composition of the present invention can contain a therapeutically effective amount and/or prophylactically effective amount of the compound represented by Formula (I). In the present invention, the compound represented by Formula (I) can be used usually in a dose of 1 to 1000 mg/day/adult or 0.01 to 20 mg/day/kg body weight. The administration of the pharmaceutical composition may be a single or multiple dose administration.

The pharmaceutical composition of the present invention may contain, if necessary, components known in the art such as a colorant, a preservative, an aroma chemical, a flavoring agent, a coating agent, an antioxidant, a vitamin, an amino acid, a peptide, a protein and a mineral (iron, zinc, magnesium, iodine, etc.). The therapeutic agent or prophylactic agent of the present invention may have dosage forms suitable for, e.g., a pharmaceutical composition, a functional food, a healthy food, a beverage and a supplement, for example, solid preparations such as a granule (including dry syrup), a capsule (soft capsule, hard capsule), a tablet (including a chewable medicine), a powder medicine (powder) and a pill, or liquid preparations such as an internal medicine solution (including a liquid medicine, a suspension agent, a syrup). The therapeutic agent or prophylactic agent of the present invention can be used directly as, e.g., a pharmaceutical composition, a functional food, a healthy food and a supplement.

Examples for additives for drug-product formulation include an excipient, a lubricant, a binder, a disintegrant, a fluidizing agent, a dispersant, a wetting agent, a preservative, a thickening agent, a pH modifier, a colorant, a flavoring agent, a surfactant and a solubilizing agent. For formulation of a liquid medicine, a thickener such as pectin, xanthan gum and guar gum can be blended. Furthermore, coated tablets can be formed by using a coating agent and pasty glue can be formed. In the cases of other dosage forms, drug products may be prepared in accordance with a conventional method.

### EXAMPLES

The present invention will be more specifically described by way of Examples; however, the present invention is not limited to these Examples.

### Example 1

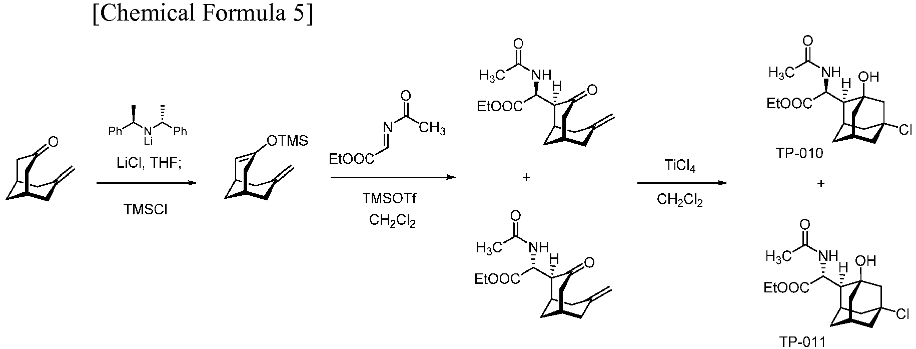

To a solution of bis((R)-1-phenylethyl)amine (1.8 g, 18 mmol) in THF (30 mL), a solution of n-BuLi in hexane (1.56 M, 4.8 mL, 7.33 mmol) was added dropwise under cooling with ice. The reaction solution was stirred for 30 minutes at the same temperature, the reaction solution was cooled down to a temperature of -78°C. Thereafter, trimethylsilyl chloride (TMSCl, 1.7 mL, 13.3 mmol) was added and subsequently a solution of 7-methylenebicyclo[3.3.1]nonan-3-one (1.0 g, 16.6 mmol) in THF (5 mL) was added by cannulation. After stirring for one hour, water was added to the reaction solution, which was extracted with diethyl ether. The resultant organic layer was washed with saturated saline and dried over MgSO₄. The solvent was distilled off under reduced pressure. The residue was subjected to silica gel chromatography (hexane:ethyl acetate = 9:1) to obtain a target substance, TMS enol ether (1.24 g, 84%) as colorless oil.

The TMS enol ether (400 mg, 1.80 mmol) obtained and ethyl (E)-2-(acetylimino)acetate (5.4 mmol) prepared in accordance with the method disclosed in the literature (Kobayashi S et al., J. Combi. Chem. 2001, 3, 401) were dissolved in dichloromethane (9 mL). The reaction solution was cooled to 0°C. To this solution, trimethylsilyl trifluoromethanesulfonate (TMSOTf, 240 µL, 900 µmol) was added. The solution was stirred at the same temperature for one hour, and then, a saturated aqueous NaHCO₃ solution was added to terminate the reaction. The reaction solution was extracted with ethyl acetate. The resultant organic layer was washed with saturated saline and dried over MgSO₄. The solvent was distilled off under reduced pressure to obtain the crude product (410 mg, oil) of the Mannich reaction. The crude product (400 mg) was dissolved in dichloromethane (7 mL) and cooled to -30°C. To the solution, TiCl₄ (120 µL, 1.09 mmol) was added. After stirring the solution for one hour at the same temperature, water was added to terminate the reaction. The solution was extracted with diethyl ether. After the resultant organic layer was washed with saturated saline and dried over MgSO₄, the solvent was distilled off under reduced pressure. The residue was subjected to silica gel column chromatography (hexane:ethyl acetate = 10:1 to 9:1) to obtain TP-010 (148 mg, 25%) and TP-011 (228 mg, 52%).

TP-010 (84% ee): amorphous; [α]_{D}²⁹ = 3.4 (c = 1.832, CHCl₃); (¹H-NMR (400 MHz, CDCl₃): δ6.15 (d, J = 8.8 Hz, 1H), 4.90 (t, J = 9.6 Hz, 1H), 4.22-4.15 (m, 2H), 3.00 (s, 1H), 2.29 (br s, 1H), 2.15-1.95 (m, 8H), 2.04 (s, 3H), 1.85-1.78 (m, 2H), 1.50 (br d, J = 12.7 Hz, 1H), 1.37 (br d, J = 13.4 Hz, 1H), 1.28 (t, J = 7.3 Hz, 3H); ¹³C-NMR (100 MHz, CDCl₃): δ173.1, 170.3, 70.8, 66.6, 61.8, 56.7, 51.8, 51.7, 47.9, 46.6, 37.7, 33.3, 31.8, 29.4, 23.4, 14.0; IR (neat, cm⁻¹): 3336, 1725, 1654; MS (EI): m/z 329 (M⁺), 256 (100%); HRMS (EI): calcd for C₁₆H₂₄NO₄Cl (M+) 329.1394, found 329.1399.

TP-011 (84% ee): mp 65-68°C (Et₂O-n-hexane); [α]_{D}²⁹ = -2.4 (c = 1.72, CHCl₃); (¹H-NMR (400 MHz, CDCl₃): δ7.64 (br s, 1H), 4.42 (d, J = 10.1, 3.9 Hz, 1H), 4.20 (q, J = 7.1 Hz, 2H), 3.70 (s, 1H), 2.30 (br s, 1H), 2.20-1.88 (m, 9H), 1.98 (s, 3H), 1.88 (br d, J = 13.9 Hz, 1H), 1.57 (br d, J = 12.5 Hz, 1H), 1.38 (br d, J = 12.5 Hz, 1H), 1.28 (t, J = 7.1 Hz, 3H); ¹³C-NMR (100 MHz, CDCl₃): δ171.7, 170.4, 72.6, 66.0, 61.1, 56.6, 55.1, 48.9, 47.3, 46.2, 38.3, 34.3, 31.8, 29.0, 22.9, 14.1; IR (neat, cm⁻¹): 3377, 1739, 1650; MS (EI): m/z 329 (M⁺), 256 (100%); HRMS (EI): calcd for C₁₆H₂₄NO₄Cl (M⁺) 329.1394, found 329.1415.

### Example 2

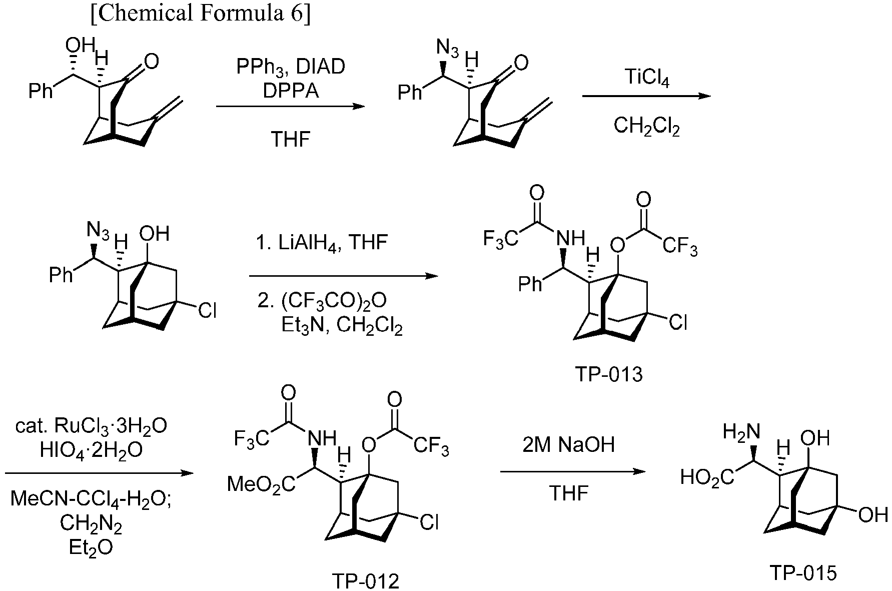

To a solution of (1R,2S,5S)-2-((R)-hydroxy(phenyl)methyl)-7-methylenebicyclo[3.3.1]nonan-3-one (750 mg, 2.9 mmol), which was prepared in accordance with the method described in J. Am. Chem. Soc. 2014, 136, 17591-17600, diphenyl phosphate azide (DPPA, 820 µL, 3.81 mmol) and triphenylphosphine (1.20 g, 4.4 mmol) in THF (15 mL), diisopropyl azodicarboxylate (DIAD, 2.2 mL, 4.4 mmol) was added under cooling with ice. After stirring for one hour at the same temperature, the solvent was distilled off under reduced pressure. To the residue, dichloromethane (15 mL) was added and TiCl₄ (820 µL, 2.3 mmol) was added under cooling with ice. After the reaction solution was stirred at room temperature for 4 hours, a saturated aqueous NaHCO₃ solution was added under cooling with ice. The reaction solution was filtered with Celite (registered trade mark). The filtrate was extracted with diethyl ether. The resultant organic layer was washed with saturated saline and dried over MgSO₄. The solvent was distilled off under reduced pressure and the residue was subjected to silica gel column chromatography (hexane:ethyl acetate = 10:1) to obtain (1R,2S,3R,5R,7R)-2-((S)-azido(phenyl)methyl)-5-chloroadamantan-1-ol (756 mg, 92%) as a white solid.

To solution of the resultant azide compound (750 mg, 2.67 mmol) in THF (14 mL), LiAlH₄ (300 mg, 8.00 mmol) was added under cooling with ice. After stirring for one hour at the same temperature, ammonia water was added to the reaction solution. The reaction solution was filtered by Celite (registered trade mark), the solvent was distilled off under reduced pressure. To the residue, dichloromethane (15 mL) was added, and thereafter, triethylamine (2.2 mL, 16.0 mmol) and anhydrous trifluoro acetic acid (TFAA, 1.2 mL, 8.0 mmol) were added under cooling with ice. After the reaction solution was stirred at room temperature overnight, a saturated aqueous NaHCO₃ solution was added. The reaction solution was extracted with dichloromethane. The resultant organic layer was dried over MgSO₄. The solvent was distilled off under reduced pressure and the residue was subjected to silica gel column chromatography (hexane:ethyl acetate = 15:1) to obtain TP-013 (871 mg, 56%) as a white solid.

mp 83-85°C (colorless needle crystal, n-hexane-Et₂O); [α]_{D}³¹ = -84.1 (c = 1.08, CHCl₃); ¹H-NMR (400 MHz, CDCl₃): δ 7.35-7.27 (m, 5H), 6.63 (d, J = 11.1 Hz, 1H), 5.44 (t, J = 10.4 Hz, 1H), 3.26 (d, J = 11.1 Hz, 1H), 2.99 (d, J = 11.1 Hz, 1H), 2.45-2.41 (m, 3H), 2.26-2.13 (m, 5H), 1.96 (br d, J = 12.4 Hz, 2H), 1.47 (br d, J = 14.0 Hz, 1H); ¹³C-NMR (100 MHz, CDCl₃): δ 156.2 (q, J = 37.4 Hz), 154.9 (q, J = 42.3 Hz), 139.1, 129.2, 128.7, 127.1, 115.8 (q, J = 288.1 Hz), 113.3 (q, J = 287.3 Hz), 86.6, 65.1, 53.4, 50.2, 48.0, 46.9, 46.1, 35.6, 34.6, 31.7, 28.5; IR (neat, cm⁻¹): 3296, 2945, 1775, 1698; MS (EI): m/z 483 (M⁺), 202 (100%); HRMS (EI): calcd for C₂₁H₂₀ClF₆NO₃ (M⁺) 483.1036, found 483.1046.

To a solution containing TP-013 (550 mg, 1.14 mmol) in acetonitrile (1.8 mL)-carbon tetrachloride (1.8 mL)-water (1.8 mL), RuCl₃·3H₂O (114 µmol) and HIO₄·2H₂O (3.6 g, 16.0 mmol) were added under cooling with ice. The reaction solution was vigorously stirred at the same temperature for 8 hours. To the reaction solution, water was added. The reaction solution was extracted with dichloromethane. To the resultant organic layer, a solution of diazomethane in diethyl ether was added was under cooling with ice until the solution turned yellow. Thirty minutes later, nitrogen was blown into the solution to remove diazomethane and then the solvent was distilled off under reduced pressure. The residue was subjected to silica gel column chromatography (hexane:ethyl acetate = 15:1) to obtain TP-012 (235 mg, 44%) as a white solid.

[α]_{D}²⁴ = -22.7 (c = 1.84, CHCl₃); ¹H-NMR (400 MHz, CDCl₃): δ7.10 (d, J = 9.8 Hz, 1H), 5.02 (d, J = 10.1 Hz, 1H), 3.76 (s, 3H), 2.92 (dd, J = 10.3, 2.0 Hz, 1H), 2.74 (d, J = 11.5 Hz, 1H), 2.69 (dd, J = 11.5, 1.7 Hz, 1H), 2.43 (br s, 1H), 2.30-2.10 (m, 7H), 1.86 (d, J = 14.1 Hz, 1H), 1.46 (d, J = 14.1 Hz, 1H); ¹³C-NMR (100 MHz, CDCl₃):δ171.1, 157.1 (q, J = 37.9 Hz), 155.2 (q, J = 41.2 Hz), 115.5 (q, J = 285.4 Hz), 113.8 (q, J = 284.6 Hz), 87.0, 64.8, 53.0, 51.3, 49.8, 47.6, 46.5, 45.9, 34.2, 33.5, 31.7, 28.8; IR (neat, cm⁻¹): 3319, 1780, 1714; MS (EI): m/z 406 (M-CO₂CH₃); HRMS (EI): calcd for C₁₅H₁₅NO₃F₆Cl (M+) 406.0645, found 406.0651.

To a solution of TP-012 (256 mg, 550 µmol) in THF (2.0 mL), an aqueous NaOH solution (2 M, 2.0 mL) was added under cooling with ice. The reaction solution was stirred for two hours. After THF was distilled off, the reaction solution was neutralized with a 10% aqueous HCl solution, subjected to ion exchange chromatography (DOWEX50), eluted with a 0.23 N aqueous ammonium chloride solution and subjected to lyophilization to obtain TP-015 (48.5 mg, 34%) as a white solid.

[α]_{D}²⁸ = -46.3 (c = 0.78, MeOH); (¹H-NMR (600 MHz, CD₃OD): δ3.87 (d, J = 9.6 Hz, 1H), 2.26 (br s, 1H), 2.19 (br s, 1H), 2.12 (d, J = 10.9 Hz, 1H), 2.08 (d, J = 12.4 Hz, 1H), 1.80-1.62 (m, 5H), 1.66 (br s, 2H), 1.50 (br d, J = 12.4 Hz, 1H), 1.37 (br d, J = 13.1 Hz, 1H); ¹³C-NMR (150 MHz, CD₃OD): δ 174.6, 70.3, 68.6, 54.7, 53.8, 49.4, 44.3, 43.4, 38.7, 32.9, 30.9, 29.2; IR (neat, cm⁻¹): 3336, 1730; MS (FAB): m/z 242 (M+1); HRMS (FAB): calcd for C₁₂H₂₀NO₄ (M+1) 242.1387, found 252.1383.

### Example 3

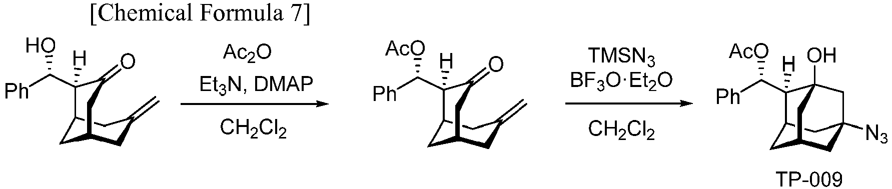

To a solution of (1R,2S,5S)-2-((R)-hydroxy(phenyl)methyl)-7-methylenebicyclo[3.3.1]nonan-3-one (256 mg, 1.00 mmol) in dichloromethane (5 mL), trimethylamine (0.42 mL, 3 mmol), dimethylaminopyridine (DMAP, 12 mg, 0.1 mmol) and anhydrous acetic acid (0.14 mL, 1.5 mmol) were added under cooling with ice. After the reaction solution was stirred at room temperature for 20 minutes, a saturated aqueous NaHCO₃ solution was added thereto under cooling with ice and extracted with dichloromethane. The resultant organic layer was dried over MgSO₄ and the solvent was distilled off under reduced pressure. The residue was subjected to silica gel column chromatography (hexane:ethyl acetate = 4:1) to obtain (1R,2S,5S)-2-((R)-acetoxy(phenyl)methyl)-7-methylenebicyclo[3.3.1]nonan-3-one (289 mg, 97%) as a white solid.

To a solution of the resultant product (75.2 mg, 0.252 mmol) in dichloromethane (2.5 mL), trimethylsilyl azide (TMSN₃, 0.10 mL, 0.76 mmol) and BF₃·OEt₃ (0.04 mL, 0.30 mmol) were added at -20°C. The temperature of the reaction solution was gradually increased up to room temperature and the reaction solution was stirred for 3 hours. Subsequently, to the reaction solution, a saturated aqueous NaHCO₃ solution was added under cooling with ice. The resultant reaction solution was extracted with dichloromethane. The resultant organic layer was dried over MgSO₄ and the solvent was distilled off under reduced pressure. The residue was subjected to silica gel column chromatography (hexane:ethyl acetate = 2:1) to obtain TP-009(33.5 mg, 39%) as a white solid.

mp 114°C (colorless crystal, n-hexane-Et₂O); [α]_{D}¹⁹ = +56.0 (c = 0.67, CHCl₃); ¹H-NMR (400 MHz, CDCl₃): δ7.37-7.31 (m, 5H), 6.01 (d, J = 10.6 Hz, 1H), 2.95 (br s, 1H), 2.27 (br s, 1H), 2.25 (d, J = 10.6 Hz, 1H), 2.15 (d, J = 13.0 Hz, 1H), 2.01 (s, 3H), 1.89-1.83 (m, 2H), 1.76-1.61 (m, 7H), 1.18 (d, J = 13.0 Hz, 1H); ¹³C-NMR (100 MHz, CDCl₃): δ 169.6, 138.9, 128.7, 128.5, 127.3, 77.0, 71.3, 60.0, 51.9, 50.7, 41.6, 40.4, 38.8, 32.5, 30.6, 29.3, 21.6; IR (neat, cm⁻¹): 3460, 2931, 2091, 1732; MS (EI): m/z 323 (M⁺-H₂O), 107 (100%); HRMS (EI): calcd for C₁₉H₂₁N₃O₂ (M⁺-H₂O) 323.1634, found: 323.1613.

### Example 4

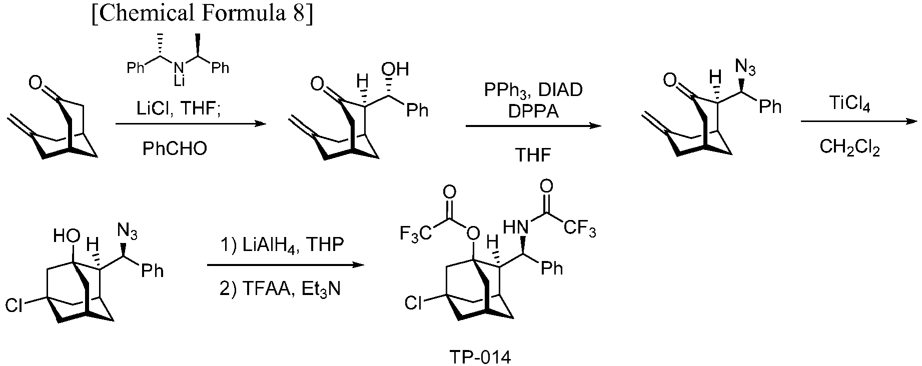

To a solution of bis((S)-1-phenylethyl)amine (10.0 mL, 44 mmol) and lithium chloride (3.4 g, 80 mmol) in THF (100 mL), a solution of n-butyl lithium in hexane (1.56 M, 28.2 mL, 44 mmol) was added dropwise under cooling with ice. The reaction solution was stirred at the same temperature for 30 minutes and then cooled up to -78°C. To the reaction mixture, a solution of 7-methylenebicyclo[3.3.1]nonan-3-one (6.00 g, 40 mmol) in THF (60 mL) was added by cannulation. After the reaction solution was stirred for one hour, a solution of benzaldehyde (6.1 mL, 60 mmol) in THF (40 mL) was added by cannulation. After the reaction solution was stirred for 2 hours, acetic acid and a saturated aqueous ammonium chloride solution were sequentially added to the reaction solution. The reaction solution was extracted with diethyl ether. The resultant organic layer was washed with saturated saline and dried over MgSO₄. The solvent was distilled off under reduced pressure and the residue was subjected to silica gel column chromatography (hexane:ethyl acetate = 4:1) to obtain (1S,2R,5R)-2-((S)-hydroxy(phenyl)methyl)-7-methylenebicyclo[3.3.1]nonan-3-one (8.3 g, 81%) as a white solid. This was recrystallized from diethyl ether to obtain colorless needle crystal.

mp 122°C; [α]_{D}²¹ = -17.9 (c = 0.32, CHCl₃); ¹H-NMR (400 MHz, CDCl₃): δ7.38-7.25 (m, 5H), 4.79 (d, J = 1.8 Hz, 1H), 4.76 (d, J = 1.8 Hz, 1H), 4.71 (d, J = 6.8 Hz, 1H), 2.90 (s, 1H), 2.64 (dd, J = 15.7, 6.8 Hz, 1H), 2.48-2.18 (m, 6H), 2.01 (br d, J = 14.3 Hz, 1H); ¹³C-NMR (100 MHz, CDCl₃): δ 211.0, 141.6, 128.8, 127.6, 114.8, 74.6, 62.7, 45.7, 42.2, 41.3, 32.4, 31.9, 28.4; IR (neat, cm⁻¹): 3390, 1711; MS (EI): m/z 256 (M⁺), 95 (100%); HRMS (EI): calcd for C₁₇H₂₀O₂ (M+) 256.1463, found 256.1450.

To a solution of (1S,2R,5R)-2-((S)-hydroxy(phenyl)methyl)-7-methylenebicyclo[3.3.1]nonan-3-one (2.00 g, 7.5 mmol), DPPA (2.3 mL, 11 mmol) and triphenylphosphine (3.0 g, 11 mmol) in THF (38 mL), DIAD (2.2 mL, 11 mmol) was added under cooling with ice. After stirring for one hour at the same temperature, the solvent was distilled off under reduced pressure. To the residue, dichloromethane (38 mL) was added and TiCl₄ (0.8 mL, 7.5 mmol) was added under cooling with ice. After the reaction solution was stirred at room temperature for 4 hours, a saturated aqueous NaHCO₃ solution was added under cooling with ice. The reaction solution was filtered by Celite (registered trade mark) and the filtrate was extracted with diethyl ether. The resultant organic layer was washed with saturated saline and dried over MgSO₄. The solvent was distilled off under reduced pressure and tetrahydropyran (THP, 40 mL) was added to the residue. To this, LiAlH₄ (430 mg, 11 mmol) was added under cooling with ice. The reaction mixture was stirred at the same temperature for 30 minutes and ammonia water was added to the reaction solution. The reaction solution was filtered by Celite (registered trade mark) and the solvent was distilled off under reduced pressure. To the residue, dichloromethane (40 mL) was added, and then, triethylamine (6.3 mL, 45 mmol) and TFAA (3.2 mL, 23 mmol) were added under cooling with ice. The resultant reaction solution was stirred at room temperature overnight and a saturated aqueous NaHCO₃ solution was added thereto and then extracted with dichloromethane. The resultant organic layer was dried over MgSO₄. The solvent was distilled off under reduced pressure and the residue was subjected to silica gel column chromatography (hexane:ethyl acetate = 15:1) to obtain a crude product as a white solid. This was recrystallized from diethyl ether-hexane to obtain TP-014 (1.27 g, 35%) as a white solid.

mp 89°C; [α]_{D}²¹ = +89.1 (c = 0.31, CHCl₃); ¹H-NMR (400 MHz, CDCl₃): δ 7.35-7.27 (m, 5H), 6.63 (d, J = 11.1 Hz, 1H), 5.44 (t, J = 10.4 Hz, 1H), 3.26 (d, J = 11.1 Hz, 1H), 2.99 (d, J = 11.1 Hz, 1H), 2.45-2.41 (m, 3H), 2.26-2.13 (m, 5H), 1.96 (br d, J = 12.4 Hz, 2H), 1.47 (br d, J = 14.0 Hz, 1H); ¹³C-NMR (100 MHz, CDCl₃): δ 156.2 (q, J = 37.4 Hz), 154.9 (q, J = 42.3 Hz), 139.1, 129.2, 128.7, 127.1, 115.8 (q, J = 288.1 Hz), 113.3 (q, J = 287.3 Hz), 86.6, 65.1, 53.4, 50.2, 48.0, 46.9, 46.1, 35.6, 34.6, 31.7, 28.5; IR (neat, cm⁻¹): 3296, 2945, 1775, 1698; MS (EI): m/z 483 (M⁺), 202 (100%); HRMS (EI): calcd for C₂₁H₂₀ClF₆NO₃ (M⁺) 483.1036, found 483.1046; Anal.: calcd for C₂₁H₂₀ClF₆NO₃: C, 52.13; H, 4.17; N, 2.89. found C, 52.27; H, 4.18; N, 2.88.

### Example 5

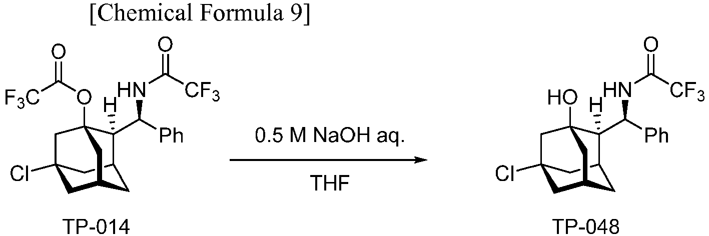

To a solution of TP-014 (84.7 mg, 0.175 mmol) in THF (2 mL), a 0.5 M aqueous NaOH solution (1 mL) was added under cooling with ice. The reaction solution was stirred at the same temperature for 15 minutes and a saturated NH₄Cl aqueous solution was added thereto and extracted with ethyl acetate. The resultant organic layer was washed with saturated saline and dried over MgSO₄. The solvent was distilled off under reduced pressure and the residue was subjected to silica gel column chromatography (hexane:ethyl acetate = 8:1 to 4:1) to obtain TP-048 (65.5 mg, 96%) as a white solid.

[α]_{D}²⁶= +109.2 (c = 0.772, CHCl₃); ¹H-NMR (400 MHz, CDCl₃): δ 7.41-7.32 (m, 5H), 6.98 (br, 1H), 5.34 (t, J = 9.7 Hz, 1H), 2.36-2.29 (m, 3H), 2.19-2.00 (m, 7H), 1.77 (br d, J = 11.6 Hz, 1H), 1.41-1.33 (m, 2H); ¹³C-NMR (100 MHz, CDCl₃): δ 156.2 (q, J = 37.1), 140.5, 129.4, 128.6, 127.4, 115.8 (q, J = 288.1 Hz), 72.3, 66.1, 56.7, 54.2, 52.4, 47.7, 46.3, 38.6, 34.4, 31.8, 28.8; IR (neat, cm⁻¹): 3553, 3297, 2940, 1698, 1552, 1208, 1183, 1165; MS (EI): m/z 387 (M⁺), 202 (100%); HRMS (EI):calcd for C₁₉H₂₁ClF₃NO₂ (M⁺) 387.1213, found 387.1196.

### Example 6

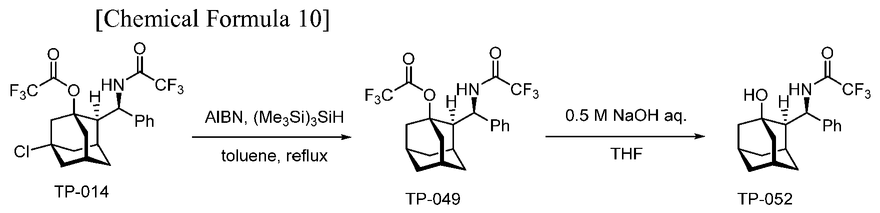

To a solution of TP-014 (30.0 mg, 0.062 mmol) in toluene (2 mL), tris(trimethylsilyl)silane (29 µL, 0.095 mmol) and azobisisobutyronitrile (AIBN, 2.0 mg, 0.012 mmol) were added at room temperature. After the reaction solution was heated under reflux overnight, the solvent was distilled off under reduced pressure. The residue was subjected to silica gel column chromatography (hexane:ethyl acetate = 15:1) to obtain TP-049 (23.0 mg, 83%) as a white solid.

[α]_{D}²⁹ = +106.4 (c = 0.385, CHCl₃); ¹H-NMR (400 MHz, CDCl₃): δ 7.33-7.27 (m, 5H), 6.31 (br d, J = 10.1 Hz, 1H), 5.50 (dd, J = 10.9, 10.1 Hz, 1H), 3.20 (br d, J = 10.9 Hz, 1H), 2.60 (br d, J = 11.6 Hz, 1H), 2.45 (br d, J = 12.1 Hz, 1H), 2.28-2.27 (m, 3H), 2.04-1.80 (m, 6H), 1.72 (br s, 2H); ¹³C-NMR (100 MHz, CDCl₃): δ 156.0 (q, J = 37.1 Hz), 155.1 (q, J = 41.8 Hz), 139.8, 129.0, 128.4, 127.2, 115.8 (q, J = 288.1 Hz), 113.5 (q, J = 287.3 Hz), 87.5, 53.6, 49.4, 41.3, 37.2, 36.1, 33.0, 30.6, 30.4, 30.2; IR (neat, cm⁻¹): 3335, 2927, 1775, 1700, 1556, 1218, 1169; MS (EI): m/z 449 (M⁺), 202 (100%); HRMS (EI): calcd for C₂₁H₂₁F₃NO₃ (M⁻) 449.1426, found 449.1447.

To a solution of TP-049 (61.5 mg, 0.137 mmol) in THF (1.4 mL), an aqueous NaOH solution (0.5 M, 0.5 mL) was added under cooling with ice. After the reaction solution was stirred at the same temperature for 5 minutes, 2 M hydrochloric acid was added to the reaction solution. The reaction solution was extracted with ethyl acetate. The resultant organic layer was washed with saturated saline and dried over MgSO₄. The solvent was distilled off under reduced pressure and the residue was subjected to silica gel column chromatography (hexane:ethyl acetate = 4:1 to 2:1) to obtain TP-052 (49.4 mg, quant.) as a white solid.

TP-052: [α]_{D}¹⁴ = +130.7 (c = 0.243, CHCl₃); ¹H-NMR (400 MHz, CDCl₃): δ 7.39-7.31 (m, 5H), 6.77 (br d, J = 8.9 Hz, 1H), 5.40 (dd, J = 9.7, 8.9 Hz, 1H), 2.32 (br d, J = 9.7 Hz, 1H), 2.31-2.07 (m, 4H), 1.85-1.79 (m, 2H), 1.72-1.57 (m, 5H), 1.52-1.44 (m, 2H), 1.29 (br, 1H); ¹³C-NMR (100 MHz, CDCl₃): δ 156.1 (q, 37.1 Hz), 140.7, 129.4, 128.5, 127.5, 115.9 (q, 288 Hz), 77.2, 54.3, 53.0, 50.5, 48.5, 41.4, 39.6, 39.4, 33.2, 30.6, 29.6; IR (neat, cm⁻¹): 3566, 3291, 2919, 1698, 1183; MS (EI): m/z 353 (M⁺), 151 (100%); HRMS (EI): calcd for C₁₉H₂₂F₃NO₂ (M⁺) 353.1603, found 353.1604.

### Example 7

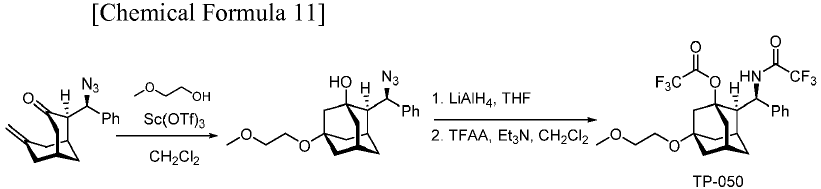

To a solution of (1S,2R,5R)-2-(R-azido(phenyl)methyl)-7-methylenebicyclo[3.3.1.]nonan-3-one (57.4 mg, 0.204 mmol) in dichloromethane (2 mL), 2-methoxyethanol (78 µL, 1.0 mmol) and scandium trifluoromethanesulfonate (5.0 mg, 0.01 mmol) were sequentially added under cooling with ice. After the reaction solution was stirred at room temperature for two days, a saturated aqueous NaHCO₃ solution was added under cooling with ice. The reaction solution was extracted with dichloromethane. The resultant organic layer was dried over MgSO₄ and the solvent was distilled off under reduced pressure. The residue was subjected to silica gel column chromatography (hexane:ethyl acetate = 1:2 to 1:1) to obtain (1S,2R,3S,5S,7S)-2-((R)-azido(phenyl)methyl)-5-(2-methoxyethoxy)adamantan-1-ol (41.2 mg, 56%) as colorless oil.

To a solution of the resultant azide compound (39.6 mg, 0.111 mmol) in THF (1 mL), LiAlH₄ (8.0 mg, 0.21 mmol) was added under cooling with ice. The temperature of the reaction solution was gradually increased up to room temperature and the reaction solution was stirred for one hour. The reaction solution was ice-cooled and then LiAlH₄ (8.0 mg, 0.21 mmol) was added. After the reaction solution was stirred at room temperature for one hour, ammonia water was added to the reaction solution under cooling with ice. The reaction solution was filtered by Celite (registered trade mark). The filtrate was dried over Na₂SO₄ and the solvent was distilled off under reduced pressure. To the residue, dichloromethane (1 mL) was added, triethylamine (77 µL, 0.56 mmol) and anhydrous trifluoro acetic acid (TFAA, 47 µL, 0.33 mmol) were added under cooling with ice. After the reaction solution was stirred at room temperature for 5 hours, a saturated aqueous NaHCO₃ solution was added under cooling with ice. The reaction solution was extracted with dichloromethane. The resultant organic layer was dried over MgSO₄. The solvent was distilled off under reduced pressure and the residue was subjected to silica gel column chromatography (hexane:ethyl acetate = 1:4 to 1:2) to obtain TP-050 (31.6 mg, 54%) as colorless oil.

[α]_{D}²¹ = +72.1 (c = 0.965, CHCl₃); ¹H-NMR (400 MHz, CDCl₃):δ 7.34-7.23 (m, 5H), 6.33 (br d, J = 9.9 Hz, 1H), 5.44 (dd, J = 10.9, 9.9 Hz, 1H), 3.59-3.56 (m, 2H), 3.51-3.48 (m, 2H), 3.37 (s, 3H), 3.17 (br d, J = 10.9 Hz, 1H), 2.65 (br d, J = 10.6 Hz, 1H), 2.43-2.37 (m, 3H), 1.95-1.81 (m, 7H), 1.38 (br d, J = 11.6 Hz, 1H); ¹³C-NMR (100 MHz, CDCl₃):δ 156.1 (q, J = 37.4 Hz), 154.9 (q, J = 42.1 Hz), 139.4, 129.1, 128.5, 127.2, 115.8 (q, J = 288.1 Hz), 113.4 (q, J = 287.3 Hz), 87.6, 73.7, 72.3, 60.2, 59.1, 53.5, 48.5, 45.0, 41.1, 39.9, 36.3, 30.5, 29.2; IR (neat, cm⁻¹): 3303, 2936, 1775, 1698, 1554, 1221, 1172; MS (EI): m/z 523 (M⁺), 202 (100%); HRMS (EI): calcd for C₂₄H₂₇F₆NO₅ (M⁺) 523.1793, found 523.1797.

### Example 8

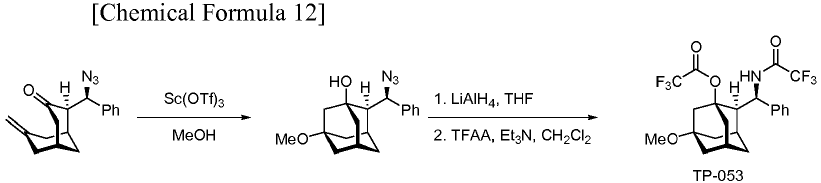

To a solution of (1S,2R,5R)-2-(R-azido(phenyl)methyl)-7-methylenebicyclo[3.3.1.]nonan-3-one (238 mg, 0.848 mmol) in methanol (8.5 mL), scandium trifluoromethanesulfonate (20 mg, 0.04 mmol) was added under cooling with ice. After the reaction solution was stirred at room temperature for 18 hours, a saturated aqueous NaHCO₃ solution was added to the reaction solution under cooling with ice. The reaction solution was extracted with ethyl acetate. The resultant organic layer was washed with saturated saline and dried over MgSO₄ and the solvent was distilled off under reduced pressure. The residue was subjected to silica gel column chromatography (hexane:ethyl acetate = 1:4 to 1:2) to obtain (1S,2R,3S,5S,7S)-2-((R)-azido(phenyl)methyl)-5-methoxyadamantan-1-ol (225 mg, 85%) as colorless oil.

To a solution of the resultant azide compound (225 mg, 0.716 mmol) in THF (4 mL), LiAlH₄ (41 mg, 1.1 mmol) was added under cooling with ice. After stirring for one hour at the same temperature, ammonia water was added to the reaction solution. The reaction solution was filtered by Celite (registered trade mark) and the solvent was distilled off under reduced pressure. To the residue, dichloromethane (4 mL) was added, and triethylamine (497 µL, 3.86 mmol) and anhydrous trifluoro acetic acid (TFAA, 299 µL, 2.15 mmol) were added under cooling with ice. After the reaction solution was stirred at room temperature for 40 hours, a saturated aqueous NaHCO₃ solution was added under cooling with ice. The reaction solution was extracted with dichloromethane. The resultant organic layer was dried over MgSO₄ and the solvent was distilled off under reduced pressure. The residue was subjected to silica gel column chromatography (hexane:ethyl acetate = 1:8 to 1:2) to obtain TP-053 (262 mg, 75%) as a white solid.

[α]_{D}¹⁴ = +97.2 (c = 0.179, CHCl₃); ¹H-NMR (400 MHz, CDCl₃):δ 7.33 (m, 5H), 6.35 (br d, J = 9.9 Hz, 1H), 5.45 (dd, J = 10.6, 9.9 Hz, 1H), 3.25 (s, 3H), 3.17 (br d, J = 10.6 Hz, 1H), 2.61 (br d J = 10.6 Hz, 1H), 2.45-2.37 (m, 3H), 1.97-1.73 (m, 7H), 1.39 (br d, J = 13.5 Hz, 1H); ¹³C-NMR (100 MHz, CDCl₃): δ 156.0 (q, J = 37.4 Hz), 155.0 (q, J = 41.8 Hz), 139.4, 129.1, 128.6, 127.1, 115.8 (q, 288.1 Hz), 113.4 (q, 287.0 Hz), 87.7, 75.5, 53.5, 48.7, 48.6, 44.5, 40.8, 39.5, 36.3, 33.3, 30.4, 29.3; IR (neat, cm⁻¹): 3299, 2941, 1776, 1697, 1221, 1172; MS (EI): m/z 479 (M⁺), 202 (100%); HRMS (EI): calcd for C₂₂H₂₃F₆NO₄ (M⁺) 479.1531, found 479.1486.

### Example 9

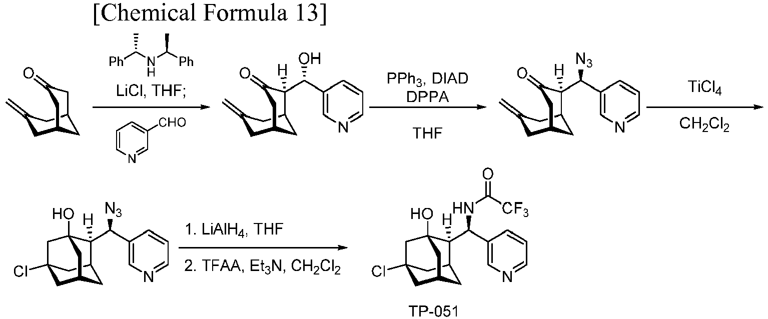

To a solution of bis((S)-1-phenylethyl)amine (2.5 mL, 11 mmol) and lithium chloride (850 mg, 20 mmol) in THF (25 mL), a solution of n-butyl lithium in hexane (1.56 M, 7.1 mL, 11 mmol) was added dropwise under cooling with ice. After the reaction solution was stirred at the same temperature for 30 minutes, the reaction solution was cooled down to -78°C. To the reaction mixture, a solution of 7-methylenebicyclo[3.3.1]nonan-3-one (1.52 g, 10 mmol) in THF (15 mL) was added by cannulation. After the reaction solution was stirred for 30 minutes, a solution of nicotinaldehyde (1.1 mL, 12 mmol) in THF (10 mL) was added by cannulation. After the reaction solution was stirred for 40 minutes, acetic acid and a saturated aqueous ammonium chloride solution were sequentially added to the reaction solution. The reaction solution was extracted with ethyl acetate. The resultant organic layer was washed with saturated saline and dried over K₂CO₃. The solvent was distilled off under reduced pressure. The residue was subjected to silica gel column chromatography (hexane-acetone = 3:2 to 1:2) to obtain (1S,2R,5R)-2-((S)-hydroxy(pyridin-3-yl)methyl)-7-methylenebicyclo[3.3.1]nonan-3-one (2.7 g, 81%) as a white solid. This was recrystallized from ethyl acetate to obtain colorless crystal (99%ee).

A solution of the resultant alcohol (258 mg, 1.0 mmol), diphenyl phosphate azide (DPPA, 237 µL, 1.1 mmol) and triphenylphosphine (239 mg, 1.1 mmol) in THF (5 mL), diisopropyl azodicarboxylate (DIAD, 214 µL, 1.1 mmol) was added under cooling with ice. The temperature of the reaction solution was gradually increased up to room temperature. After the reaction solution was stirred for 5 hours, the solvent was distilled off under reduced pressure. The residue was subjected to silica gel column chromatography (hexane:ethyl acetate = 4:1 to 2:1) to obtain (1S,2R,5R)-2-((R)-azido(pyridin-3-yl)methyl)-7-methylenebicyclo[3.3.1]nonan-3-one (187 mg, 66%) as colorless oil.

To a solution of the resultant azide compound (187 mg, 0.66 mmol) in dichloromethane (7 mL), TiCl₄ (300 µL, 0.27 mmol) was added under cooling with ice. After the reaction solution was stirred at room temperature for 3 hours, a saturated aqueous NaHCO₃ solution was added under cooling with ice. The reaction solution was extracted with diethyl ether. The resultant organic layer was washed with saturated saline, and dried over MgSO₄. The solvent was distilled off under reduced pressure and the resultant solid was washed with cold diethyl ether to obtain (1S,2R,3S,5S,7S)-2-((R)-azido(pyridin-3-yl)methyl)-5-chloroadamantan-1-ol (98.5 mg, 92%).

To a solution of the resultant compound (75.4 mg, 0.257 mmol) in THF (2 mL), LiAlH₄ (23 mg, 0.61 mmol) was added under cooling with ice. After the reaction solution was stirred for one hour at the same temperature, ammonia water was added to the reaction solution under cooling with ice. The reaction solution was filtered by Celite (registered trade mark) and the solvent was distilled off under reduced pressure. The residue was subjected to silica gel column chromatography (CHCl₃:methanol = 1:0 to 4:1) to obtain crude amine.

To the resultant crude amine, dichloromethane (2 mL) was added and then triethylamine (178 µL, 1.28 mmol) and anhydrous trifluoro acetic acid (TFAA, 107 µL, 0.76 mmol) were added under cooling with ice. The temperature of the reaction solution was increased up to room temperature. After the reaction solution was stirred for 4 hours, a saturated aqueous NaHCO₃ solution was added thereto under cooling with ice. The reaction solution was extracted with dichloromethane. The resultant organic layer was dried over Na₂SO₄. The solvent was distilled off under reduced pressure. The residue was subjected to silica gel column chromatography (hexane:ethyl acetate = 2:1 to 1:4) to obtain TP-051(48.8 mg, 49%) as a white solid.

[α]_{D}²⁰ = +53.9 (c = 0.379, CHCl₃);¹H-NMR (400 MHz, CDCl₃): δ 8.57 (d, J = 1.0 Hz, 1H), 8.50 (dd, J = 4.9, 1.5 Hz, 1H), 7.72 (br d, J = 7.8 Hz, 1H), 7.41 (br d, J =9.8 Hz, 1H), 7.32 (dd, J = 7.8, 4.9 Hz, 1H), 5.35 (dd, J = 9.8, 9.3 Hz, 1H), 2.40-2.38 (m, 2H), 2.29 (br s, 1H), 2.22-1.99 (m, 7H), 1.75 (br, 1H), 1.68 (br d, J = 13.7 Hz, 1H), 1.48 (br d, J = 13.2 Hz, 1H), 1.42 (br d, J = 13.2 Hz, 1H); ¹³C-NMR (100MHz, CDCl₃): δ 156.4 (q, J = 37.1 Hz), 148.2, 147.7, 138.3, 136.5, 124.0, 115.8 (q, J = 287.8 Hz), 71.9, 66.1, 57.3, 52.6, 51.7, 47.6, 46.3, 38.3, 34.3, 31.6, 28.6; IR (neat, cm⁻¹): 3292, 2938, 1700, 1558, 1212, 1184, 1161, 759; MS (EI): m/z 388 (M⁺), 203 (100%); HRMS (EI): calcd for C₁₈H₂₀ClF₃N₂O₂ (M⁺) 388.1165, found 388.1177.

### Test Example 1

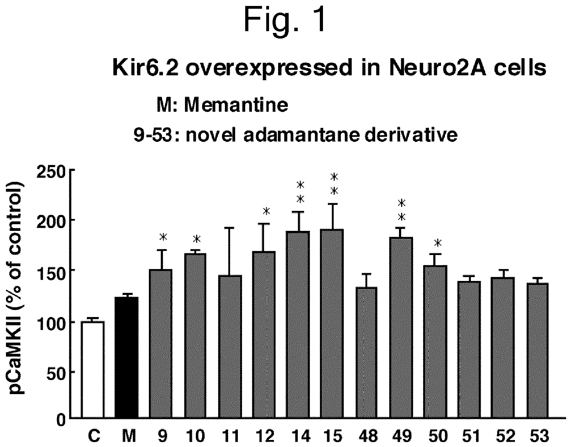

A plasmid vector having cDNA of Kir6.2 channel inserted therein: pcDNA3.1-Kir6.2, was obtained from Dr. Toru Ishizuka (Graduate School of Life Sciences, Tohoku University). The plasmid vector, pcDNA3.1-Kir6.2, was conditioned in accordance with the manual attached to GenElute HP Plasmid Maxiprep Kit (manufactured by Sigma-Aldrich). A culture solution (composition: DMEM culture solution 450 mL containing 50 ml of bovine serum and 100 units of penicillin/streptomycin) of Neuro2A cells (N2A cells, the National Instituted of Biomedical Innovation) cultured in DMEM culture solution (Gibco) was exchanged with Opti-Mem (Gibco)(containing Lipofectamine R2000 (1 µg/1ml)) containing pcDNA3.1-Kir6.2 (1 µg/µl) conditioned above, and cultured for 5 hours to obtain N2A cells overexpressing Kir6.2 channel. Exchange with the DMEM culture solution was again performed and culture was carried out for two days. Thereafter, memantine (manufactured by Sigma-Aldrich) and the compound of the present invention (n = 4 per group) were added in the culture solutions (DMEM, Gibco) so as to obtain a concentration of 10 nM based on the culture solution, and the culture solutions were allowed to stand still for one hour. Thereafter, Kir6.2 channel overexpressing N2A cells were collected and an SDS sample buffer was added to the N2A cells to prepare a suspension. The suspension was subjected to immunoblotting by use of an anti-phosphorylated CaMKII antibody (Fukunaga K et al., J. Biol. Chem. 1992, 267, 22527-22533) as a primary antibody and an anti-rabbit IgG antibody (manufactured by SouthernBiotech) as a secondary antibody (other conditions except the aforementioned antibodies were the same as those employed in ordinary immunoblotting), to investigate activation of CaMKII. As a result, in TP-009, TP-010, TP-011, TP-012, TP-014, TP-015, TP-048, TP-049, TP-050, TP-051, TP-052, TP-053, bands showing a reaction with the antibody against phosphorylated CaMKII were obtained. It was confirmed that activation of CaMKII is enhanced. The results are shown in Fig. 1. In Fig. 1, the result of the case (control: c) where a test compound was not added is regarded as 100%. CaMKII activation of the cases containing memantine, TP-009, TP-010, TP-011, TP-012, TP-014, TP-015, TP-048, TP-049, TP-050, TP-051, TP-052, TP-053 (corresponding to M, 9, 10, 11, 12,12, 15, 48, 49, 50, 51, 52 and 53, respectively) were shown in Fig. 1.

### Test Example 2

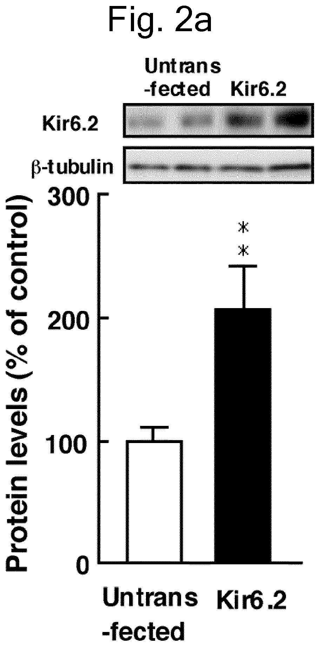

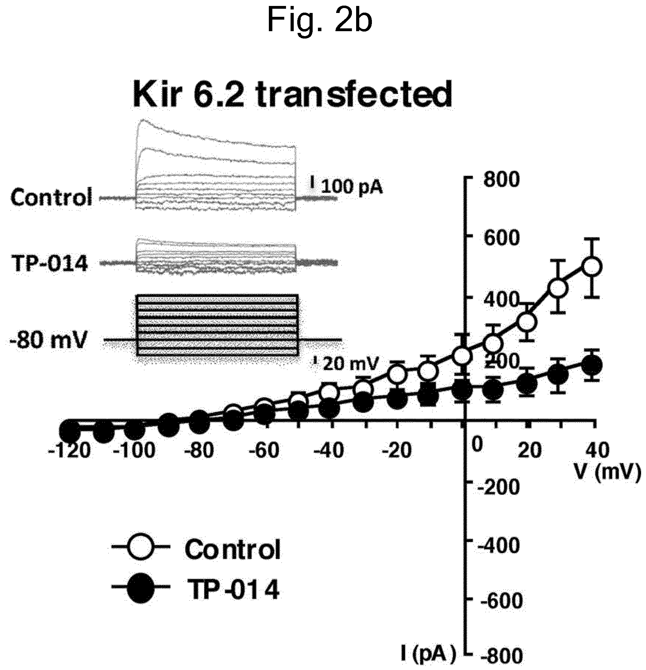

Using Kir6.2 channel-overexpressing cells obtained in Test Example 1, potassium current flowing outside from the cells was measured by an ordinary patch-clamp method. The results are shown in Fig. 2. ATP-sensitive potassium channel (Kir6.2 channel) was localized in the cell membrane of the nerve cells. If the channel is inhibited and closed, the threshold of the nerve cell membrane rises to produce a state analogous to the state where an action potential is temporarily generated, with the result that potassium current flows out from cells outside and, in place, calcium current flows into the cells from outside. Fig. 2a shows that Kir6.2 channel is overexpressed in N2A cells (the upper figure shows stained images by immunoblotting; whereas the lower figure quantitatively expresses the signal intensity of bands). This was confirmed by applying immunoblotting (the same conditions as in Test Example 1 were employed except that anti-Kir6.2 channel antibody, n = 5) with an anti-Kir6.2 channel antibody (prepared based on a customary method) to Kir6.2 channel-overexpressing cells (prepared by the aforementioned method). No change was observed in a housekeeping gene product, i.e., β tubulin (anti-β tubulin antibody was obtained from Sigma-Aldrich, and other conditions are the same as those in the detection of Kir6.2). Fig. 2b (confirmed results) shows that if Kir6.2 channel-overexpressing cells are allowed to stand still in an electrophysiological experimental buffer containing TP-014 so as to obtain a concentration of 10 nM, potassium current, which outwardly flows when the membrane potential of nerve cells is changed toward a plus side, is suppressed (n = 5 per group). The results show that TP-014 inhibits Kir6.2 channel and inhibits potassium current flowing outside from the cells.

### Test Example 3

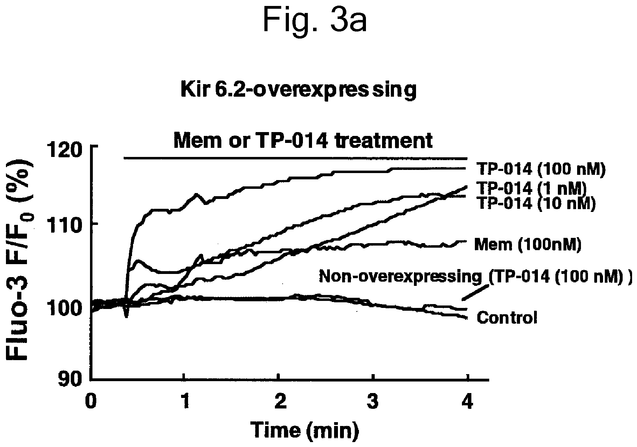

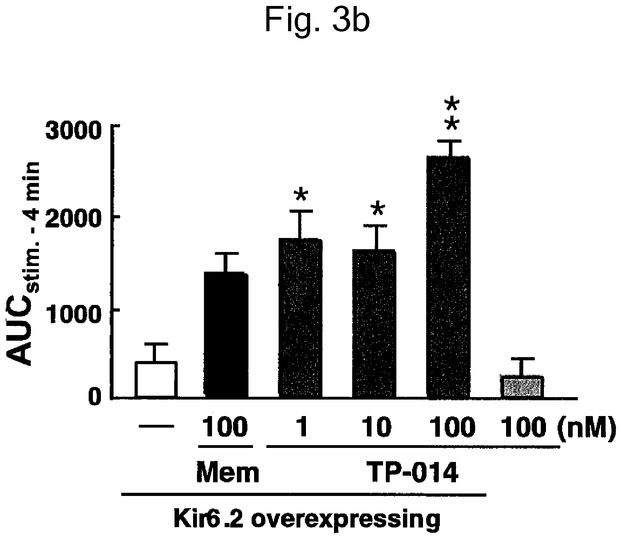

Using the same Kir6.2 channel-overexpressing cells as in Test Example 1, the amount of calcium that flows into cells from outside the cells by TP-014 treatment was measured by a calcium imaging method. The results are shown in Fig. 3. The calcium imaging method is a method of measuring the amount of calcium based on the intensity of fluorescence of cultured nerve cells, which are treated in a culture solution containing a calcium fluorescent dye (Fura2, manufactured by Dojindo Laboratories) in a concentration of 4 µM. Imaging was carried out by an imaging apparatus (LAMBDA10-2, manufactured by SUTTER INSTRUMENT) in accordance with the manual attached to the apparatus. Fig. 3a shows a change of the TP-014 concentration-dependent calcium amount with time (for 4 minutes) when treatment was performed with TP-014 (1 to 100 nM) and memantine (100 nM). Fig. 3b shows the measurement results of calcium amount when measurement was carried out 4 minutes after the treatment with memantine (100 nM) and TP-014 (1-100 nM) (n = 5 per group). TP-014 has a larger calcium concentration increasing effect than memantine. It was confirmed that the amount of calcium within cells is significantly increased by the treatment with TP-014, due to inhibition of outflow of potassium from the cells confirmed in Test Example 2.

### Test Example 4

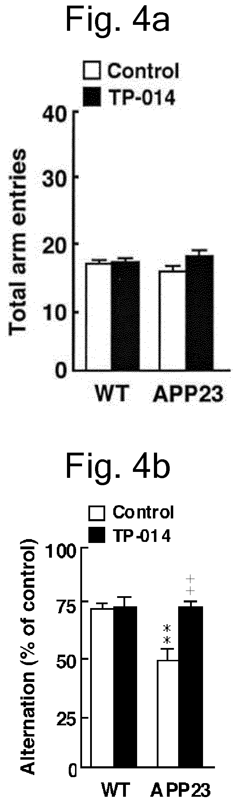

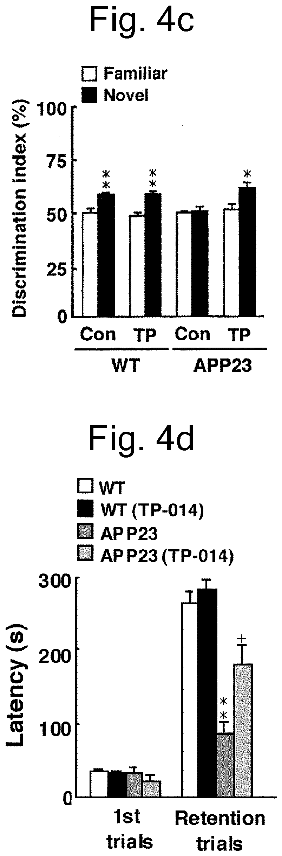

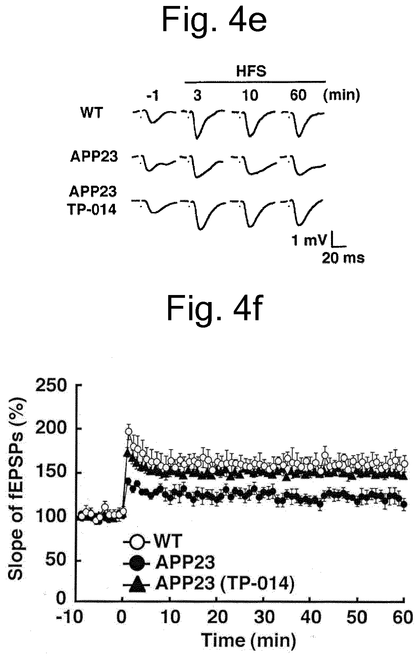

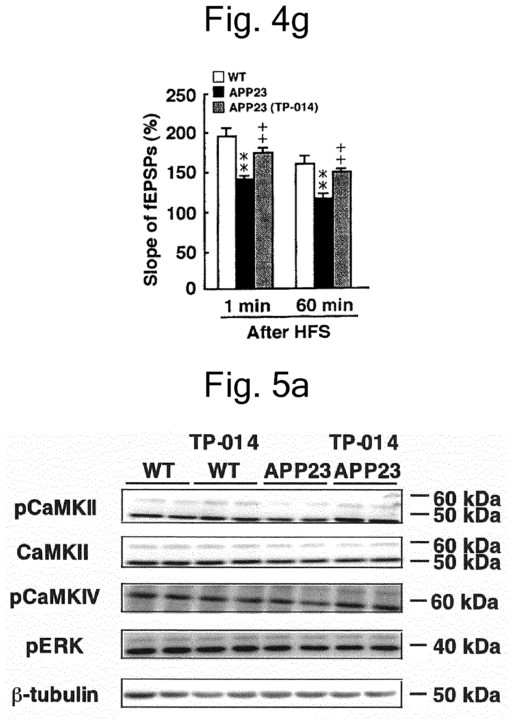

To Alzheimer's disease model mice (APP23 mice, Sturchler-Pierrat et al., Proc. Natl. Acad. Sci. U.S.A. 1997, 94, 13287-13292)(12 months old), TP-014 (1 mg/kg) was orally administered once a day for two months (chronic treatment). As a result, a significance cognitive function improvement effect was confirmed. The results are shown in Fig. 4. Figs. 4a-d show the results of behavior analysis. Fig. 4a and Fig. 4b show attentional function analyzed on WT mice (C57BL/6J, Japan SLC) and APP23 mice (n = 5 per group) by an ordinary Y-maze method. As a result, it was confirmed that a significant attentional function improvement effect is exerted by treatment with TP-014. The Y-maze method is a method of allowing mice to freely walk on three arms for 8 minutes. The arms will be provisionally designated as A, B, C, respectively. A mouse positioned on A will move to B or C (arm). The case where the mouse moves to B and then moves to C, in short, the case of sequentially moving A-B-C, is regarded as a right answer; whereas the case where a mouse moves A-B-A and avoid choosing a new arm is regarded as a wrong answer. The arms to which a mouse moved are recorded in chronological order and the number of times a mouse moves to each of the arms within a predetermined time is counted and determined as "total arm entries". Of them, the number of the right-answer cases (the case where a mouse consecutively chooses three different arms) was counted and determined as the number of alternating behaviors ("No. of alternation"). The ratio of "No. of alternation" to the number obtained by subtracting 2 from the "total arm entries" is expressed as alternation (%) and used as the normal alternate behavior index (spatial working memory correct answer ratio).

Mice tends to favor a new (unfamiliar) object. The ratio of ordinary mice giving the correct answer is 70%; however the ratio of APP23 mice giving correct answer is as low as about 50%. Based on the percentages as the reference, attentional function (cognitive function) is analyzed.

Fig. 4c shows the results of memory for a new (unfamiliar) object of WT mice and APP23 mice (both, n = 5) assessed by an ordinary new-object recognition test method. The new object recognition test method is carried out as follows. Two identical shape building blocks are placed in a mouse cage and mice are allowed to play for 10 minutes (this is referred to as a training trial). One hour later, one of the building blocks was replaced with a different-shaped building block. Since normal mice show interest on a new object, they play a longer time with the different-shaped building block. The Alzheimer's disease (model) mice, since the mice fail to recognize the new object (building block), are seemed to have memory disturbance. Mice are allowed to freely play for further 5 minutes with two building blocks different in shape (this is referred to as retention trial). In the training trial and retention trial, the number of times a mouse is in contact with each of the two objects was counted. The ratio (%) of the number of contact times to the different-shaped building block to the total number of contact times in the retention trial was calculated and used as a discrimination index.

Fig. 4d shows the results of fear memory analyzed by an ordinary fear conditioning test method (n = 5 per group). The fear conditioning test method is an analysis taking an advantage of mouse's preposition in favor of a dark place rather than a bright place. Day 1, a mouse is placed in a bright place. Since a mouse is fond of a dark place, the mouse enters a dark place (dark box). When an electrical stimulation is given to the mouse at this time, the mouse is surprised and returns to the bright place and never enters the dark place. Day 2, the mouse is placed in the bright place (the same place as on Day 1), and then, whether the mouse enters a dark place or not is observed for 5 minutes. If the mouse enters the dark place right away, it is determined that fear memory of the mouse declined. "Latency" is a time period (seconds) until the mouse placed in a bright place enters the dark plate (darkroom) on Day 2. Since APP23 mice entered a dark place right away, it was confirmed that fear memory declined; however, in the mice treated with TP-014 for two months, an improvement effect was confirmed.

Fig. 4e to Fig. 4g show the results of long-term potentiation phenomenon (LTP)(serving as an index of memory formation) analyzed by an electrophysiological technique. The hippocampus in the brain plays an important role in memorization. The hippocampus was cut into slice sections (400 micrometers in thickness). The slice sections were placed in an artificial cerebrospinal fluid (126 mM NaCl, 5 mM KCL, 26 mM NaHCO₃, 1.3 mM MgSO₄-7H₂O, 1.26 mM KH₂PO₄, 2.4 mM CaCl₂-2H₂O, 10 mM glucose) saturated with a 95% O₂/5% CO₂ gas at 34 °C for two hours and recovered. The hippocampal slice sections were transferred to a measuring chamber and the artificial cerebrospinal fluid containing TP-014 was perfused. The activity of the nerve cells when electrical stimulation was applied was recorded and the postsynaptic assembly potential (fEPSP) was measured. Based on them, the degree of LTP improvement was evaluated. The waveforms recorded are shown in Fig. 4e. Thereafter, an electrical stimulation (100Hz) was applied to the hippocampus to cause a sparse change (it is considered that memory is formed by a sparse change in the hippocampus). It was confirmed that an increase rate of the neuronal excitation decreases in APP23 mice; whereas the increase rate is improved in the mice chronically treated with TP-014. It is shown that memory learning is effectively improved by improvement of LTP.

### Test Example 5

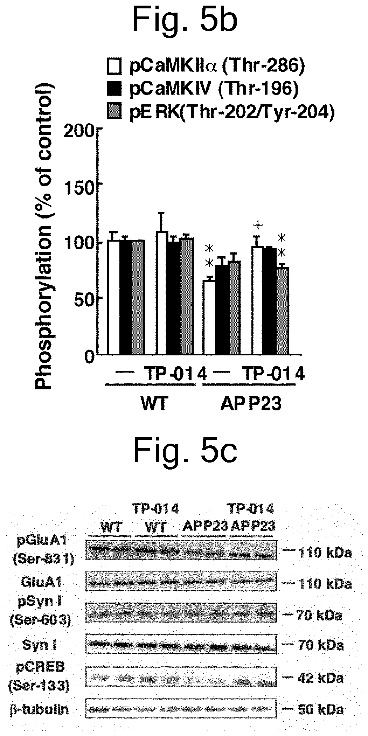

The hippocampus of APP23 mouse was excised out. To hippocampal slices, an SDS sample buffer was added to prepare a suspension, which was subjected to immunoblotting using antibodies against CaMKII, CaMKIV and ERK (CaMKII: Fukunaga et al., J. Biol. Chem. 1992, 267, 22527-22533, CaMKIV: Kasahara et al., J. Biol. Chem. 2001, 276, 24044-24050, ERK: manufactured by Sigma-Aldrich). In this manner, phosphorylation of the individual proteins was analyzed. The results are shown in Fig. 5a and Fig. 5b. CaMKII, CaMKIV and ERK are molecules all considered to play an important role in memory formation. As a result, it was observed that phosphorylation of CaMKII decreases in ordinary APP23 mice; whereas, in APP23 mice chronically treated with TP-014 (the treatment conditions are the same as in the case of Test Example 4), phosphorylation of CaMKII is accelerated. From the result, it is demonstrated that activation of CaMKII is important in effectively improving memory of APP23 mice by TP-014 treatment.

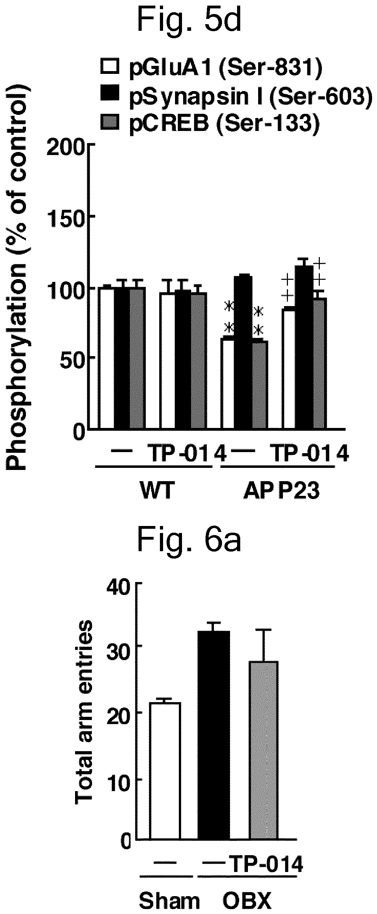

GluAl (Ser-831), Synapsin I (Ser-603) and CREB (Ser-133), which are molecules known to be activated if CaMKII is activated. These molecules were analyzed by immunoblotting of hippocampal slice sections suspended in an SDS sample buffer. Antibodies against the individual molecules were obtained from Millipore. The results are shown in Fig. 5c and Fig. 5d. It is shown that activation of GluAl (Ser-831) and CREB (Ser-133) is induced by activation of CaMKII. Fig. 5a and Fig. 5c show bands (image) actually obtained by electrophoresis of immunoblots. Fig. 5b and Fig. 5d show analysis results quantitatively showing signal intensities of the bands shown in Fig. 5a and Fig. 5c.

### Test Example 6

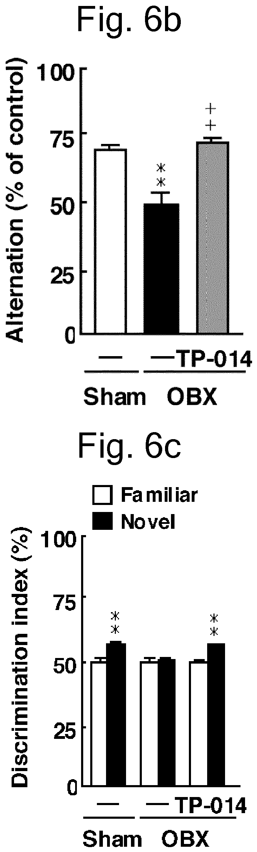

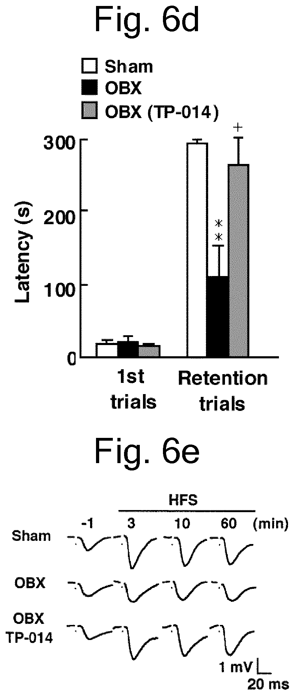

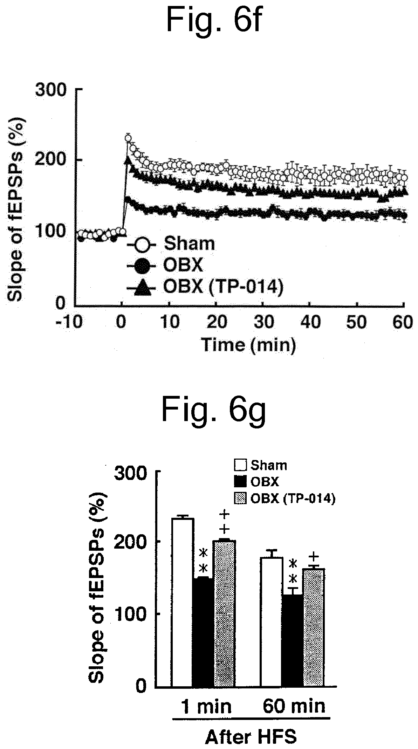

The same experiment as that shown in Fig. 4 was carried out by using neurodegenerative disease model mice, i.e., olfactory bulbectomized mice (OBX mice). The results are shown in Fig. 6a to Fig. 6g. Cognitive dysfunction of OBX mice was significantly improved by chronic administration (2 weeks) with TP-014. The OBX mice were prepared from DDY male mice of 10 weeks old (Nippon SLC, Hamamatsu, Japan). Bulbectomy surgery was carried out under anesthesia with pentobarbital sodium (50 mg/kg i.p.; Dainippon, Osaka, Japan). The mouse was fixed on a brain anchor and the skull above the olfactory bulb was punctuated by a drill to make a hole of 1 mm in diameter. The olfactory bulb was removed by a suction pump so as not to hurt the prefrontal cortex. A sham group was prepared by subjecting mice to the same operation as in OBX group except that the olfactory bulb was removed by suction.

### Test Example 7

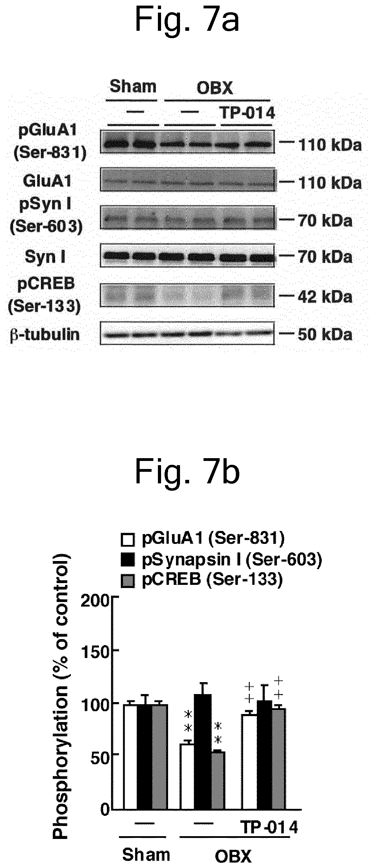

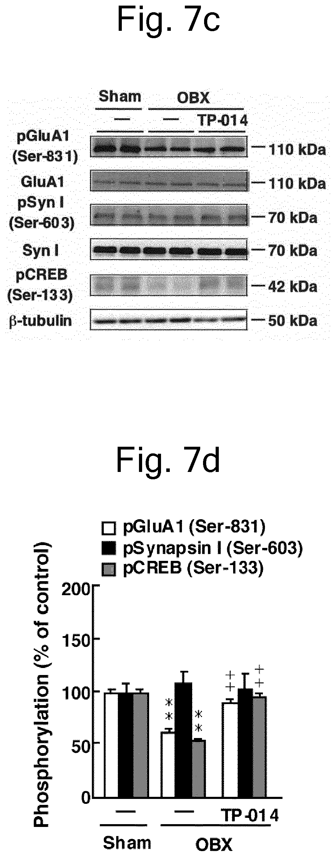

The intracellular mechanism of cognitive dysfunction in OBX mice was investigated in the same manner as in Fig. 5. The results are shown in Fig. 7a to Fig. 7d. It was found that activation of CaMKII and CaMKIV is important in the hippocampus, which plays an important role in memory formation. In addition, it was confirmed that activation of GluAl (Ser-831) and CREB (Ser-133), which are downstream molecules of CaMKII and CaMKIV in activation, is also important. The antibodies against GluAl (Ser-831) and CREB (Ser-133) were both obtained from Millipore. From the results of Figs. 4 to 7, it was found that it is important to accelerate activation of CaMKII and CaMKIV for cognitive function improvement effect of TP-014. Since cognitive dysfunction is not observed in CaMKIV gene defective mice, CaMKII is important in cognitive function improvement effect.

### Test Example 8

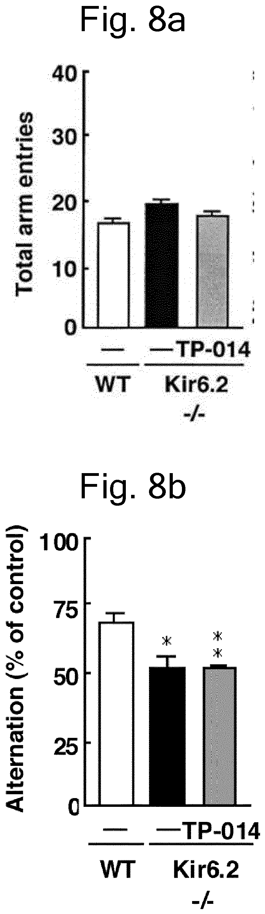

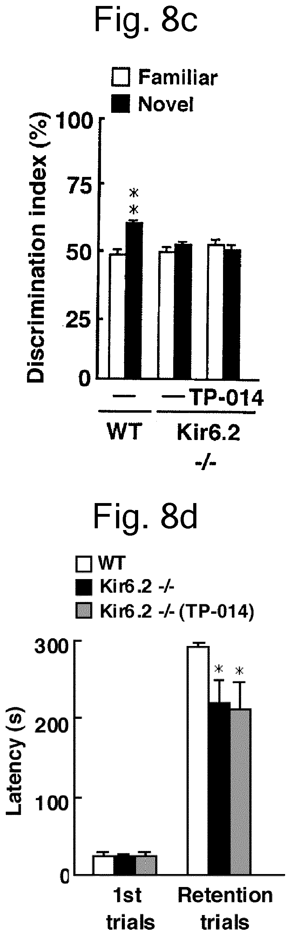

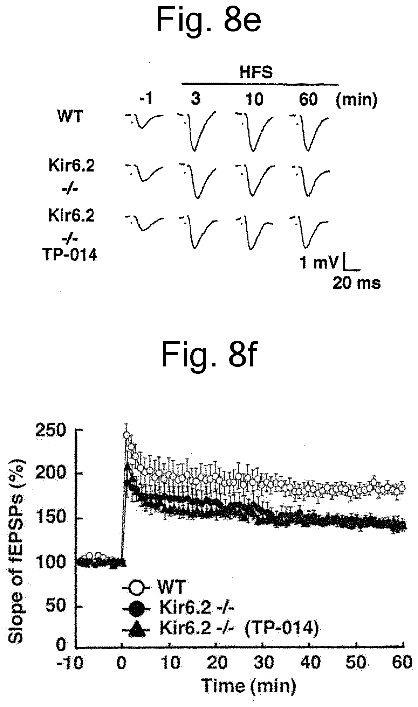

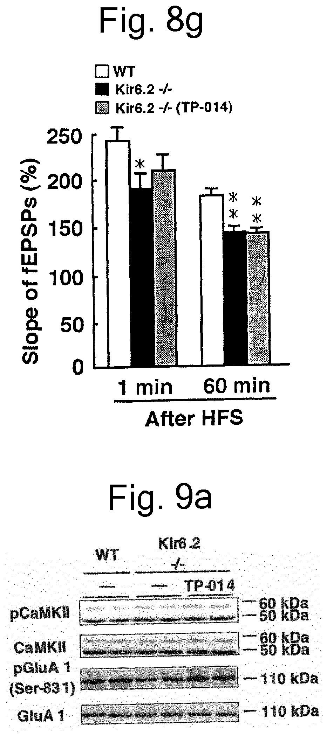

To confirm that TP-014 action is Kir6.2 channel inhibitory action, the action site of TP-014 was identified by the same behavioral experiment as in Fig. 4 (Fig. 8a and Fig. 8b: Y-maze test, Fig. 8c: new object recognition test method, Fig. 8d: fear conditioning test method, Fig. 8e to Fig. 8g: LTP improvement evaluation, n = 5 per group) using Kir6.2 channel deficient mice. The results are shown in Fig. 8. It was confirmed that cognitive dysfunction is induced in Kir6.2 defective mice. The results show that Kir6.2 channel is important for memory formation. It was also shown that memory disturbance and LTP attenuation of Kir6.2 defective mice are not improved by TP-014 chronic treatment (two months). The result shows that the action site of TP-014 is Kir6.2 channel. The analytical methods are the same as in Test Examples 4 to 7. Note that, Kir6.2 defective mice were obtained from Professor Susumu Seino, School of Medicine of Kobe University (Miki T et al., Proc. Natl. Acad. Sci. U.S.A. 1998, 95, 10402-10406).

### Test Example 9

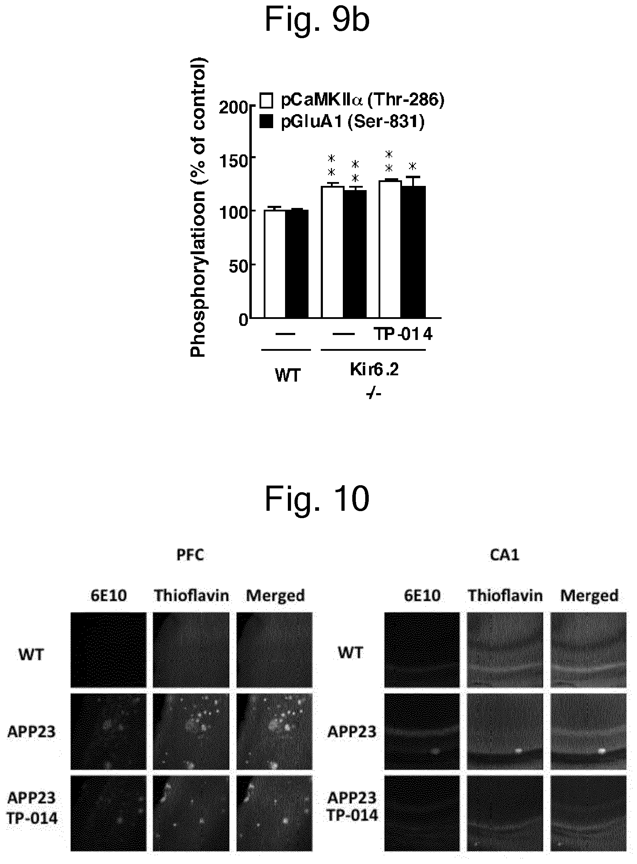

Activation of CaMKII was investigated in the same manner as in Figs. 4 to 7. In addition, GluAl (Ser-831), which is known as a molecule activated by activation of CaMKII, was investigated. Hippocampal slices were suspended in an SDS sample buffer to prepare a suspension, which was subjected to immunoblotting. In this way, the intracellular mechanism of cognitive dysfunction in Kir6.2 defective mice was analyzed. The results are shown in Fig. 9 (Fig. 9a: band images by immunoblotting, Fig. 9b: quantification results of signal intensities of bands). In the hippocampus of Kir6.2 defective mice, activation of CaMKII was accelerated. Even in the case of chronic treatment with TP-014, no influence was observed. Abnormality was observed in intracellular and extracellular calcium homeostasis (balance) by a defect of Kir6.2 channel and phosphorylation of CaMKII was accelerated. It was demonstrated that TP-014 has no influence on activation of CaMKII, which suggests that an action site of TP-014 is Kir6.2 channel.

### Test Example 10

The hypothesis that amyloid-β (Aβ) is a cause of Alzheimer's disease has now still important. Aβ aggregation, which occurs in APP23 mice (14 months old) was confirmed by immunostaining. The brains of a WT (control mouse) and APP23 mouse were cut into slice sections of 50 micrometers in thickness. The slice sections were stained with 6E10 (Aβ antibody, manufactured by Abcam) and thioflavin. The results (index for evaluating aggregates) are shown in Fig. 10. The conditions other than the above were the same as in the ordinary immunostaining method. It was found that Aβ aggregation was accelerated in the APP23 mouse, in particular, many aggregates were observed in the cerebral cortex (PFC). In contrast, aggregation was not virtually observed in the hippocampus (CA1). Aβ aggregation was inhibited by TP-014 chronic treatment. The result demonstrates that TP-014 has an inhibitory effect against Aβ aggregation.

### Test Example 11

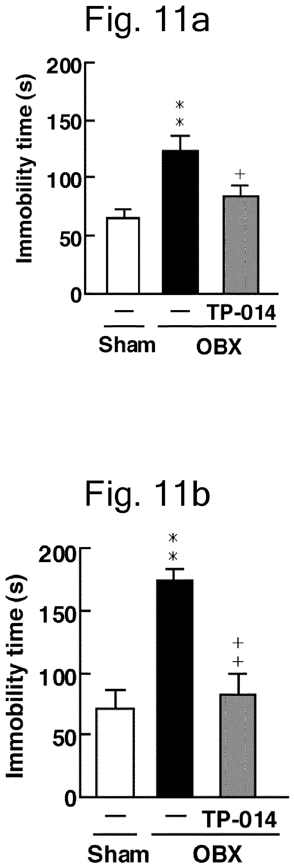

The improvement effect of TP-014 on a depression-like symptom was checked by using OBX mice as depression model mice. The results are shown in Fig. 11. OBX mice were originally established as depression model mice although a deterioration of cognitive function was observed. Depression was analyzed by a tail-suspension method (a) and a forced swim method (b). The tail-suspension method is a method of pinching the tail of a mouse and hanging the mouse upside down. If the mouse hung has depression, immobility time is long. Since a normal mouse moves even if it is hung, the immobility time is short. In the forced swim method, a mouse is forced to swim in water in a beaker. A mouse having depression neither swims nor moves (just floating). Thus the immobility time is measured. Immobility time was long in OBX mice both in the tail-suspension method (a) and the forced swim method (b). The immobility time of a TP-014 chronic administration group (2 weeks, in the same administration method as that previously mentioned) was improved. From the result, it was demonstrated that TP-014 has a depression-like symptom improving effect on OBX mice (n = 5 per group).

### Test Example 12

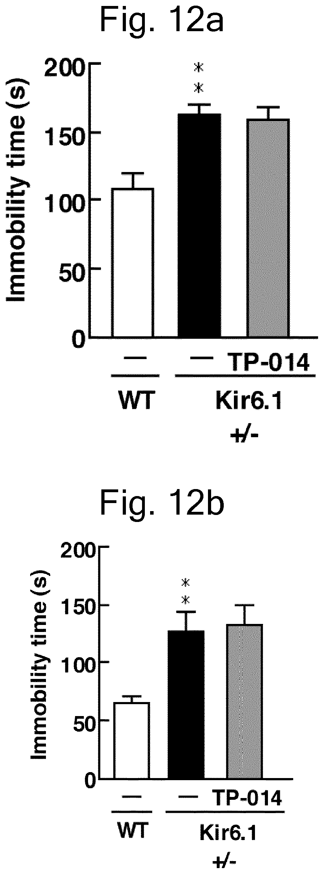

Immobility time of Kir6.1 defective mice (hetero-type, n = 5 per group) was measured by the tail-suspension method (a) and the forced swim method (b) in the same manner as in Fig. 11. The hetero-type mice are the mice in which the expression level of Kir6.1 channel is half (the homo type mice develops arrhythmia after birth and dies) in contrast to the homo type mice (complete defective mice). The results are shown in Fig. 12. The Kir6.1 defective mice showed a hyperactivated depression-like symptom. From the results, it was found that Kir6.1 is an important molecule for depression. The chronic treatment with TP-014 was not effective. It was confirmed that TP-014 produces depression-improvement effect through an inhibitory action of Kir6.1 channel.
Kir6.1 defective mice were obtained from Professor Susumu Seino, School of Medicine of Kobe University (Miki T et al., Nature Medicine, 2002, 8, 466-472).

### Test Example 13

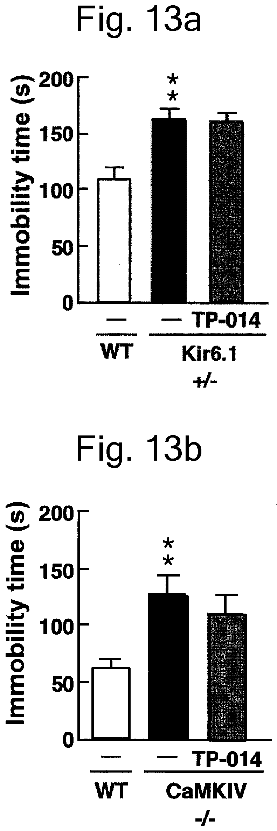

CaMKIV induced by Kir6.1 channel was analyzed by using CaMKIV defective mice (n = 5, per group*) in the same manner as in Fig. 12. The results are shown in Fig. 13. Acceleration of depression-like symptom was observed also in the CaMKIV defective mice. From the result, it is found that CaMKIV is important for a mechanism of developing depression. TP-014 had no effect on CaMKIV depression-like symptom (increase of immobility time). It was found that TP-014 inhibits Kir6.1 channel and exerts a depression improvement effect through activation of CaMKIV. CaMKIV defective mice were obtained from professor, Hiroyuki Sakagami, Kitasato University School of Medicine (Takao K et al., PLoS One 2010, 5, e9460).

### Test Example 14

A hypoglycemic effect by TP-014 was checked by measuring the blood glucose level by an assay kit (manufactured by Technicon International co). The results are shown in Fig. 14. The measurement was carried out for 4 weeks. As a result that chronic treatment with TP-014 (1 mg/kg) was carried out for 4 weeks, blood glucose level significantly decreased on and after 3 weeks. As a control, tolbutamide was used.
Kir6.2 channel binds to SUR1 (urea receptor) on the cell membrane to form a channel. Action mechanism is considered due to Kir6.2 channel inhibitory action. Tolbutamide binds to SUR1 to inhibit Kir6.2 channel.

### Test Example 15

A plasmid vector having cDNA of Kir6.1 channel inserted therein: pcDNA3.1-Kir6.1, was obtained from professor Toru Ishizuka of Graduate School of Life Sciences, Tohoku University. N2A cells overexpressing Kir6.1 channel were obtained in the same manner as in "preparation of N2A cells overexpressing Kir6.2 channel" of Test Example 1 except that the above plasmid was used.

Activation of CaMKIV was analyzed (measured) by using Kir6.1 channel-overexpressing cells obtained. The same immunoblotting as in Test Example 1 was employed as the analysis method. As the primary antibody, an anti-phosphorylated CaMKIV antibody (Kasahara J et al., J. Biol. Chem. 2001, 276, 24044-50) was used. As the secondary antibody, an anti-rabbit IgG antibody (manufactured by SouthernBiotech) was used.

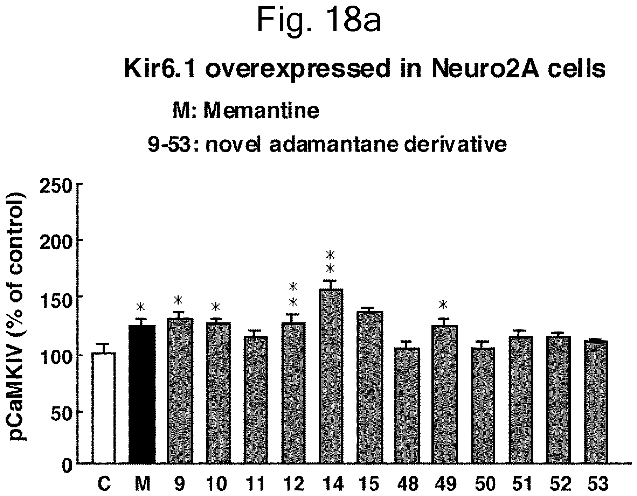

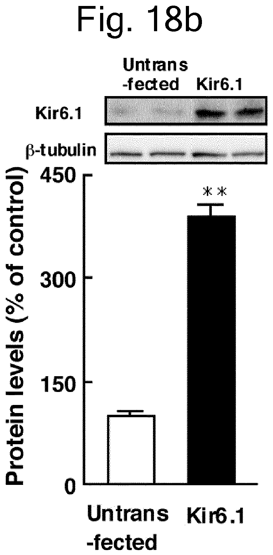

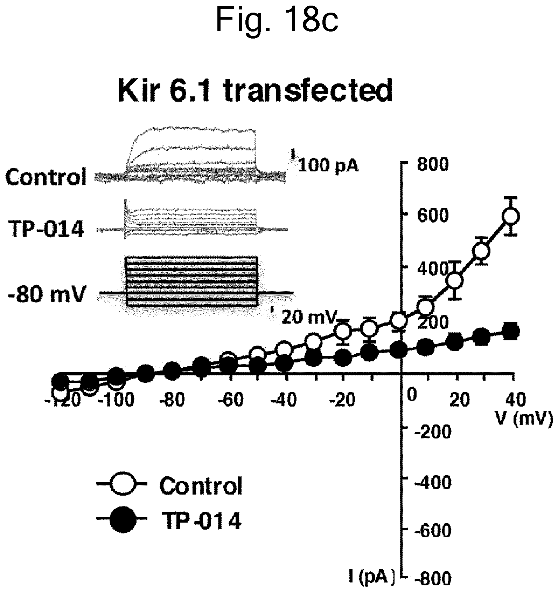

Using Kir6.1 channel-overexpressing cells obtained, potassium current flowing outside from cells was measured by the ordinary patch-clamp method. The results are shown in Fig. 18. ATP-sensitive potassium channel (Kir6.1 channel) was localized in the cell membrane of the nerve cells. If the channel is inhibited and clogged, the threshold of the nerve cell membrane rises to produce the analogous state as where an action potential is temporarily generated, with the result that potassium current flows out from cells outside and, in place, calcium current flows into the cells from outside. Fig. 18a shows that Kir6.1 channel is overexpressed in N2A cells (the upper figure shows stained images by immunoblotting; whereas the lower figure quantitatively expresses the signal intensity of bands). This was confirmed by applying immunoblotting (the same conditions as in Test Example 1 were employed except that anti-Kir6.1 channel antibody, n = 5) with an anti-Kir6.1 channel antibody (prepared based on an ordinary method) to Kir6.1 channel-overexpressing cells (prepared by the aforementioned method). No change was observed in a housekeeping gene, i.e., β-tubulin (an anti-β-tubulin antibody was obtained from Sigma-Aldrich. Other conditions are the same as those in detection of Kir6.1). Fig. 18b shows that if Kir6.2 channel-overexpressing cells are allowed to stand still in an electrophysiological experimental buffer containing TP-014 so as to obtain a concentration of 10 nM, potassium current, which outwardly flows when the membrane potential of nerve cells is changed toward a plus side, is suppressed (n = 5 per group). The results show that TP-014 inhibits Kir6.1 channel and inhibits potassium current flowing outside from the cells.

### Test Example 16

Using WT mice (C57BL/6J, Japan SLC, two months old) administered with corticosterone (dose of 5 mg/kg was administered once a day for 2 weeks); and Kir6.1 defective mice administered with corticosterone, as disease model mice showing anxiety-like symptoms, five behavioral tests regarding anxiety-related behavior were conducted. Note that, the Kir6.1 defective mice were obtained from Professor Susumu Seino, School of Medicine of Kobe University (Miki T et al., Nature Medicine 2002, 8, 466-472).

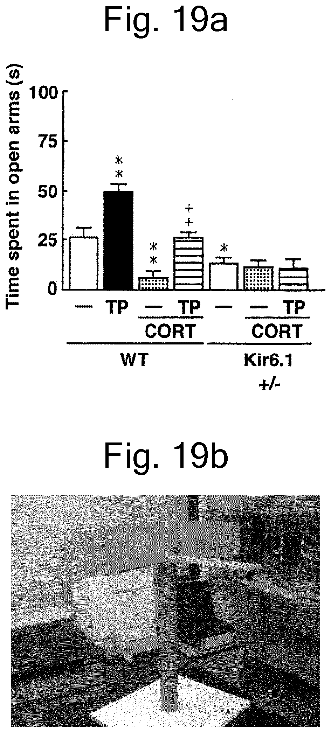

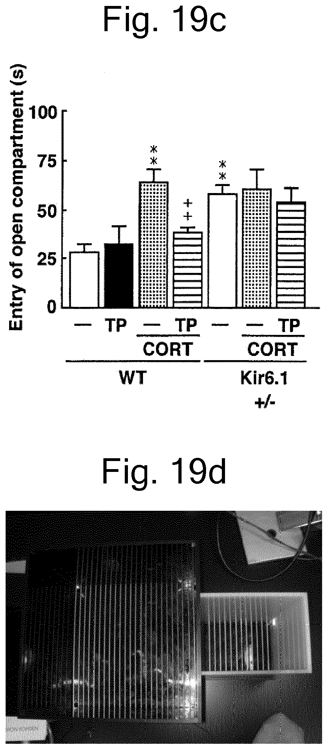

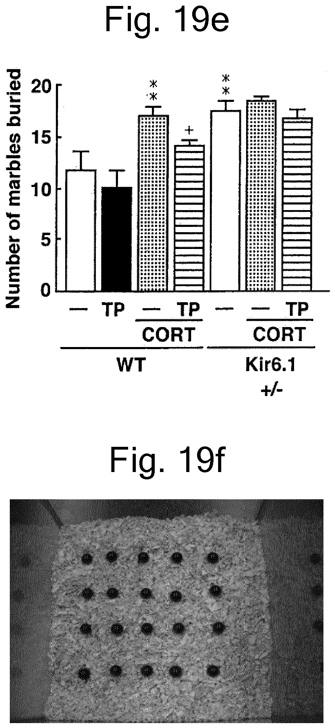

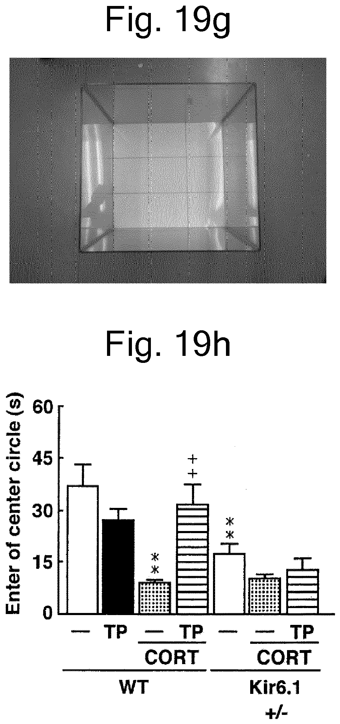

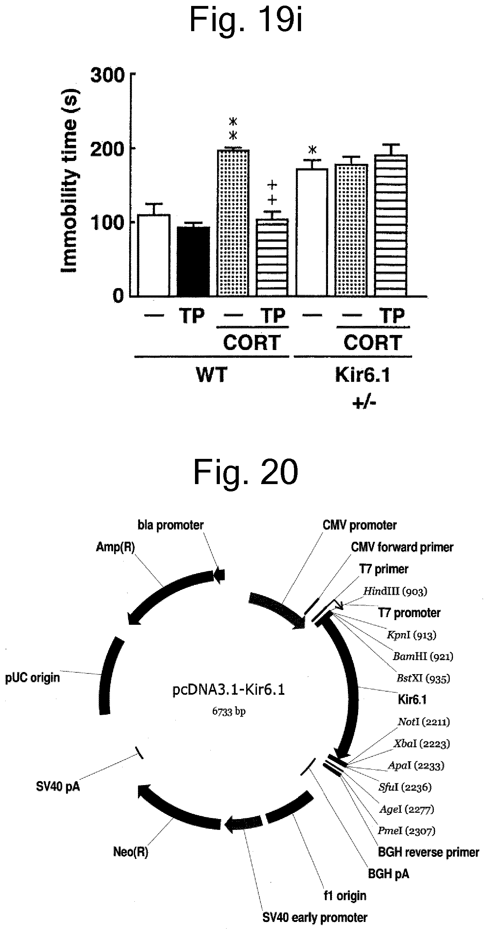

To the WT mice and Kir6.1 defective mice administered with corticosterone, TP-014 (1 mg/kg) was administered via the oral route once a day for 2 weeks (chronic treatment). As a result, it was confirmed that a significant anxiety symptom acceleration improving effect was obtained. The results are shown in Fig. 19.

Fig. 19a shows the results of vulnerability of each group (n = 5 per group) to anxiety analyzed by elevated plus-maze method (Fig. 19b). In the apparatus used herein, plus-shaped apparatus with arms is provided at a high place and individual arms are either open (visible) or enclosed. Mice vulnerable to anxiety stay for a long time in the enclosed arm; whereas mice resistant to anxiety stay in the open arm (visible). The retention time on the open arm is indicated on the vertical axis of Fig. 19a.

Fig. 19c shows the results by a light/dark test method (Fig. 19d) (n = 5 per group). The time until a mouse placed in a black box (dark place) feels anxiety to light and comes out of the box to a bright place) was measured. The time until a mouse comes out (entry of open compartment) is indicated in the vertical axis of Fig. 19c.

Fig. 19e shows the results of a marble burying method, (Fig. 19f) (n = 5 per group). In the cage for housing a mouse is filled with wood chip bedding and 20 marbles are placed such that a mouse can see them. A mouse is allowed to freely move for 30 minutes. The number of marbles buried and hidden in the wood chip bedding is counted. Since a mouse does not like a glowing object, mice resistant to anxiety come to be in contact with many marbles. The number of marbles buried is indicated on the vertical axis of Fig. 19e.

Fig. 19g shows the test results by an open field method (Fig. 19h) (n = 5 per group). A mouse is placed in a square box and allowed to move for 30 minutes within the box. Usually, a mouse which feels strong anxiety tends to walk along the edge of the box; whereas a mouse having strong resistance to anxiety frequently walks in the center portion of the box. Such a tendency is used as a reference. The time of staying in the center portion of the box is indicated in Fig. 19h.

Fig. 19i shows the results of a test performed in accordance with a fear conditioning method (n = 5 per group). Using the same test apparatus as in the light/dark test method, a mouse is placed in a dark place, and then, sound (treble) is produced for 30 seconds and thereafter electrical stimulation is given for 3 seconds. After the sound, electrical stimulation is repeatedly given three times. If so, the mouse learns that electrical stimulation will be given after the sound. On the following day, sound is kept ringing for 5 minutes. A mouse if it felt fear/anxiety, does not move. The immobility time of the mouse is measured. The immobility time is indicated on the vertical axis of Fig. 19i.

In all above test results, it was confirmed that chronic administration (2 weeks) with TP-014 improve acceleration of anxiety-like symptom. Kir6.1 defective mice administered with corticosterone exhibit anxiety-like symptoms; however, no improvement effect was obtained by administration of TP-014. From the results, it was confirmed that the improvement effect of the compound of the present invention on acceleration of anxiety-like symptom is exerted by way of Kir6.1.

Note that, in the drawings of this application, indications of significant difference, ** or ++ represent P < 0.01; whereas indications of significant difference, + or * represent P < 0.05.

## Claims

1. A compound represented by Formula (I): 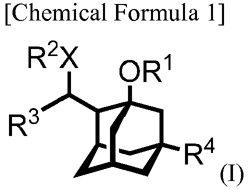
wherein R¹ represents a hydrogen atom or (C₁₋₆ alkyl)carbonyl optionally substituted with one or more halogen atoms;
R² represents a hydrogen atom or (C₁₋₆ alkyl)carbonyl optionally substituted with one or more halogen atoms;
X represents O or NR⁵;
R³ represents phenyl optionally substituted with one or more substituents selected from X¹, 5- or 6-membered heteroaryl optionally substituted with one or more substituents selected from X¹, or COOR⁶;
R⁴ represents a hydrogen atom, a halogen atom, azido, -OR⁷ or -NHR⁸;
R⁵ represents a hydrogen atom or C₁₋₆ alkyl;
R⁶ represents a hydrogen atom or C₁₋₆ alkyl;
R⁷ represents a hydrogen atom, C₁₋₆ alkyl, C₁₋₆ alkoxy-C₁₋₆ alkyl or (C₁₋₆ alkyl)carbonyl optionally substituted with one or more halogen atoms;
R⁸ represents a hydrogen atom, C₁₋₆ alkyl or (C₁₋₆ alkyl)carbonyl optionally substituted with one or more halogen atoms; and
X¹ represents C₁₋₆ alkyl, a halogen atom, C₁₋₆ alkoxy, nitro or cyano,
an enantiomer thereof, a diastereomer thereof, or a pharmaceutically acceptable salt thereof.

2. The compound, an enantiomer thereof, a diastereomer thereof, or a pharmaceutically acceptable salt thereof according to Claim 1, wherein R⁴ represents a chlorine atom or azido.

3. The compound, an enantiomer thereof, a diastereomer thereof, or a pharmaceutically acceptable salt thereof according to Claim 1 or 2, wherein R¹ represents trifluoroacetyl.

4. The compound, an enantiomer thereof, a diastereomer thereof, or a pharmaceutically acceptable salt thereof according to any one of Claims 1 to 3, wherein R² represents (C₁₋₆ alkyl)carbonyl optionally substituted with one or more halogen atoms.

5. The compound, an enantiomer thereof, a diastereomer thereof, or a pharmaceutically acceptable salt thereof according to Claim 4, wherein R² represents trifluoroacetyl.

6. The compound, an enantiomer thereof, a diastereomer thereof, or a pharmaceutically acceptable salt thereof according to any one of Claims 1 to 5, wherein R³ represents phenyl optionally substituted with one or more substituents selected from X¹ or pyridyl optionally substituted with one or more substituents selected from X¹.

7. The compound, an enantiomer thereof, a diastereomer thereof, or a pharmaceutically acceptable salt thereof according to Claim 1, selected from
(R)-((1R,2S,3R,5R,7S)-5-azido-1-hydroxyadamantan-2-yl)(phenyl)methyl acetate;
ethyl (S)-2-acetamido-2-((1R,2S,3R,5R,7R)-5-chloro-1-hydroxyadamantan-2-yl)acetate;
ethyl (R)-2-acetamido-2-((1R,2S,3R,5R,7R)-5-chloro-1-hydroxyadamantan-2-yl)acetate;
(1R,2S,3R,5R,7R)-5-chloro-2-((S)-2-methoxy-2-oxo-1-(2,2,2-trifluoroacetamido)ethyl)adamantan-1-yl 2,2,2-trifluoroacetate;
(1S,2R,3S,5S,7S)-5-chloro-2-((R)-phenyl(2,2,2-trifluoroacetamido)methyl)adamantan-1-yl 2,2,2-trifluoroacetate;
(S)-2-amino-2-((1R,2S,3R,5R,7S)-1,5-dihydroxyadamantan-2-yl)acetic acid;
N-((R)-((1S,2R,3S,5S,7S)-5-chloro-1-hydroxyadamantan-2-yl)(phenyl)methyl)-2,2,2-trifluoroacetamide;
(1S,2R,3S,5R,7S)-2-((R)-phenyl(2,2,2-trifluoroacetamido)methyl)adamantan-1-yl 2,2,2-trifluoroacetate;
(1S,2R,3S,5S,7R)-5-(2-methoxyethoxy)-2-((R)-phenyl(2,2,2-trifluoroacetamido)methyl)adamantan-1-yl 2,2,2-trifluoroacetate;
N-((R)-((1S,2R,3S,5S,7S)-5-chloro-1-hydroxyadamantan-2-yl)(pyridin-3-yl)methyl)-2,2,2-trifluoroacetamide;
2,2,2-trifluoro-N-((R)-((1S,2R,3S,5R,7S)-1-hydroxyadamantan-2-yl)(phenyl)methyl)acetamide; and
(1S,2R,3S,5S,7R)-5-methoxy-2-((R)-phenyl(2,2,2-trifluoroacetamido)methyl)adamantan-1-yl 2,2,2-trifluoroacetate,
or an enantiomer thereof, a diastereomer thereof, or a pharmaceutically acceptable salt thereof.

8. A pharmaceutical composition containing the compound, an enantiomer thereof, a diastereomer thereof, or a pharmaceutically acceptable salt thereof according to any one of Claims 1 to 7.

9. The compound, an enantiomer thereof, a diastereomer thereof, or a pharmaceutically acceptable salt thereof according to any one of Claims 1 to 7 or the pharmaceutical composition according to Claim 8, for use in treating or preventing a cognitive disease or disorder.

10. The compound, an enantiomer thereof, a diastereomer thereof, or a pharmaceutically acceptable salt thereof or the pharmaceutical composition for use according to Claim 9, wherein the cognitive disease or disorder is selected from Alzheimer's dementia, cerebrovascular dementia, Lewy body dementia, frontotemporal dementia, Parkinson's disease, a mental disease and a neurodegenerative disease.

11. The compound, an enantiomer thereof, a diastereomer thereof, or a pharmaceutically acceptable salt thereof according to any one of Claims 1 to 7 or the pharmaceutical composition according to Claim 8, for use in treating or preventing diabetes or a diabetic complication.

12. The compound, an enantiomer thereof, a diastereomer thereof, or a pharmaceutically acceptable salt thereof according to any one of Claims 1 to 7 for use as a Kir6.2 inhibitor.

13. The compound, an enantiomer thereof, a diastereomer thereof, or a pharmaceutically acceptable salt thereof according to any one of Claims 1 to 7 for use as a Kir6.1 inhibitor.

## Patentansprüche

1. Verbindung der Formel (I): 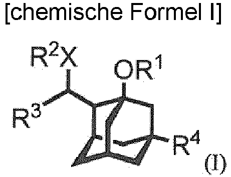
worin R¹ für ein Wasserstoffatom oder (C₁₋₆-Alkyl)carbonyl, das gegebenenfalls mit einem oder mehreren Halogenatomen substituiert ist, steht;
R² für ein Wasserstoffatom oder (C₁₋₆-Alkyl)carbonyl, das gegebenenfalls mit einem oder mehreren Halogenatomen substituiert ist, steht;
X für O oder NR⁵ steht;
R³ für Phenyl, das gegebenenfalls mit einem oder mehreren aus X¹ ausgewählten Substituenten substituiert ist, 5- oder 6-gliedriges Heteroaryl, das gegebenenfalls mit einem oder mehreren aus X¹ ausgewählten Substituenten substituiert ist, oder COOR⁶ steht;
R⁴ für ein Wasserstoffatom, ein Halogenatom, Azido, -OR⁷ oder -NHR⁸ steht;
R⁵ für ein Wasserstoffatom oder C₁₋₆-Alkyl steht;
R⁶ für ein Wasserstoffatom oder C₁₋₆-Alkyl steht;
R⁷ für ein Wasserstoffatom, C₁₋₆-Alkyl, C₁₋₆-Alkoxy-C₁₋₆-alkyl oder (C₁₋₆-Alkyl)carbonyl, das gegebenenfalls mit einem oder mehreren Halogenatomen substituiert ist, steht;
R⁸ für ein Wasserstoffatom, C₁₋₆-Alkyl oder (C₁₋₆-Alkyl)carbonyl, das gegebenenfalls mit einem oder mehreren Halogenatomen substituiert ist, steht; und
X¹ für C₁₋₆-Alkyl, ein Halogenatom, C₁₋₆-Alkoxy, Nitro oder Cyano steht,
ein Enantiomer davon, ein Diastereomer davon oder ein pharmazeutisch annehmbares Salz davon.

2. Verbindung, ein Enantiomer davon, ein Diastereomer davon oder ein pharmazeutisch annehmbares Salz davon nach Anspruch 1, worin R⁴ für ein Chloratom oder Azido steht.

3. Verbindung, ein Enantiomer davon, ein Diastereomer davon oder ein pharmazeutisch annehmbares Salz davon nach Anspruch 1 oder 2, worin R¹ für Trifluoracetyl steht.

4. Verbindung, ein Enantiomer davon, ein Diastereomer davon oder ein pharmazeutisch annehmbares Salz davon nach einem der Ansprüche 1 bis 3, worin R² für (C₁₋₆-Alkyl)-carbonyl, das gegebenenfalls mit einem oder mehreren Halogenatomen substituiert ist, steht.

5. Verbindung, ein Enantiomer davon, ein Diastereomer davon oder ein pharmazeutisch annehmbares Salz davon nach Anspruch 4, worin R² für Trifluoracetyl steht.

6. Verbindung, ein Enantiomer davon, ein Diastereomer davon oder ein pharmazeutisch annehmbares Salz davon nach einem der Ansprüche 1 bis 5, worin R³ für Phenyl, das gegebenenfalls mit einem oder mehreren aus X¹ ausgewählten Substituenten substituiert ist, oder Pyridyl, das gegebenenfalls mit einem oder mehreren aus X¹ ausgewählten Substituenten substituiert ist, steht.

7. Verbindung, ein Enantiomer davon, ein Diastereomer davon oder ein pharmazeutisch annehmbares Salz davon nach Anspruch 1, die aus den folgenden ausgewählt ist:
(R)-((1R,2S,3R,5R,7S)-5-Azido-1-hydroxyadamantan-2-yl)(phenyl)methylacetat;
Ethyl-(S)-2-acetamido-2-((1R,2S,3R,5R,7R)-5-chlor-1-hydroxyadamantan-2-yl)acetat;
Ethyl-(R)-2-acetamido-2-((1R,2S,3R,5R,7R)-5-chlor-1-hydroxyadamantan-2-yl)acetat;
(1R,2S,3R,5R,7R)-5-Chlor-2-((S)-2-methoxy-2-oxo-1-(2,2,2-trifluoracetamido)ethyl)-adamantan-1-yl-2,2,2-trifluoracetat;
(1S,2R,3S,5S,7S)-5-Chlor-2-((R)-phenyl(2,2,2-trifluoracetamido)methyl)adamantan-1-yl-2,2,2-trifluoracetat;
(S)-2-Amino-2-((1R,2S,3R,5R,7S)-1,5-dihydroxyadamantan-2-yl)essigsäure;
N-((R)-((1S,2R,3S,5S,7S)-5-Chlor-1-hydroxyadamantan-2-yl)(phenyl)methyl)-2,2,2-trifluoracetamid;
(1S,2R,3S,5R,7S)-2-((R)-Phenyl(2,2,2-trifluoracetamido)methyl)adamantan-1-yl-2,2,2-trifluoracetat;
(1S,2R,3S,5S,7R)-5-(2-Methoxyethoxy)-2-((R)-phenyl(2,2,2-trifluoracetamido)methyl)-adamantan-1-yl-2,2,2-trifluoracetat;
N-((R)-((1S,2R,3S,5S,7S)-5-Chlor-1-hydroxyadamantan-2-yl)(pyridin-3-yl)methyl)-2,2,2-trifluoracetamid;
2,2,2-Trifluor-N-((R)-((1S,2R,3S,5R,7S)-1-hydroxyadamantan-2-yl)(phenyl)methyl)-acetamid; und
(1S,2R,3S,5S,7R)-5-Methoxy-2-((R)-phenyl(2,2,2-trifluoracetamido)methyl)-adamantan-1-yl-2,2,2-trifluoracetat,
oder ein Enantiomer davon, ein Diastereomer davon oder ein pharmazeutisch annehmbares Salz davon.

8. Pharmazeutische Zusammensetzung, die eine Verbindung, ein Enantiomer davon, ein Diastereomer davon oder ein pharmazeutisch annehmbares Salz davon nach einem der Ansprüche 1 bis 7 enthält.

9. Verbindung, ein Enantiomer davon, ein Diastereomer davon oder ein pharmazeutisch annehmbares Salz davon nach einem der Ansprüche 1 bis 7 oder eine pharmazeutische Zusammensetzung nach Anspruch 8 zur Verwendung bei der Behandlung oder Prävention einer kognitiven Erkrankung oder Störung.

10. Verbindung, ein Enantiomer davon, ein Diastereomer davon oder ein pharmazeutisch annehmbares Salz davon zur Verwendung nach Anspruch 9, wobei die kognitive Erkrankung oder Störung aus Alzheimer-Demenz, zerebrovaskulärer Demenz, Lewy-Body-Demenz, frontotemporaler Demenz, Parkinson-Krankheit, einer psychischen Erkrankung und einer neurodegenerativen Erkrankung ausgewählt ist.

11. Verbindung, ein Enantiomer davon, ein Diastereomer davon oder ein pharmazeutisch annehmbares Salz davon nach einem der Ansprüche 1 bis 7 oder pharmazeutische Zusammensetzung nach Anspruch 8 zur Verwendung bei der Behandlung oder Prävention von Diabetes oder einer diabetischen Komplikation.

12. Verbindung, ein Enantiomer davon, ein Diastereomer davon oder ein pharmazeutisch annehmbares Salz davon nach einem der Ansprüche 1 bis 7 zur Verwendung als Kir6.2-Inhibitor.

13. Verbindung, ein Enantiomer davon, ein Diastereomer davon oder ein pharmazeutisch annehmbares Salz davon nach einem der Ansprüche 1 bis 7 zur Verwendung als Kir6.1-Inhibitor.

## Revendications

1. Composé représenté par la formule (I) : 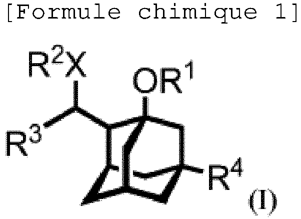
dans laquelle R¹ représente un atome d'hydrogène ou un groupe (alkyle en C₁₋₆) carbonyle éventuellement substitué par un ou plusieurs atomes d'halogène ;
R² représente un atome d'hydrogène ou un groupe (alkyle en C₁₋₆)carbonyle éventuellement substitué par un ou plusieurs atomes d'halogène ;
X représente O ou NR⁵ ;
R³ représente un groupe phényle éventuellement substitué par un ou plusieurs substituants choisis parmi X¹, un groupe hétéroaryle à 5 ou 6 éléments éventuellement par un ou plusieurs substituants choisis parmi X¹, ou COOR⁶ ;
R⁴ représente un atome d'hydrogène, un atome d'halogène, un groupe azido, -OR⁷ ou -NHR⁸ ;
R⁵ représente un atome d'hydrogène ou un groupe alkyle en C₁₋₆ ;
R⁶ représente un atome d'hydrogène ou un groupe alkyle en C₁₋₆ ;
R⁷ représente un atome d'hydrogène, un groupe alkyle en C₁₋₆, un groupe alcoxy en C₁₋₆-alkyle en C₁₋₆ ou un groupe (alkyle en C₁₋₆)carbonyle éventuellement substitué par un ou plusieurs atomes d'halogène ;
R⁸ représente un atome d'hydrogène, un groupe alkyle en C₁₋₆ ou un groupe (alkyle en C₁₋₆)carbonyle éventuellement substitué par un ou plusieurs atomes d'halogène ; et
X¹ représente un groupe alkyle en C₁₋₆, un atome d'halogène, un groupe alcoxy en C₁₋₆, un groupe nitro ou un groupe cyano, un énantiomère de celui-ci, un diastéréoisomère de celui-ci ou un sel pharmaceutiquement acceptable de celui-ci.

2. Composé, un énantiomère de celui-ci, un diastéréoisomère de celui-ci ou un sel pharmaceutiquement acceptable de celui-ci selon la revendication 1, dans lequel R⁴ représente un atome de chlore ou un groupe azido.

3. Composé, un énantiomère de celui-ci, un diastéréoisomère de celui-ci ou un sel pharmaceutiquement acceptable de celui-ci selon la revendication 1 ou 2, dans lequel R¹ représente un groupe trifluoroacétyle.

4. Composé, un énantiomère de celui-ci, un diastéréoisomère de celui-ci ou un sel pharmaceutiquement acceptable de celui--ci selon l'une quelconque des revendications 1 à 3, dans lequel R² représente un groupe (alkyle en C₁₋₆)carbonyle éventuellement substitué par un ou plusieurs atomes d'halogène.

5. Composé, un énantiomère de celui-ci, un diastéréoisomère de celui-ci ou un sel pharmaceutiquement acceptable de celui-ci selon la revendication 4, dans lequel R² représente un groupe trifluoroacétyle.

6. Composé, un énantiomère de celui-ci, un diastéréoisomère de celui-ci ou un sel pharmaceutiquement acceptable de celui-ci selon l'une quelconque des revendications 1 à 5, dans lequel R³ représente un groupe phényle éventuellement substitué par un ou plusieurs substituants choisis parmi X¹ ou un groupe pyridyle éventuellement substitué par un ou plusieurs substituants choisis parmi X¹.

7. Composé, un énantiomère de celui-ci, un diastéréoisomère de celui-ci ou un sel pharmaceutiquement acceptable de celui-ci selon la revendication 1, choisi parmi
l'acétate de (R)-((1R,2S,3R,5R,7S)-5-azido-1-hydroxyadamantan-2-yl)(phényl)méthyle ;
le (S)-2-acétamido-2-((1R,2S,3R,5R,7R)-5-chloro-1-hydroxyadamantan-2-yl)acétate d'éthyle ;
le (R)-2-acétamido-2-((1R,2S,3R,5R,7R)-5-chloro-1-hydroxyadamantan-2-yl)acétate d'éthyle ;
le 2,2,2-trifluoroacétate de (1R,2S,3R,5R,7R)-5-chloro-2-((S)-2-méthoxy-2-oxo-1-(2,2,2-trifluoroacétamido)éthyl)adamantan-1-yle ;
le 2,2,2-trifluoroacétate de (1S,2R,3S,5S,7S)-5-chloro-2-((R)-phényl(2,2,2-trifluoroacétamido)méthyl)adamantan-1-yle ;
l'acide (S)-2-amino-2-((1R,2S,3R,5R,7S)-1,5-dihydroxyadamantan-2-yl)acétique ;
le N-((R)-((1S,2R,3S,5S,7S)-5-chloro-1-hydroxyadamantan-2-yl)(phenyl)methyl)-2,2,2-trifluoroacétamide ;
le 2,2,2-trifluoroacétate de (1S,2R,3S,5R,7S)-2-((R)-phényl(2,2,2-trifluoroacétamido)méthyl)adamantan-1-yle ;
le 2,2,2-trifluoroacétate de (1S,2R,3S,5S,7R)-5-(2-méthoxyéthoxy)-2-((R)-phényl(2,2,2-trifluoroacétamido)méthyl)adamantan-l-yle ;
le N-((R)-((1S,2R,3S,5S,7S)-5-chloro-1-hydroxyadamantan-2-yl)(pyridin-3-yl)methyl)-2,2,2-trifluoroacétamide ;
le 2,2,2-trifluoro-N-((R)-((1S,2R,3S,5R,7S)-1-hydroxyadamantan-2-yl) (phenyl)methyl)-2,2,2-trifluoroacétamide ; et
le 2,2,2-trifluoroacétate de (1S,2R,3S,5S,7R)-5-méthoxy-2-((R)-phényl(2,2,2-trifluoroacétamido)méthyl)adamantan-1-yle,
ou un énantiomère de ceux-ci, un diastéréoisomère de ceux-ci ou un sel pharmaceutiquement acceptable de ceux-ci.

8. Composition pharmaceutique contenant le composé, un énantiomère de celui-ci, un diastéréoisomère de celui-ci ou un sel pharmaceutiquement acceptable de celui-ci selon l'une quelconque des revendications 1 à 7.

9. Composé, un énantiomère de celui-ci, un diastéréoisomère de celui-ci ou un sel pharmaceutiquement acceptable de celui-ci selon l'une quelconque des revendications 1 à 7 ou composition pharmaceutique selon la revendication 8, pour une utilisation dans le traitement ou la prévention d'une maladie cognitive ou d'un trouble cognitif.

10. Composé, un énantiomère de celui-ci, un diastéréoisomère de celui-ci ou un sel pharmaceutiquement acceptable de celui-ci ou composition pharmaceutique pour une utilisation selon la revendication 9, dans lesquels la maladie cognitive ou le trouble cognitif est choisi parmi la démence d'Alzheimer, la démence vasculaire cérébrale, la démence à corps de Lewy, la démence fronto-temporale, la maladie de Parkinson, une maladie mentale et une maladie neurodégénérative.

11. Composé, un énantiomère de celui-ci, un diastéréoisomère de celui-ci ou un sel pharmaceutiquement acceptable de celui-ci selon l'une quelconque des revendications 1 à 7 ou composition pharmaceutique selon la revendication 8, pour une utilisation dans le traitement ou la prévention d'un diabète ou d'une complication du diabète.

12. Composé, un énantiomère de celui-ci, un diastéréoisomère de celui-ci ou un sel pharmaceutiquement acceptable de celui-ci selon l'une quelconque des revendications 1 à 7 pour une utilisation en tant qu'inhibiteur de Kir6.2.

13. Composé, un énantiomère de celui-ci, un diastéréoisomère de celui-ci ou un sel pharmaceutiquement acceptable de celui-ci selon l'une quelconque des revendications 1 à 7 pour une utilisation en tant qu'inhibiteur de Kir6.1.
